# EUROPÄISCHE PATENTSCHRIFT

(11) **EP 1 900 393 B1**
(45) Veröffentlichungstag und Bekanntmachung des Hinweises auf die Patenterteilung: **28.02.2018**
(21) Anmeldenummer: 07017339.8
(22) Anmeldetag: 05.09.2007
(51) Int. Cl.: A61K 8/35, A61Q 5/08, A61Q 5/10, A61K 8/22

(54) **Wasserstoffperoxid-Aktivierung mit Dionen und Imidazolen**
Hydrogen peroxide activation with diones and imidazoles
Activation de peroxyde d'hydrogène à l'aide de dions et d'imidazoles

(30) Priorität: 14.09.2006 DE 102006043920
(43) Veröffentlichungstag der Anmeldung: 19.03.2008
(73) Patentinhaber: Henkel AG & Co. KGaA, 40589 Düsseldorf (DE)
(72) Erfinder: Höffkes, Horst, 40595 Düsseldorf (DE); Fuhr, Denise, 40468 Düsseldorf (DE); Groß, Wibke, 40549 Düsseldorf (DE); Knübel, Georg, 40219 Düsseldorf (DE); Oberkobusch, Doris, 40591 Düsseldorf (DE)

(56) Entgegenhaltungen:
- EP-A1- 1 598 052
- WO-A1-91/03230
- WO-A1-2005/120447
- WO-A1-2005/123019
- DE-A1- 3 814 685
- DE-A1- 19 820 894
- US-A- 3 816 615
- US-A- 5 246 019

## Beschreibung

Die vorliegende Erfindung betrifft den Einsatz von Dionen und Imidazolen zur Aktivierung von Wasserstoffperoxid, in Mitteln, die zur Färbung und Aufhellung keratinischer Fasern dienen. Darüber hinaus betrifft die Erfindung Mittel zum Färben und Aufhellen keratinischer Fasern, d.h. Mittel zur Anwendung auf Keratinfasern, insbesondere menschlichen Haaren, und deren Verwendung.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Gleichzeitig besteht aber auch vielfach der Wunsch, die Haarfarbe zu ändern und zusammen mit der Haarfärbung auch eine Aufhellung des zu färbenden Haares zu erreichen.

Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Vor ihrer Anwendung auf menschliches Haar werden Haarfärbe- und/oder -aufhellungsmittel in fester oder pastöser Form mit verdünnter wäßriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist naheliegend, daß bei den Benutzern dieser Haarfarben oder Blondiermittel ein Bedürfnis besteht, diese Einwirkungszeit zu verringern.

Weder die pastenförmigen noch die pulverförmigen Färbe- und/oder Blondiermittel, die heute auf dem Markt sind, können als optimal angesehen werden. Während die Färbe- und/oder Blondierwirkung auf dem Haar als auf die Verbraucherbedürfnisse zugeschnitten bezeichnet werden kann, bestehen doch noch eine Reihe von Nachteilen und Problemen sowohl bei Herstellung als auch bei der Handhabung dieser Mittel.

So verlaufen Colorations- und Blondierprozesse an keratinischen Fasern üblicherweise bei alkalischen pH-Werten, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für die Anwender den Nachteil des intensiven Geruches und eventueller Reizung aufweist.

In Marktprodukten werden als alternative Alkalisierungsmittel zu Ammoniak beispielsweise Aminomethylpropanol oder Monoethanolamin eingesetzt. Da sie in ihrer Leistung allerdings oft hinter Ammoniak zurückbleiben, werden sie üblicherweise in Abmischung mit Ammoniak eingesetzt. Aber auch hier wird die Leistung des Ammoniaks beeinträchtigt, insbesondere in Bezug auf die Parameter Graubadeckung, Aufhellleistung und Farbergebnis.

Es besteht daher weiterhin ein Bedarf nach Wirkstoffen bzw. Wirkstoffkombinationen für Aufhell- und Färbemittel mit reduziertem Ammoniakgehalt. Insbesondere sollte in ammoniakreduzierten oder gar -freien farbstoff- und/oder wasserstoffperoxidhaltigen Formulierungen eine mit herkömmlichen ammoniakbasierten Mitteln vergleichbare Leistung erzielt werden.

US3816615 offenbart die Verwendung von Dicarbonyl-Verbindungen zur Aktivierung von Wasserstoffhaltigen Farbaufhellungsmittel. Bevorzugt ist 2,3-Butandion.

US5246019 offenbart die Verwendung von einem Photosensibilisator zusammen mit einem Wasserstoff-Spender zur Aufhellung von Haar. 2,3-Butandion ist als Photosensibilisator genannt.

DE19820894 offenbart ein 2-Komponenten-Mittel zum Färben von Fasern, enthaltend (a) eine Thiobarbitursäure und (b) eine Carbonylverbindung. Als Carbonylverbindungen können gesättigten Diketonen wie Diacetyl (2,3-Butandion) verwendet werden. Tabelle 3 zeigt Färbungen, die mit Diacetyl und Acetylaceton durchgeführt werden.

WO2005123019 offenbart ein Mittel zum gleichzeitigen Färben und Aufhellen von keratinhaltigen Fasern. Direktziehende Farbstoffe und/oder Oxidationsfarbstoff-vorprodukte werden verwendet. Das Mittel wird mit einem Oxidationsmittel (Wasserstoffperoxidlösung) vor der Verwendung gemischt.

EP1598052 offenbart ein Mittel zum Färben und Aufhellen von keratinhaltigen Fasern enthaltend direktziehende Farbstoffe.

DE3814685 offenbart eine Zubereitung zum gleichzeitigen Färben und Bleichen des Haares enthaltend Wasserstoffperoxid und Farbstoff.

WO 2005/120447 beschreibt die Verwendung von Imidazole zur Steigerung der Aufhelleistung von Bleichmittel für keratinhaltige Fasern.

WO 91/03230 beschreibt, dass Histidin die egalisierende Wirkung von Oxidationshaarfärbemittel verbessert. Es wurde nun gefunden, daß sich Dione mit besonderem Vorteil in Haarfärbe- und -aufhellungsmittel einarbeiten lassen und zu einer Leistungssteigerung dieser Mittel führen.

Gegenstand der vorliegenden Erfindung ist in einer ersten Ausführungsform ein Mittel zum Färben und Aufhellen von Keratinfasern gemäss Anspruch 1, insbesondere menschlichen Haaren, enthaltend -bezogen auf sein Gewicht - 0,001 bis 15 Gew.-% mindestens einer Verbindung der Formel (Ia): 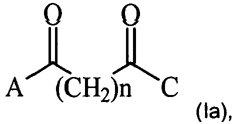 in der
- A, C: stehen unabhängig voneinander für eine gegebenenfalls substituierte, insbesondere halogenierte, C₁₋₂₈-Alkylgruppe, vorzugsweise eine gegebenenfalls substituierte, insbesondere halogenierte, C₁₋₆-Alkylgruppe,
oder eine C₂₋₂₈-Alkylengruppe, vorzugsweise eine C₂₋₆-Alkylengruppe,
wobei die Reste A und C miteinander einen Ring bilden können und wobei bevorzugte Reste A bzw. C ausgewählt sind aus -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃, -CF₃, -CCl₃, -CBr₃, -Cl₃, -CF₂CF₃,-CCl₂CCl₃
- n: steht für 0, 1, oder 2, vorzugsweise für 0.

Besonders bevorzugte Verbindungen der Formel (Ia), die in den erfindungsgemäßen Mitteln enthalten sein können, sind nachstehend aufgeführt:
1. H₃C-C(O)-C(O)-CH₃
2. H₃C-CH₂-C(O)-C(O)-CH₃
3. H₃C-(CH₂)₂-C(O)-C(O)-CH₃
4. (H₃C)₂CH-C(O)-C(O)-CH₃
5. H₃C-(CH₂)₃-C(O)-C(O)-CH₃
6. H₃C-CH₂-CH(CH₃)-C(O)-C(O)-CH₃
7. (H₃C)₂CH-CH₂-C(O)-C(O)-CH₃
8. (H₃C)₃C-C(O)-C(O)-CH₃
9. F₃C-C(O)-C(O)-CH₃
10. Cl₃C-C(O)-C(O)-CH₃
11. Br₃C-C(O)-C(O)-CH₃
12. I₃C-C(O)-C(O)-CH₃
13. F₃C-CF₂-C(O)-C(O)-CH₃
14. Cl₃C-CCl₂-C(O)-C(O)-CH₃
15. H₃C-CH₂-C(O)-C(O)-CH₂CH₃
16. H₃C-(CH₂)₂-C(O)-C(O)-CH₂CH₃
17. (H₃C)₂CH-C(O)-C(O)-CH₂CH₃
18. H₃C-(CH₂)₃-C(O)-C(O)-CH₂CH₃
19. H₃C-CH₂-CH(CH₃)-C(O)-C(O)-CH₂CH₃
20. (H₃C)₂CH-CH₂-C(O)-C(O)-CH₂CH₃
21. (H₃C)₃C-C(O)-C(O)-CH₂CH₃
22. F₃C-C(O)-C(O)-CH₂CH₃
23. Cl₃C-C(O)-C(O)-CH₂CH₃
24. Br₃C-C(O)-C(O)-CH₂CH₃
25. I₃C-C(O)-C(O)-CH₂CH₃
26. F₃C-CF₂-C(O)-C(O)-CH₂CH₃
27. Cl₃C-CCl₂-C(O)-C(O)-CH₂CH₃
28. H₃C-(CH₂)₂-C(O)-C(O)-(CH₂)₂CH₂
29. (H₃C)₂CH-C(O)-C(O)-(CH₂)₂CH₂
30. H₃C-(CH₂)₃-C(O)-C(O)-(CH₂)₂CH₂
31. H₃C-CH₂-CH(CH₃)-C(O)-C(O)-(CH₂)₂CH₂
32. (H₃C)₂CH-CH₂-C(O)-C(O)-(CH₂)₂CH₂
33. (H₃C)₃C-C(O)-C(O)-(CH₂)₂CH₂
34. F₃C-C(O)-C(O)-(CH₂)₂CH₂
35. Cl₃C-C(O)-C(O)-(CH₂)₂CH₂
36. Br₃C-C(O)-C(O)-(CH₂)₂CH₂
37. I₃C-C(O)-C(O)-(CH₂)₂CH₂
38. F₃C-CF₂-C(O)-C(O)-(CH₂)₂CH₂
39. Cl₃C-CCl₂-C(O)-C(O)-(CH₂)₂CH₂
40. (H₃C)₂CH-C(O)-C(O)-CH(CH₃)₂
41. H₃C-(CH₂)₃-C(O)-C(O)-CH(CH₃)₂
42. H₃C-CH₂-CH(CH₃)-C(O)-C(O)-CH(CH₃)₂
43. (H₃C)₂CH-CH₂-C(O)-C(O)-CH(CH₃)₂
44. (H₃C)₃C-C(O)-C(O)-CH(CH₃)₂
45. F₃C-C(O)-C(O)-CH(CH₃)₂
46. Cl₃C-C(O)-C(O)-CH(CH₃)₂
47. Br₃C-C(O)-C(O)-CH(CH₃)₂
48. I₃C-C(O)-C(O)-CH(CH₃)₂
49. F₃C-CF₂-C(O)-C(O)-CH(CH₃)₂
50. Cl₃C-CCl₂-C(O)-C(O)-CH(CH₃)₂
51. H₃C-(CH₂)₃-C(O)-C(O)-(CH₂)₃CH₃
52. H₃C-CH₂-CH(CH₃)-C(O)-C(O)-(CH₂)₃CH₃
53. (H₃C)₂CH-CH₂-C(O)-C(O)-(CH₂)₃CH₃
54. (H₃C)₃C-C(O)-C(O)-(CH₂)₃CH₃
55. F₃C-C(O)-C(O)-(CH₂)₃CH₃
56. Cl₃C-C(O)-C(O)-(CH₂)₃CH₃
57. Br₃C-C(O)-C(O)-(CH₂)₃CH₃
58. I₃C-C(O)-C(O)-(CH₂)₃CH₃
59. F₃C-CF₂-C(O)-C(O)-(CH₂)₃CH₃
60. Cl₃C-CCl₂-C(O)-C(O)-(CH₂)₃CH₃
61. H₃C-CH₂-CH(CH₃)-C(O)-C(O)-CH(CH₃)CH₂CH₃
62. (H₃C)₂CH-CH₂-C(O)-C(O)-CH₂CH(CH₃)₂
63. (H₃C)₃C-C(O)-C(O)-CH(CH₃)CH₂CH₃
64. F₃C-C(O)-C(O)-CH(CH₃)CH₂CH₃
65. Cl₃C-C(O)-C(O)-CH(CH₃)CH₂CH₃
66. Br₃C-C(O)-C(O)-CH(CH₃)CH₂CH₃
67. I₃C-C(O)-C(O)-CH(CH₃)CH₂CH₃
68. F₃C-CF₂-C(O)-C(O)-CH(CH₃)CH₂CH₃
69. Cl₃C-CCl₂-C(O)-C(O)-CH(CH₃)CH₂CH₃
70. (H₃C)₂CH-CH₂-C(O)-C(O)-CH₂CH(CH₃)₂
71. (H₃C)₃C-C(O)-C(O)-CH₂CH(CH₃)₂
72. F₃C-C(O)-C(O)-CH₂CH(CH₃)₂
73. Cl₃C-C(O)-C(O)-CH₂CH(CH₃)₂
74. Br₃C-C(O)-C(O)-CH₂CH(CH₃)₂
75. I₃C-C(O)-C(O)-CH₂CH(CH₃)₂
76. F₃C-CF₂-C(O)-C(O)-CH₂CH(CH₃)₂
77. Cl₃C-CCl₂-C(O)-C(O)-CH₂CH(CH₃)₂
78. (H₃C)₃C-C(O)-C(O)-C(CH₃)₃
79. F₃C-C(O)-C(O)-C(CH₃)₃
80. Cl₃C-C(O)-C(O)-C(CH₃)₃
81. Br₃C-C(O)-C(O)-C(CH₃)₃
82. I₃C-C(O)-C(O)-C(CH₃)₃
83. F₃C-CF₂-C(O)-C(O)-C(CH₃)₃
84. Cl₃C-CCl₂-C(O)-C(O)-C(CH₃)₃
85. F₃C-C(O)-C(O)-CF₃
86. Cl₃C-C(O)-C(O)-CF₃
87. Br₃C-C(O)-C(O)-CF₃
88. I₃C-C(O)-C(O)-CF₃
89. F₃C-CF₂-C(O)-C(O)-CF₃
90. Cl₃C-CCl₂-C(O)-C(O)-CF₃
91. Cl₃C-C(O)-C(O)-CCl₃
92. Br₃C-C(O)-C(O)-CCl₃
93. I₃C-C(O)-C(O)-CCl₃
94. F₃C-CF₂-C(O)-C(O)-CCl₃
95. Cl₃C-CCl₂-C(O)-C(O)-CCl₃
96. Br₃C-C(O)-C(O)-CBr₃
97. I₃C-C(O)-C(O)-CBr₃
98. F₃C-CF₂-C(O)-C(O)-CBr₃
99. Cl₃C-CCl₂-C(O)-C(O)-CBr₃
100. I₃C-C(O)-C(O)-Cl₃
101. F₃C-CF₂-C(O)-C(O)-Cl₃
102. Cl₃C-CCl₂-C(O)-C(O)-Cl₃
103. CF₂CF₃-C(O)-C(O)-CF₂CF₃
104. CCl₂CCl₃-C(O)-C(O)-CF₂CF₃
105. CCl₂CCl₃-C(O)-C(O)-CCl₂CCl₃
106. H₃C-C(O)-(CH₂)-C(O)-CH₃
107. H₃C-CH₂-C(O)-(CH₂)-C(O)-CH₃
108. H₃C-(CH₂)₂-C(O)-(CH₂)-C(O)-CH₃
109. (H₃C)₂CH-C(O)-(CH₂)-C(O)-CH₃
110. H₃C-(CH₂)₃-C(O)-(CH₂)-C(O)-CH₃
111. H₃C-CH₂-CH(CH₃)-C(O)-(CH₂)-C(O)-CH₃
112. (H₃C)₂CH-CH₂-C(O)-(CH₂)-C(O)-CH₃
113. (H₃C)₃C-C(O)-(CH₂)-C(O)-CH₃
114. F₃C-C(O)-(CH₂)-C(O)-CH₃
115. Cl₃C-C(O)-(CH₂)-C(O)-CH₃
116. Br₃C-C(O)-(CH₂)-C(O)-CH₃
117. I₃C-C(O)-(CH₂)-C(O)-CH₃
118. F₃C-CF₂-C(O)-(CH₂)-C(O)-CH₃
119. Cl₃C-CCl₂-C(O)-(CH₂)-C(O)-CH₃
120. H₃C-CH₂-C(O)-(CH₂)-C(O)-CH₂CH₃
121. H₃C-(CH₂)₂-C(O)-(CH₂)-C(O)-CH₂CH₃
122. (H₃C)₂CH-C(O)-(CH₂)-C(O)-CH₂CH₃
123. H₃C-(CH₂)₃-C(O)-(CH₂)-C(O)-CH₂CH₃
124. H₃C-CH₂-CH(CH₃)-C(O)-(CH₂)-C(O)-CH₂CH₃
125. (H₃C)₂CH-CH₂-C(O)-(CH₂)-C(O)-CH₂CH₃
126. (H₃C)₃C-C(O)-(CH₂)-C(O)-CH₂CH₃
127. F₃C-C(O)-(CH₂)-C(O)-CH₂CH₃
128. Cl₃C-C(O)-(CH₂)-C(O)-CH₂CH₃
129. Br₃C-C(O)-(CH₂)-C(O)-CH₂CH₃
130. I₃C-C(O)-(CH₂)-C(O)-CH₂CH₃
131. F₃C-CF₂-C(O)-(CH₂)-C(O)-CH₂CH₃
132. Cl₃C-CCl₂-C(O)-(CH₂)-C(O)-CH₂CH₃
133. H₃C-(CH₂)₂-C(O)-(CH₂)-C(O)-(CH₂)₂CH₂
134. (H₃C)₂CH-C(O)-(CH₂)-C(O)-(CH₂)₂CH₂
135. H₃C-(CH₂)₃-C(O)-(CH₂)-C(O)-(CH₂)₂CH₂
136. H₃C-CH₂-CH(CH₃)-C(O)-(CH₂)-C(O)-(CH₂)₂CH₂
137. (H₃C)₂CH-CH₂-C(O)-(CH₂)-C(O)-(CH₂)₂CH₂
138. (H₃C)₃C-C(O)-(CH₂)-C(O)-(CH₂)₂CH₂
139. F₃C-C(O)-(CH₂)-C(O)-(CH₂)₂CH₂
140. Cl₃C-C(O)-(CH₂)-C(O)-(CH₂)₂CH₂
141. Br₃C-C(O)-(CH₂)-C(O)-(CH₂)₂CH₂
142. I₃C-C(O)-(CH₂)-C(O)-(CH₂)₂CH₂
143. F₃C-CF₂-C(O)-(CH₂)-C(O)-(CH₂)₂CH₂
144. Cl₃C-CCl₂-C(O)-(CH₂)-C(O)-(CH₂)₂CH₂
145. (H₃C)₂CH-C(O)-(CH₂)-C(O)-CH(CH₃)₂
146. H₃C-(CH₂)₃-C(O)-(CH₂)-C(O)-CH(CH₃)₂
147. H₃C-CH₂-CH(CH₃)-C(O)-(CH₂)-C(O)-CH(CH₃)₂
148. (H₃C)₂CH-CH₂-C(O)-(CH₂)-C(O)-CH(CH₃)₂
149. (H₃C)₃C-C(O)-(CH₂)-C(O)-CH(CH₃)₂
150. F₃C-C(OHCH₂)-C(O)-CH(CH₃)₂
151. Cl₃C-C(O)-(CH₂)-C(O)-CH(CH₃)₂
152. Br₃C-C(O)-(CH₂)-C(O)-CH(CH₃)₂
153. I₃C-C(O)-(CH₂)-C(O)-CH(CH₃)₂
154. F₃C-CF₂-C(O)-(CH₂)-C(O)-CH(CH₃)₂
155. Cl₃C-CCl₂-C(O)-(CH₂)-C(O)-CH(CH₃)₂
156. H₃C-(CH₂)₃-C(O)-(CH₂)-C(O)-(CH₂)₃CH₃
157. H₃C-CH₂-CH(CH₃)-C(O)-(CH₂)-C(O)-(CH₂)₃CH₃
158. (H₃C)₂CH-CH₂-C(O)-(CH₂)-C(O)-(CH₂)₃CH₃
159. (H₃C)₃C-C(O)-(CH₂)-C(O)-(CH₂)₃CH₃
160. F₃C-C(OHCH₂)-C(O)-(CH₂)₃CH₃
161. Cl₃C-C(O)-(CH₂)-C(O)-(CH₂)₃CH₃
162. Br₃C-C(O)-(CH₂)-C(O)-(CH₂)₃CH₃
163. I₃C-C(O)-(CH₂)-C(O)-(CH₂)₃CH₃
164. F₃C-CF₂-C(O)-(CH₂)-C(O)-(CH₂)₃CH₃
165. Cl₃C-CCl₂-C(O)-(CH₂)-C(O)-(CH₂)₃CH₃
166. H₃C-CH₂-CH(CH₃)-C(O)-(CH₂)-C(O)-CH(CH₃)CH₂CH₃
167. (H₃C)₂CH-CH₂-C(O)-(CH₂)-C(O)-CH₂CH(CH₃)₂
168. (H₃C)₃C-C(O)-(CH₂)-C(O)-CH(CH₃)CH₂CH₃
169. F₃C-C(O)-(CH₂)-C(O)-CH(CH₃)CH₂CH₃
170. Cl₃C-C(O)-(CH₂)-C(O)-CH(CH₃)CH₂CH₃
171. Br₃C-C(O)-(CH₂)-C(O)-CH(CH₃)CH₂CH₃
172. I₃C-C(O)-(CH₂)-C(O)-CH(CH₃)CH₂CH₃
173. F₃C-CF₂-C(O)-(CH₂)-C(O)-CH(CH₃)CH₂CH₃
174. Cl₃C-CCl₂-C(O)-(CH₂)-C(O)-CH(CH₃)CH₂CH₃
175. (H₃C)₂CH-CH₂-C(O)-(CH₂)-C(O)-CH₂CH(CH₃)₂
176. (H₃C)₃C-C(O)-(CH₂)-C(O)-CH₂CH(CH₃)₂
177. F₃C-C(O)-(CH₂)-C(O)-CH₂CH(CH₃)₂
178. Cl₃C-C(O)-(CH₂)-C(O)-CH₂CH(CH₃)₂
179. Br₃C-C(O)-(CH₂)-C(O)-CH₂CH(CH₃)₂
180. I₃C-C(O)-(CH₂)-C(O)-CH₂CH(CH₃)₂
181. F₃C-CF₂-C(O)-(CH₂)-C(O)-CH₂CH(CH₃)₂
182. Cl₃C-CCl₂-C(O)-(CH₂)-C(O)-CH₂CH(CH₃)₂
183. (H₃C)₃C-C(O)-(CH₂)-C(O)-C(CH₃)₃
184. F₃C-C(O)-(CH₂)-C(O)-C(CH₃)₃
185. Cl₃C-C(O)-(CH₂)-C(O)-C(CH₃)₃
186. Br₃C-C(O)-(CH₂)-C(O)-C(CH₃)₃
187. I₃C-C(O)-(CH₂)-C(O)-C(CH₃)₃
188. F₃C-CF₂-C(O)-(CH₂)-C(O)-C(CH₃)₃
189. Cl₃C-CCl₂-C(O)-(CH₂)-C(O)-C(CH₃)₃
190. F₃C-C(O)-(CH₂)-C(O)-CF₃
191. Cl₃C-C(O)-(CH₂)-C(O)-CF₃
192. Br₃C-C(O)-(CH₂)-C(O)-CF₃
193. I₃C-C(O)-(CH₂)-C(O)-CF₃
194. F₃C-CF₂-C(O)-(CH₂)-C(O)-CF₃
195. Cl₃C-CCl₂-C(O)-(CH₂)-C(O)-CF₃
196. Cl₃C-C(O)-(CH₂)-C(O)-CCl₃
197. Br₃C-C(O)-(CH₂)-C(O)-CCl₃
198. I₃C-C(O)-(CH₂)-C(O)-CCl₃
199. F₃C-CF₂-C(O)-(CH₂)-C(O)-CCl₃
200. Cl₃C-CCl₂-C(O)-(CH₂)-C(O)-CCl₃
201. Br₃C-C(O)-(CH₂)-C(O)-CBr₃
202. I₃C-C(O)-(CH₂)-C(O)-CBr₃
203. F₃C-CF₂-C(O)-(CH₂)-C(O)-CBr₃
204. Cl₃C-CCl₂-C(O)-(CH₂)-C(O)-CBr₃
205. I₃C-C(O)-(CH₂)-C(O)-Cl₃
206. F₃C-CF₂-C(O)-(CH₂)-C(O)-Cl₃
207.Cl₃C-CCl₂-C(O)-(CH₂)-C(O)-Cl₃
208. CF₂CF₃-C(O)-(CH₂)-C(O)-CF₂CF₃
209. CCl₂CCl₃-C(O)-(CH₂)-C(O)-CF₂CF₃
210. CCl₂CCl₃-C(O)-(CH₂)-C(O)-CCl₂CCl₃
211. H₃C-C(O)-(CH₂)₂-C(O)-CH₃
212. H₃C-CH₂-C(O)-(CH₂)₂-C(O)-CH₃
213. H₃C-(CH₂)₂-C(O)-(CH₂)₂-C(O)-CH₃
214. (H₃C)₂CH-C(O)-(CH₂)₂-C(O)-CH₃
215. H₃C-(CH₂)₃-C(O)-(CH₂)₂-C(O)-CH₃
216. H₃C-CH₂-CH(CH₃)-C(O)-(CH₂)₂-C(O)-CH₃
217. (H₃C)₂CH-CH₂-C(O)-(CH₂)₂-C(O)-CH₃
218. (H₃C)₃C-C(O)-(CH₂)₂-C(O)-CH₃
219. F₃C-C(O)-(CH₂)₂-C(O)-CH₃
220. Cl₃C-C(O)-(CH₂)₂-C(O)-CH₃
221. Br₃C-C(O)-(CH₂)₂-C(O)-CH₃
222. I₃C-C(O)-(CH₂)₂-C(O)-CH₃
223. F₃C-CF₂-C(O)-(CH₂)₂-C(O)-CH₃
224. Cl₃C-CCl₂-C(O)-(CH₂)₂-C(O)-CH₃
225. H₃C-CH₂-C(O)-(CH₂)₂-C(O)-CH₂CH₃
226. H₃C-(CH₂)₂-C(O)-(CH₂)₂-C(O)-CH₂CH₃
227. (H₃C)₂CH-C(O)-(CH₂)₂-C(O)-CH₂CH₃
228. H₃C-(CH₂)₃-C(O)-(CH₂)₂-C(O)-CH₂CH₃
229. H₃C-CH₂-CH(CH₃)-C(O)-(CH₂)₂-C(O)-CH₂CH₃
230. (H₃C)₂CH-CH₂-C(O)-(CH₂)₂-C(O)-CH₂CH₃
231. (H₃C)₃C-C(O)-(CH₂)₂-C(O)-CH₂CH₃
232. F₃C-C(O)-(CH₂)₂-C(O)-CH₂CH₃
233. Cl₃C-C(O)-(CH₂)₂-C(O)-CH₂CH₃
234. Br₃C-C(O)-(CH₂)₂-C(O)-CH₂CH₃
235. I₃C-C(O)-(CH₂)₂-C(O)-CH₂CH₃
236. F₃C-CF₂-C(O)-(CH₂)₂-C(O)-CH₂CH₃
237. Cl₃C-CCl₂-C(O)-(CH₂)₂-C(O)-CH₂CH₃
238. H₃C-(CH₂)₂-C(O)-(CH₂)₂-C(O)-(CH₂)₂CH₂
239. (H₃C)₂CH-C(O)-(CH₂)₂-C(O)-(CH₂)₂CH₂
240. H₃C-(CH₂)₃-C(O)-(CH₂)₂-C(O)-(CH₂)₂CH₂
241. H₃C-CH₂-CH(CH₃)-C(O)-(CH₂)₂-C(O)-(CH₂)₂CH₂
242. (H₃C)₂CH-CH₂-C(O)-(CH₂)₂-C(O)-(CH₂)₂CH₂
243. (H₃C)₃C-C(O)-(CH₂)₂-C(O)-(CH₂)₂CH₂
244. F₃C-C(O)-(CH₂)₂-C(O)-(CH₂)₂CH₂
245. Cl₃C-C(O)-(CH₂)₂-C(O)-(CH₂)₂CH₂
246. Br₃C-C(O)-(CH₂)₂-C(O)-(CH₂)₂CH₂
247. I₃C-C(O)-(CH₂)₂-C(O)-(CH₂)₂CH₂
248. F₃C-CF₂-C(O)-(CH₂)₂-C(O)-(CH₂)₂CH₂
249. Cl₃C-CCl₂-C(O)-(CH₂)₂-C(O)-(CH₂)₂CH₂
250. (H₃C)₂CH-C(O)-(CH₂)₂-C(O)-CH(CH₃)₂
251. H₃C-(CH₂)₃-C(O)-(CH₂)₂-C(O)-CH(CH₃)₂
252. H₃C-CH₂-CH(CH₃)-C(O)-(CH₂)₂-C(O)-CH(CH₃)₂
253. (H₃C)₂CH-CH₂-C(O)-(CH₂)₂-C(O)-CH(CH₃)₂
254. (H₃C)₃C-C(O)-(CH₂)₂-C(O)-CH(CH₃)₂
255. F₃C-C(O)-(CH₂)₂-C(O)-CH(CH₃)₂
256. Cl₃C-C(O)-(CH₂)₂-C(O)-CH(CH₃)₂
257. Br₃C-C(O)-(CH₂)₂-C(O)-CH(CH₃)₂
258. I₃C-C(O)-(CH₂)₂-C(O)-CH(CH₃)₂
259. F₃C-CF₂-C(O)-(CH₂)₂-C(O)-CH(CH₃)₂
260. Cl₃C-CCl₂-C(O)-(CH₂)₂-C(O)-CH(CH₃)₂
261. H₃C-(CH₂)₃-C(O)-(CH₂)₂-C(O)-(CH₂)₃CH₃
262. H₃C-CH₂-CH(CH₃)-C(O)-(CH₂)₂-C(O)-(CH₂)₃CH₃
263. (H₃C)₂CH-CH₂-C(O)-(CH₂)₂-C(O)-(CH₂)₃CH₃
264. (H₃C)₃C-C(O)-(CH₂)₂-C(O)-(CH₂)₃CH₃
265. F₃C-C(O)-(CH₂)₂-C(O)-(CH₂)₃CH₃
266. Cl₃C-C(O)-(CH₂)₂-C(O)-(CH₂)₃CH₃
267. Br₃C-C(O)-(CH₂)₂-C(O)-(CH₂)₃CH₃
268. I₃C-C(O)-(CH₂)₂-C(O)-(CH₂)₃CH₃
269. F₃C-CF₂-C(O)-(CH₂)₂-C(O)-(CH₂)₃CH₃
270. Cl₃C-CCl₂-C(O)-(CH₂)₂-C(O)-(CH₂)₃CH₃
271. H₃C-CH₂-CH(CH₃)-C(O)-(CH₂)₂-C(O)-CH(CH₃)CH₂CH₃
272. (H₃C)₂CH-CH₂-C(O)-(CH₂)₂-C(O)-CH₂CH(CH₃)₂
273. (H₃C)₃C-C(O)-(CH₂)₂-C(O)-CH(CH₃)CH₂CH₃
274. F₃C-C(O)-(CH₂)₂-C(O)-CH(CH₃)CH₂CH₃
275. Cl₃C-C(O)-(CH₂)₂-C(O)-CH(CH₃)CH₂CH₃
276. Br₃C-C(O)-(CH₂)₂-C(O)-CH(CH₃)CH₂CH₃
277. I₃C-C(O)-(CH₂)₂-C(O)-CH(CH₃)CH₂CH₃
278. F₃C-CF₂-C(O)-(CH₂)₂-C(O)-CH(CH₃)CH₂CH₃
279. Cl₃C-CCl₂-C(O)-(CH₂)₂-C(O)-CH(CH₃)CH₂CH₃
280. (H₃C)₂CH-CH₂-C(O)-(CH₂)₂-C(O)-CH₂CH(CH₃)₂
281. (H₃C)₃C-C(O)-(CH₂)₂-C(O)-CH₂CH(CH₃)₂
282. F₃C-C(O)-(CH₂)₂-C(O)-CH₂CH(CH₃)₂
283. Cl₃C-C(O)-(CH₂)₂-C(O)-CH₂CH(CH₃)₂
284. Br₃C-C(O)-(CH₂)₂-C(O)-CH₂CH(CH₃)₂
285. I₃C-C(O)-(CH₂)₂-C(O)-CH₂CH(CH₃)₂
286. F₃C-CF₂-C(O)-(CH₂)₂-C(O)-CH₂CH(CH₃)₂
287. Cl₃C-CCl₂-C(O)-(CH₂)₂-C(O)-CH₂CH(CH₃)₂
288. (H₃C)₃C-C(O)-(CH₂)₂-C(O)-C(CH₃)₃
289. F₃C-C(O)-(CH₂)₂-C(O)-C(CH₃)₃
290. Cl₃C-C(O)-(CH₂)₂-C(O)-C(CH₃)₃
291. Br₃C-C(O)-(CH₂)₂-C(O)-C(CH₃)₃
292. I₃C-C(O)-(CH₂)₂-C(O)-C(CH₃)₃
293. F₃C-CF₂-C(O)-(CH₂)₂-C(O)-C(CH₃)₃
294. Cl₃C-CCl₂-C(O)-(CH₂)₂-C(O)-C(CH₃)₃
295. F₃C-C(O)-(CH₂)₂-C(O)-CF₃
296. Cl₃C-C(O)-(CH₂)₂-C(O)-CF₃
297. Br₃C-C(O)-(CH₂)₂-C(O)-CF₃
298. I₃C-C(O)-(CH₂)₂-C(O)-CF₃
299. F₃C-CF₂-C(O)-(CH₂)₂-C(O)-CF₃
300. Cl₃C-CCl₂-C(O)-(CH₂)₂-C(O)-CF₃
301. Cl₃C-C(O)-(CH₂)₂-C(O)-CCl₃
302. Br₃C-C(O)-(CH₂)₂-C(O)-CCl₃
303. I₃C-C(O)-(CH₂)₂-C(O)-CCl₃
304. F₃C-CF₂-C(O)-(CH₂)₂-C(O)-CCl₃
305. Cl₃C-CCl₂-C(O)-(CH₂)₂-C(O)-CCl₃
306. Br₃C-C(O)-(CH₂)₂-C(O)-CBr₃
307. I₃C-C(O)-(CH₂)₂-C(O)-CBr₃
308. F₃C-CF₂-C(O)-(CH₂)₂-C(O)-CBr₃
309. Cl₃C-CCl₂-C(O)-(CH₂)₂-C(O)-CBr₃
310. I₃C-C(O)-(CH₂)₂-C(O)-Cl₃
311. F₃C-CF₂-C(O)-(CH₂)₂-C(O)-Cl₃
312. Cl₃C-CCl₂-C(O)-(CH₂)₂-C(O)-Cl₃
313. CF₂CF₃-C(O)-(CH₂)₂-C(O)-CF₂CF₃
314. CCl₂CCl₃-C(O)-(CH₂)₂-C(O)-CF₂CF₃
315. CCl₂CCl₃-C(O)-(CH₂)₂-C(O)-CCl₂CCl₃

Erfndungsgemäß besonders bevorzugte Mittel zum Färben und Aufhellen keratinischer Fasern sind dadurch gekennzeichnet, daß sie ein oder mehrere Verbindungen aus der Gruppe H₃C-C(O)-C(O)-CH₃, H₃C-CH₂-C(O)-C(O)-CH₃, H₃C-(CH₂)₂-C(O)-C(O)-CH₃, (H₃C)₂CH-C(O)-C(O)-CH₃, H₃C-(CH₂)₃-C(O)-C(O)-CH₃, H₃C-CH₂-CH(CH₃)-C(O)-C(O)-CH₃, (H₃C)₂CH-CH₂-C(O)-C(O)-CH₃, (H₃C)₃C-C(O)-C(O)-CH₃, F₃C-C(O)-C(O)-CH₃, Cl₃C-C(O)-C(O)-CH₃, Br₃C-C(O)-C(O)-CH₃, I₃C-C(O)-C(O)-CH₃, F₃C-CF₂-C(O)-C(O)-CH₃, Cl₃C-CCl₂-C(O)-C(O)-CH₃, H₃C-CH₂-C(O)-C(O)-CH₂CH₃, H₃C-(CH₂)₂-C(O)-C(O)-CH₂CH₃, (H₃C)₂CH-C(O)-C(O)-CH₂CH₃, H₃C-(CH₂)₃-C(O)-C(O)-CH₂CH₃, H₃C-CH₂-CH(CH₃)-C(O)-C(O)-CH₂CH₃, (H₃C)₂CH-CH₂-C(O)-C(O)-CH₂CH₃, (H₃C)₃C-C(O)-C(O)-CH₂CH₃, F₃C-C(O)-C(O)-CH₂CH₃, Cl₃C-C(O)-C(O)-CH₂CH₃, Br₃C-C(O)-C(O)-CH₂CH₃, I₃C-C(O)-C(O)-CH₂CH₃, F₃C-CF₂-C(O)-C(O)-CH₂CH₃, Cl₃C-CCl₂-C(O)-C(O)-CH₂CH₃, H₃C-(CH₂)₂-C(O)-C(O)-(CH₂)₂CH₂, (H₃C)₂CH-C(O)-C(O)-(CH₂)₂CH₂, H₃C-(CH₂)₃-C(O)-C(O)-(CH₂)₂CH₂, H₃C-CH₂-CH(CH₃)-C(O)-C(O)-(CH₂)₂CH₂, (H₃C)₂CH-CH₂-C(O)-C(O)-(CH₂)₂CH₂, (H₃C)₃C-C(O)-C(O)-(CH₂)₂CH₂, F₃C-C(O)-C(O)-(CH₂)₂CH₂, Cl₃C-C(O)-C(O)-(CH₂)₂CH₂, Br₃C-C(O)-C(O)-(CH₂)₂CH₂, I₃C-C(O)-C(O)-(CH₂)₂CH₂, F₃C-CF₂-C(O)-C(O)-(CH₂)₂CH₂, Cl₃C-CCl₂-C(O)-C(O)-(CH₂)₂CH₂, (H₃C)₂CH-C(O)-C(O)-CH(CH₃)₂, H₃C-(CH₂)₃-C(O)-C(O)-CH(CH₃)₂, H₃C-CH₂-CH(CH₃)-C(O)-C(O)-CH(CH₃)₂, (H₃C)₂CH-CH₂-C(O)-C(O)-CH(CH₃)₂, (H₃C)₃C-C(O)-C(O)-CH(CH₃)₂, F₃C-C(O)-C(O)-CH(CH₃)₂, Cl₃C-C(O)-C(O)-CH(CH₃)₂, Br₃C-C(O)-C(O)-CH(CH₃)₂, I₃C-C(O)-C(O)-CH(CH₃)₂, F₃C-CF₂-C(O)-C(O)-CH(CH₃)₂, Cl₃C-CCl₂-C(O)-C(O)-CH(CH₃)₂, H₃C-(CH₂)₃-C(O)-C(O)-(CH₂)₃CH₃, H₃C-CH₂-CH(CH₃)-C(O)-C(O)-(CH₂)₃CH₃, (H₃C)₂CH-CH₂-C(O)-C(O)-(CH₂)₃CH₃, (H₃C)₃C-C(O)-C(O)-(CH₂)₃CH_{3,} F₃C-C(O)-C(O)-(CH₂)₃CH₃, Cl₃C-C(O)-C(O)-(CH₂)₃CH₃, Br₃C-C(O)-C(O)-(CH₂)₃CH₃, I₃C-C(O)-C(O)-(CH₂)₃CH₃, F₃C-CF₂-C(O)-C(O)-(CH₂)₃CH₃, Cl₃C-CCl₂-C(O)-C(O)-(CH₂)₃CH₃, H₃C-CH₂-CH(CH₃)-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH(CH₃)CH₂CH₃, F₃C-C(O)-C(O)-CH(CH₃)CH₂CH₃, Cl₃C-C(O)-C(O)-CH(CH₃)CH₂CH₃, Br₃C-C(O)-C(O)-CH(CH₃)CH₂CH₃, I₃C-C(O)-C(O)-CH(CH₃)CH₂CH₃, F₃C-CF₂-C(O)-C(O)-CH(CH₃)CH₂CH₃, Cl₃C-CCl₂-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH₂CH(CH₃)₂, F₃C-C(O)-C(O)-CH₂CH(CH₃)₂, Cl₃C-C(O)-C(O)-CH₂CH(CH₃)₂, Br₃C-C(O)-C(O)-CH₂CH(CH₃)₂, I₃C-C(O)-C(O)-CH₂CH(CH₃)₂, F₃C-CF₂-C(O)-C(O)-CH₂CH(CH₃)₂, Cl₃C-CCl₂-C(O)-C(O)-CH₂CH(CH₃)₂, (H₃C)₃C-C(O)-C(O)-C(CH₃)₃, F₃C-C(O)-C(O)-C(CH₃)₃, Cl₃C-C(O)-C(O)-C(CH₃)₃, Br₃C-C(O)-C(O)-C(CH₃)₃, I₃C-C(O)-C(O)-C(CH₃)₃, F₃C-CF₂-C(O)-C(O)-C(CH₃)₃, Cl₃C-CCl₂-C(O)-C(O)-C(CH₃)₃, F₃C-C(O)-C(O)-CF₃, Cl₃C-C(O)-C(O)-CF₃, Br₃C-C(O)-C(O)-CF₃, I₃C-C(O)-C(O)-CF₃, F₃C-CF₂-C(O)-C(O)-CF₃, Cl₃C-CCl₂-C(O)-C(O)-CF₃, Cl₃C-C(O)-C(O)-CCl₃, Br₃C-C(O)-C(O)-CCl₃, I₃C-C(O)-C(O)-CCl₃, F₃C-CF₂-C(O)-C(O)-CCl₃, Cl₃C-CCl₂-C(O)-C(O)-CCl₃, Br₃C-C(O)-C(O)-CBr₃, I₃C-C(O)-C(O)-CBr₃, F₃C-CF₂-C(O)-C(O)-CBr₃, Cl₃C-CCl₂-C(O)-C(O)-CBr₃, I₃C-C(O)-C(O)-Cl₃, F₃C-CF₂-C(O)-C(O)-Cl₃, Cl₃C-CCl₂-C(O)-C(O)-Cl₃, CF₂CF₃-C(O)-C(O)-CF₂CF₃, CCl₂CCl₃-C(O)-C(O)-CF₂CF₃, CCl₂CCl₃-C(O)-C(O)-CCl₂CCl₃, H₃C-C(O)-(CH₂)-C(O)-CH₃, H₃C-CH₂-C(O)-(CH₂)-C(O)-CH₃, H₃C-(CH₂)₂-C(O)-(CH₂)-C(O)-CH₃, (H₃C)₂CH-C(O)-(CH₂)-C(O)-CH₃, H₃C-(CH₂)₃-C(O)-(CH₂)-C(O)-CH₃, H₃C-CH₂-CH(CH₃)-C(O)-(CH₂)-C(O)-CH₃, (H₃C)₂CH-CH₂-C(O)-(CH₂)-C(O)-CH₃, (H₃C)₃C-C(O)-(CH₂)-C(O)-CH₃, F₃C-C(O)-(CH₂)-C(O)-CH₃, Cl₃C-C(O)-(CH₂)-C(O)-CH₃, Br₃C-C(O)-(CH₂)-C(O)-CH₃, I₃C-C(O)-(CH₂)-C(O)-CH₃, F₃C-CF₂-C(O)-(CH₂)-C(O)-CH₃, Cl₃C-CCl₂-C(O)-(CH₂)-C(O)-CH₃, H₃C-CH₂-C(O)-(CH₂)-C(O)-CH₂CH₃, H₃C-(CH₂)₂-C(O)-(CH₂)-C(O)-CH₂CH₃, (H₃C)₂CH-C(O)-(CH₂)-C(O)-CH₂CH₃, H₃C-(CH₂)₃-C(O)-(CH₂)-C(O)-CH₂CH₃, H₃C-CH₂-CH(CH₃)-C(O)-(CH₂)-C(O)-CH₂CH₃, (H₃C)₂CH-CH₂-C(O)-(CH₂)-C(O)-CH₂CH₃, (H₃C)₃C-C(O)-(CH₂)-C(O)-CH₂CH₃, F₃C-C(O)-(CH₂)-C(O)-CH₂CH₃, Cl₃C-C(O)-(CH₂)-C(O)-CH₂CH₃, Br₃C-C(O)-(CH₂)-C(O)-CH₂CH₃, I₃C-C(O)-(CH₂)-C(O)-CH₂CH₃, F₃C-CF₂-C(O)-(CH₂)-C(O)-CH₂CH₃, Cl₃C-CCl₂-C(O)-(CH₂)-C(O)-CH₂CH₃, H₃C-(CH₂)₂-C(O)-(CH₂)-C(O)-(CH₂)₂CH₂, (H₃C)₂CH-C(O)-(CH₂)-C(O)-(CH₂)₂CH₂, H₃C-(CH₂)₃-C(O)-(CH₂)-C(O)-(CH₂)₂CH₂, H₃C-CH₂-CH(CH₃)-C(O)-(CH₂)-C(O)-(CH₂)₂CH₂, (H₃C)₂CH-CH₂-C(O)-(CH₂)-C(O)-(CH₂)₂CH₂, (H₃C)₃C-C(O)-(CH₂)-C(O)-(CH₂)₂CH₂, F₃C-C(O)-(CH₂)-C(O)-(CH₂)₂CH₂, Cl₃C-C(O)-(CH₂)-C(O)-(CH₂)₂CH₂, Br₃C-C(O)-(CH₂)-C(O)-(CH₂)₂CH₂, I₃C-C(O)-(CH₂)-C(O)-(CH₂)₂CH₂, F₃C-CF₂-C(O)-(CH₂)-C(O)-(CH₂)₂CH₂, Cl₃C-CCl₂-C(O)-(CH₂)-C(O)-(CH₂)₂CH₂, (H₃C)₂CH-C(O)-(CH₂)-C(O)-CH(CH₃)₂, H₃C-(CH₂)₃-C(O)-(CH₂)-C(O)-CH(CH₃)₂, H₃C-CH₂-CH(CH₃)-C(O)-(CH₂)-C(O)-CH(CH₃)₂, (H₃C)₂CH-CH₂-C(O)-(CH₂)-C(O)-CH(CH₃)₂, (H₃C)₃C-C(O)-(CH₂)-C(O)-CH(CH₃)₂, F₃C-C(O)-(CH₂)-C(O)-CH(CH₃)₂, Cl₃C-C(O)-(CH₂)-C(O)-CH(CH₃)₂, Br₃C-C(O)-(CH₂)-C(O)-CH(CH₃)₂, I₃C-C(O)-(CH₂)-C(O)-CH(CH₃)₂, F₃C-CF₂-C(O)-(CH₂)-C(O)-CH(CH₃)₂, Cl₃C-CCl₂-C(O)-(CH₂)-C(O)-CH(CH₃)₂, H₃C-(CH₂)₃-C(O)-(CH₂)-C(O)-(CH₂)₃CH₃, H₃C-CH₂-CH(CH₃)-C(O)-(CH₂)-C(O)-(CH₂)₃CH₃, (H₃C)₂CH-CH₂-C(O)-(CH₂)-C(O)-(CH₂)₃CH₃, (H₃C)₃C-C(O)-(CH₂)-C(O)-(CH₂)₃CH₃, F₃C-C(O)-(CH₂)-C(O)-(CH₂)₃CH₃, Cl₃C-C(O)-(CH₂)-C(O)-(CH₂)₃CH₃, Br₃C-C(O)-(CH₂)-C(O)-(CH₂)₃CH₃, I₃C-C(O)-(CH₂)-C(O)-(CH₂)₃CH₃, F₃C-CF₂-C(O)-(CH₂)-C(O)-(CH₂)₃CH₃, Cl₃C-CCl₂-C(O)-(CH₂)-C(O)-(CH₂)₃CH₃, H₃C-CH₂-CH(CH₃)-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH(CH₃)CH₂CH₃, F₃C-C(O)-(CH₂)-C(O)-CH(CH₃)CH₂CH₃, Cl₃C-C(O)-(CH₂)-C(O)-CH(CH₃)CH₂CH₃, Br₃C-C(O)-(CH₂)-C(O)-CH(CH₃)CH₂CH₃, I₃C-C(O)-(CH₂)-C(O)-CH(CH₃)CH₂CH₃, F₃C-CF₂-C(O)-(CH₂)-C(O)-CH(CH₃)CH₂CH₃, Cl₃C-CCl₂-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH₂CH(CH₃)₂, F₃C-C(O)-(CH₂)-C(O)-CH₂CH(CH₃)₂, Cl₃C-C(O)-(CH₂)-C(O)-CH₂CH(CH₃)₂, Br₃C-C(O)-(CH₂)-C(O)-CH₂CH(CH₃)₂, I₃C-C(O)-(CH₂)-C(O)-CH₂CH(CH₃)₂, F₃C-CF₂-C(O)-(CH₂)-C(O)-CH₂CH(CH₃)₂, Cl₃C-CCl₂-C(O)-(CH₂)-C(O)-CH₂CH(CH₃)₂, (H₃C)₃C-C(O)-(CH₂)-C(O)-C(CH₃)₃, F₃C-C(O)-(CH₂)-C(O)-C(CH₃)₃, Cl₃C-C(O)-(CH₂)-C(O)-C(CH₃)₃, Br₃C-C(O)-(CH₂)-C(O)-C(CH₃)₃, I₃C-C(O)-(CH₂)-C(O)-C(CH₃)₃, F₃C-CF₂-C(O)-(CH₂)-C(O)-C(CH₃)₃, Cl₃C-CCl₂-C(O)-(CH₂)-C(O)-C(CH₃)₃, F₃C-C(O)-(CH₂)-C(O)-CF₃, Cl₃C-C(O)-(CH₂)-C(O)-CF₃, Br₃C-C(O)-(CH₂)-C(O)-CF₃, I₃C-C(O)-(CH₂)-C(O)-CF₃, F₃C-CF₂-C(O)-(CH₂)-C(O)-CF₃, Cl₃C-CCl₂-C(O)-(CH₂)-C(O)-CF₃, Cl₃C-C(O)-(CH₂)-C(O)-CCl₃, Br₃C-C(O)-(CH₂)-C(O)-CCl₃, I₃C-C(O)-(CH₂)-C(O)-CCl₃, F₃C-CF₂-C(O)-(CH₂)-C(O)-CCl₃, Cl₃C-CCl₂-C(O)-(CH₂)-C(O)-CCl₃, Br₃C-C(O)-(CH₂)-C(O)-CBr₃, I₃C-C(O)-(CH₂)-C(O)-CBr₃, F₃C-CF₂-C(O)-(CH₂)-C(O)-CBr₃, Cl₃C-CCl₂-C(O)-(CH₂)-C(O)-CBr₃, I₃C-C(O)-(CH₂)-C(O)-Cl₃, F₃C-CF₂-C(O)-(CH₂)-C(O)-Cl₃, Cl₃C-CCl₂-C(O)-(CH₂)-C(O)-Cl₃, CF₂CF₃-C(O)-(CH₂)-C(O)-CF₂CF₃, CCl₂CCl₃-C(O)-(CH₂)-C(O)-CF₂CF₃, CCl₂CCl₃-C(O)-(CH₂)-C(O)-CCl₂CCl₃, H₃C-C(O)-(CH₂)₂-C(O)-CH₃, H₃C-CH₂-C(O)-(CH₂)₂-C(O)-CH₃, H₃C-(CH₂)₂-C(O)-(CH₂)₂-C(O)-CH₃, (H₃C)₂CH-C(O)-(CH₂)₂-C(O)-CH₃, H₃C-(CH₂)₃-C(O)-(CH₂)₂-C(O)-CH₃, H₃C-CH₂-CH(CH₃)-C(O)-(CH₂)₂-C(O)-CH₃, (H₃C)₂CH-CH₂-C(O)-(CH₂)₂-C(O)-CH₃, (H₃C)₃C-C(O)-(CH₂)₂-C(O)-CH₃, F₃C-C(O)-(CH₂)₂-C(O)-CH₃, Cl₃C-C(O)-(CH₂)₂-C(O)-CH₃, Br₃C-C(O)-(CH₂)₂-C(O)-CH₃, I₃C-C(O)-(CH₂)₂-C(O)-CH₃, F₃C-CF₂-C(O)-(CH₂)₂-C(O)-CH₃, Cl₃C-CCl₂-C(O)-(CH₂)₂-C(O)-CH₃, H₃C-CH₂-C(O)-(CH₂)₂-C(O)-CH₂CH₃, H₃C-(CH₂)₂-C(O)-(CH₂)₂-C(O)-CH₂CH₃, (H₃C)₂CH-C(O)-(CH₂)₂-C(O)-CH₂CH₃, H₃C-(CH₂)₃-C(O)-(CH₂)₂-C(O)-CH₂CH₃, H₃C-CH₂-CH(CH₃)-C(O)-(CH₂)₂-C(O)-CH₂CH₃, (H₃C)₂CH-CH₂-C(O)-(CH₂)₂-C(O)-CH₂CH₃, (H₃C)₃C-C(O)-(CH₂)₂-C(O)-CH₂CH₃, F₃C-C(O)-(CH₂)₂-C(O)-CH₂CH₃, Cl₃C-C(O)-(CH₂)₂-C(O)-CH₂CH₃, Br₃C-C(O)-(CH₂)₂-C(O)-CH₂CH₃, I₃C-C(O)-(CH₂)₂-C(O)-CH₂CH₃, F₃C-CF₂-C(O)-(CH₂)₂-C(O)-CH₂CH₃, Cl₃C-CCl₂-C(O)-(CH₂)₂-C(O)-CH₂CH₃, H₃C-(CH₂)₂-C(O)-(CH₂)₂-C(O)-(CH₂)₂CH₂, (H₃C)₂CH-C(O)-(CH₂)₂-C(O)-(CH₂)₂CH₂, H₃C-(CH₂)₃-C(O)-(CH₂)₂-C(O)-(CH₂)₂CH₂, H₃C-CH₂-CH(CH₃)-C(O)-(CH₂)₂-C(O)-(CH₂)₂CH₂, (H₃C)₂CH-CH₂-C(O)-(CH₂)₂-C(O)-(CH₂)₂CH₂, (H₃C)₃C-C(O)-(CH₂)₂-C(O)-(CH₂)₂CH₂, F₃C-C(O)-(CH₂)₂-C(O)-(CH₂)₂CH₂, Cl₃C-C(O)-(CH₂)₂-C(O)-(CH₂)₂CH₂, Br₃C-C(O)-(CH₂)₂-C(O)-(CH₂)₂CH₂, I₃C-C(O)-(CH₂)₂-C(O)-(CH₂)₂CH₂, F₃C-CF₂-C(O)-(CH₂)₂-C(O)-(CH₂)₂CH₂, Cl₃C-CCl₂-C(O)-(CH₂)₂-C(O)-(CH₂)₂CH₂, (H₃C)₂CH-C(O)-(CH₂)₂-C(O)-CH(CH₃)₂, H₃C-(CH₂)₃-C(O)-(CH₂)₂-C(O)-CH(CH₃)₂, H₃C-CH₂-CH(CH₃)-C(O)-(CH₂)₂-C(O)-CH(CH₃)₂, (H₃C)₂CH-CH₂-C(O)-(CH₂)₂-C(O)-CH(CH₃)₂, (H₃C)₃C-C(O)-(CH₂)₂-C(O)-CH(CH₃)₂, F₃C-C(O)-(CH₂)₂-C(O)-CH(CH₃)₂, Cl₃C-C(OHCH₂)₂-C(O)-CH(CH₃)₂, Br₃C-C(O)-(CH₂)₂-C(O)-CH(CH₃)₂, I₃C-C(O)-(CH₂)₂-C(O)-CH(CH₃)₂, F₃C-CF₂-C(O)-(CH₂)₂-C(O)-CH(CH₃)₂, Cl₃C-CCl₂-C(O)-(CH₂)₂-C(O)-CH(CH₃)₂, H₃C-(CH₂)₃-C(OHCH₂)₂-C(O)-(CH₂)₃CH₃, H₃C-CH₂-CH(CH₃)-C(O)-(CH₂)₂-C(O)-(CH₂)₃CH₃, (H₃C)₂CH-CH₂-C(O)-(CH₂)₂-C(O)-(CH₂)₃CH₃, (H₃C)₃C-C(O)-(CH₂)₂-C(O)-(CH₂)₃CH₃, F₃C-C(O)-(CH₂)₂-C(O)-(CH₂)₃CH₃, Cl₃C-C(O)-(CH₂)₂-C(O)-(CH₂)₃CH₃, Br₃C-C(O)-(CH₂)₂-C(O)-(CH₂)₃CH₃, I₃C-C(O)-(CH₂)₂-C(O)-(CH₂)₃CH₃, F₃C-CF₂-C(O)-(CH₂)₂-C(O)-(CH₂)₃CH₃, Cl₃C-CCl₂-C(O)-(CH₂)₂-C(O)-(CH₂)₃CH₃, H₃C-CH₂-CH(CH₃)-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH(CH₃)CH₂CH₃, F₃C-C(O)-(CH₂)₂-C(O)-CH(CH₃)CH₂CH₃, Cl₃C-C(O)-(CH₂)₂-C(O)-CH(CH₃)CH₂CH₃, Br₃C-C(O)-(CH₂)₂-C(O)-CH(CH₃)CH₂CH₃, I₃C-C(O)-(CH₂)₂-C(O)-CH(CH₃)CH₂CH₃, F₃C-CF₂-C(O)-(CH₂)₂-C(O)-CH(CH₃)CH₂CH₃, Cl₃C-CCl₂-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH₂CH(CH₃)₂, F₃C-C(O)-(CH₂)₂-C(O)-CH₂CH(CH₃)₂, Cl₃C-C(O)-(CH₂)₂-C(O)-CH₂CH(CH₃)₂, Br₃C-C(O)-(CH₂)₂-C(O)-CH₂CH(CH₃)₂, I₃C-C(O)-(CH₂)₂-C(O)-CH₂CH(CH₃)₂, F₃C-CF₂-C(O)-(CH₂)₂-C(O)-CH₂CH(CH₃)₂, Cl₃C-CCl₂-C(O)-(CH₂)₂-C(O)-CH₂CH(CH₃)₂, (H₃C)₃C-C(O)-(CH₂)₂-C(O)-C(CH₃)₃, F₃C-C(O)-(CH₂)₂-C(O)-C(CH₃)₃, Cl₃C-C(O)-(CH₂)₂-C(O)-C(CH₃)₃, Br₃C-C(O)-(CH₂)₂-C(O)-C(CH₃)₃, I₃C-C(O)-(CH₂)₂-C(O)-C(CH₃)₃, F₃C-CF₂-C(O)-(CH₂)₂-C(O)-C(CH₃)₃, Cl₃C-CCl₂-C(O)-(CH₂)₂-C(O)-C(CH₃)₃, F₃C-C(O)-(CH₂)₂-C(O)-CF₃, Cl₃C-C(O)-(CH₂)₂-C(O)-CF₃, Br₃C-C(O)-(CH₂)₂-C(O)-CF₃, I₃C-C(O)-(CH₂)₂-C(O)-CF₃, F₃C-CF₂-C(O)-(CH₂)₂-C(O)-CF₃, Cl₃C-CCl₂-C(O)-(CH₂)₂-C(O)-CF₃, Cl₃C-C(O)-(CH₂)₂-C(O)-CCl₃, Br₃C-C(O)-(CH₂)₂-C(O)-CCl₃, I₃C-C(O)-(CH₂)₂-C(O)-CCl₃, F₃C-CF₂-C(O)-(CH₂)₂-C(O)-CCl₃, Cl₃C-CCl₂-C(O)-(CH₂)₂-C(O)-CCl₃, Br₃C-C(O)-(CH₂)₂-C(O)-CBr₃, I₃C-C(O)-(CH₂)₂-C(O)-CBr₃, F₃C-CF₂-C(O)-(CH₂)₂-C(O)-CBr₃, Cl₃C-CCl₂-C(O)-(CH₂)₂-C(O)-CBr₃, I₃C-C(O)-(CH₂)₂-C(O)-Cl₃, F₃C-CF₂-C(O)-(CH₂)₂-C(O)-Cl₃, Cl₃C-CCl₂-C(O)-(CH₂)₂-C(O)-Cl₃, CF₂CF₃-C(O)-(CH₂)₂-C(O)-CF₂CF₃, CCl₂CCl₃-C(O)-(CH₂)₂-C(O)-CF₂CF₃, CCl₂CCl₃-C(O)-(CH₂)₂-C(O)-CCl₂CCl₃, enthalten.

Vorzugsweise werden die Verbindung(en) der Formel (Ia) innerhalb engerer Mengenbereiche eingesetzt. Hier sind erfindungsgemäße Mittel zum Färben und/oder Aufhellen keratinischer Fasern bevorzugt, die die Verbindung(en) der Formel (Ia) in Mengen von 0,1 bis 12,5 Gew.-%, vorzugsweise von 0,25 bis 10 Gew.-%, besonders bevorzugt von 0,5 bis 7,5 Gew.-% und insbesondere von 1 bis 5 Gew.-%, jeweils bezogen auf das gesamte Mittel, enthalten.

Die erfindungsgemäßen Mittel enthalten zusätzlich Wasserstoffperoxid. Hier sind erfindungsgemäße Mittel zum Färben und Aufhellen keratinischer Fasern besonders bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Das Wasserstoffperoxid kann auch in Form dessen Anlagerungsverbindungen an feste Träger eingesetzt werden, bevorzugt wird Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Die erfindungsgemäßen Mittel können zusätzlich weitere Oxidationsmittel enthalten, die auch als "Booster" bezeichnet werden. Die Auswahl dieser weiteren Peroxoverbindungen unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten. Hier sind erfindungsgemäße Mittel zum Färben und/oder Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, -peroxomonosulfaten und -peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Die erfindungsgemäßen Mittel sind Färbemittel, d.h. Mittel zur Veränderung der Farbe keratinischer Fasern. Unter diesen sind insbesondere die so genannten Oxidationsfärbemittel bevorzugt. Erfindungsgemäße Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten. Erfindungsgemäß bevorzugte Mittel zum Färben und/oder Aufhellen keratinischer Fasern sind demnach dadurch gekennzeichnet, daß sie zusätzlich - bezogen auf ihr Gewicht - 0,001 bis 5 Gew.-% eines oder mehrerer Oxidationsfarbstoffvorprodukte und/oder direktziehender Farbstoffe enthalten.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 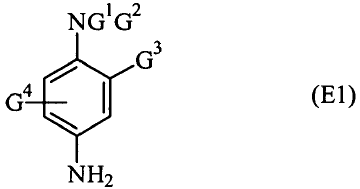 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (E2) entsprechen, sowie ihre physiologisch verträglichen Salze: 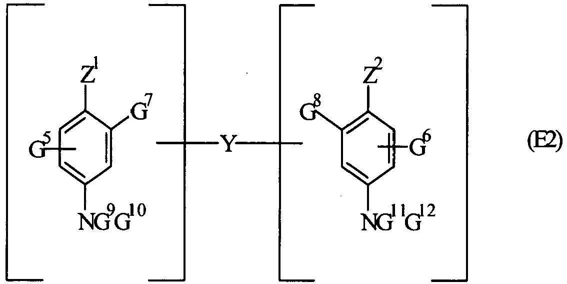 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
   mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 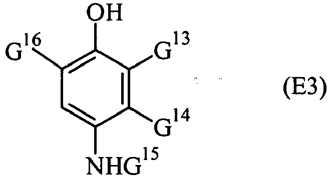 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 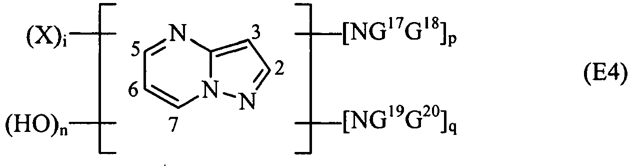 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁-bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄- Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁-bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

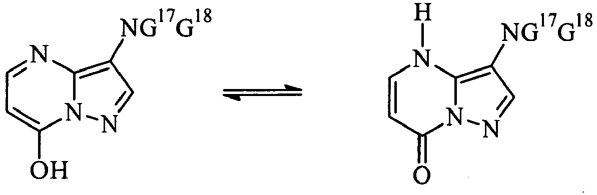

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß besonders bevorzugte Mittel zum Färben und/oder Aufhellen keratinischer Fasern sind dadurch gekennzeichnet, daß sie als Oxidationsfarbstoffvorprodukt mindestens eine Entwicklerkomponente enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol.

Die erfindungsgemäßen Färbemittel können weiterhin mindestens eine Kupplerkomponente enthalten.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

### Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (RN-1a), 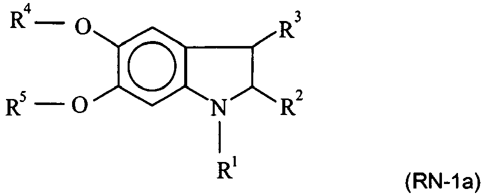 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (RN-1b), 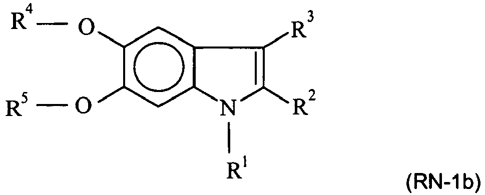 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Erfindungsgemäß besonders bevorzugte Mittel zum Färben und/oder Aufhellen keratinischer Fasern sind dadurch gekennzeichnet, daß sie als Oxidationsfarbstoffvorprodukt mindestens eine Kupplerkomponente enthalten, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Zusätzlich können die Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole, Triphenylmethanfarbstoffe, saure Farbstoffe, basische Farbstoffe, vorzugsweise aus der Gruppe der unter den internationalen Bezeichnungen bzw. Handelsnamen HC Yellow 2, HC Yellow 4, HC Yellow 5, HC Yellow 6, HC Yellow 12, HC Yellow 13, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, Acid Red 92, HC Red BN, Pigment Red 57:1, HC Blue 2, HC Blue 12, Disperse Blue 3, Disperse Red 17, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol, 3-(2',6'-Diaminopyridyl-3'-azo)-pyridin; 2-((4-(Ethyl(2-hydroxyethy1)-amino)-2-methylpheny1)azo)-5-nitro-1,3-thiazol; NI N-Di(2-hydroxyethy1)-3-methyl-4-((4-nitropheny1)azo)-anilin; 3-Diethylamino-7-(4-dimethylaminophenylaz0)-5-phenyl-phenaziniumchlorid; 4-(2-Thiazolylazo)-resorcin;4-(((4-Phenylamino)azo)benzosulfonsäure-natriumsalz; 1 -((3-Aminopropyl)amino)-9,IO-anthracendion; 3',3",4,5,5',5",6,7-Octabromphenolsulfonphtalein;1-((4-Amino-3,5-dimethylpheny1)-(2,6-dichlorphenyl)methylen)-3,5-dimethyl-4-imino-2,5-cyclohexadien-Phosphorsäure(1 :I) (Basic Blue 77); 3',3",5',5"-Tetrabrom-m-kresolsulfonphthalein;2,4-Dinitro-I -naphthol-7-sulfonsäure-Dinatriumsalz;4-[2'-Hydroxy-1 '-naphthyl)azo]-benzolsulfonsäure-Natriumsalz;3',6'-Dihydroxy-2',41I51I71-tetraiodospiro-[isobenzo-furan-I (3H), g1(9H)-xanthenl-3-on-Dinatriumsalz; 6-Hydroxy-5-((2-methoxy-5-methyl-4-sulfophenyl)azo)-2-Naphthalin-sulfonsäure-dinatriumsalz; 2,4-Dinitro-Inaphthol-Natriumsalz; 2',4',5',7'-tetrabrom-4151617-tetrachlor-3'16'-dihydroxy-Spiro[isobenzofuran-I (3H),9'[9H]xanthen]-3-on-dinatriumsalz;4-(2-Hydroxy-1-naphthylazo)-3-methyl-benzolsulfonsäurenatriumsalz; Basic Orange 31, Basic Blue 6, Basic Blue 7, Basic Blue 9, Basic Blue 26, Basic Blue 41, Basic Blue 99, Basic Brown 4, Basic, Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 ,Basic Yellow 57, Basic Yellow 87, Basic Red 46.

Erfindungsgemäß bevorzugte Mittel zum Färben und/oder Aufhellen keratinischer Fasern sind dadurch gekennzeichnet, daß sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Basic Yellow 87, Basic Orange 31 und Basic Red 51 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

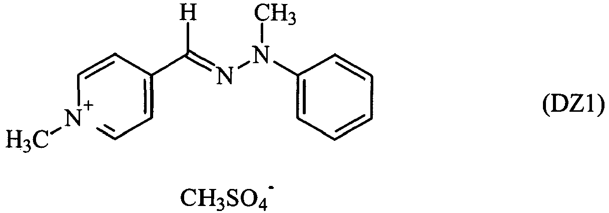

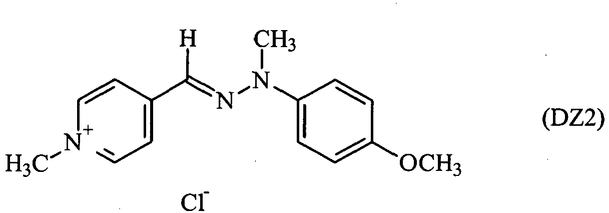

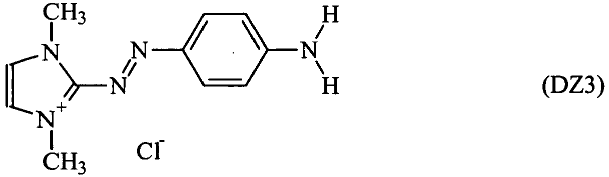

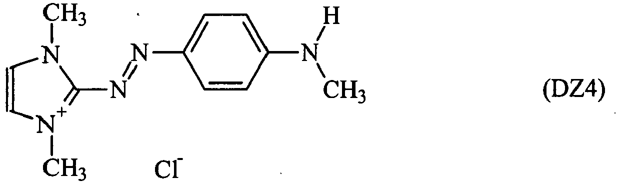

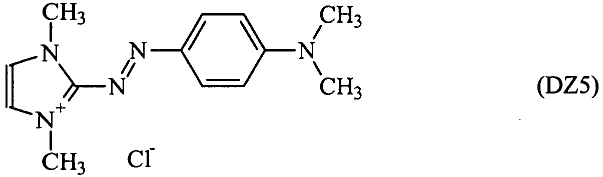

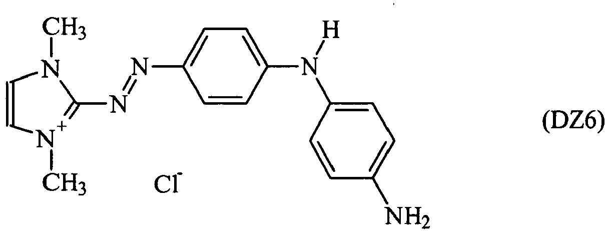

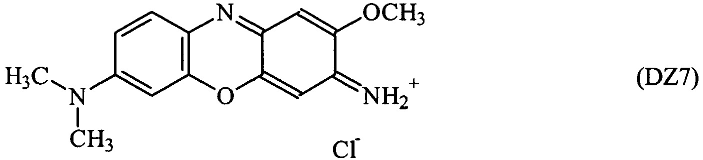

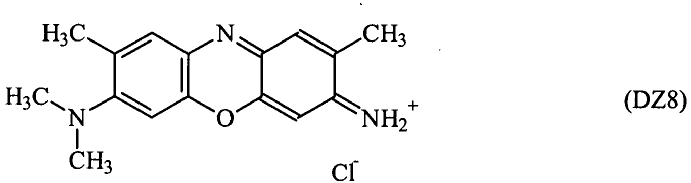

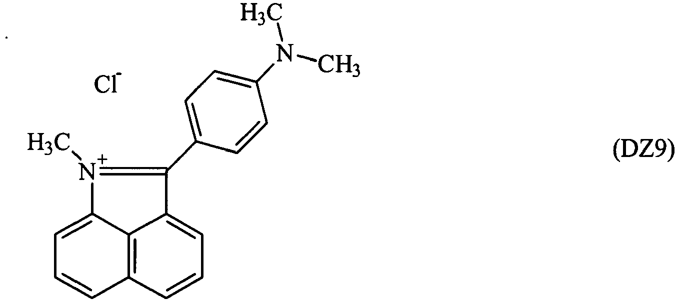

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Eine Färbung keratinischer Fasern kann auch mittels Farbstoffen erfolgen, die in einer oxidativ katalysierten Reaktion von C,H-aciden Verbindungen mit reaktiven Carbonylverbindungen gebildet werden.

In einer weiteren Ausführungsform enthält das erfindungsgemäße Mittel daher eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung
c) von Wasser unter Bildung von Hydraten als Additionsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxyacetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylaminobenzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylin-dolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formyl-benzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methyl-pyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinotinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, - tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- oder Aminosubstituenten, als reaktive Carbonylverbindung verwendet. Dabei werden wiederum die Verbindungen gemäß Formel (Ca-1) bevorzugt, 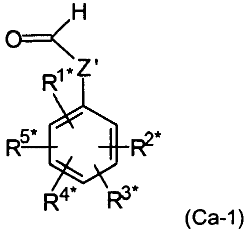 worin
- R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, eine Carbamoylgruppe, eine C₂-C₆-Acylgruppe oder eine Nitrogruppe,
- Z' steht für eine direkte Bindung oder eine Vinylengruppe,
- R^{4*} und R^{5*} stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente C werden besonders bevorzugt ausgewählt aus bestimmten Aldehyden. Hier sind erfindungsgemäße Mittel bevorzugt, die zusätzlich mindestens eine reaktive Carbonylverbindung enthalten, die ausgewählt wird, aus der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benza!dehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer reaktive Carbonylverbindung(en) enthalten.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Geeignete Verbindungen mit primärer oder sekundärer Aminogruppe als Komponente B sind z.B. primäre aromatische Amine wie N,N-Dimethyl-, N,N-Diethyl-, N- (2- Hydroxyethyl)-N-ethyl-, N,N-Bis-(2-hydroxyethyl)-, N-(2-Methoxyethyl-), 2,3-, 2,4-, 2,5- Dichlor-p-phenylendiamin, 2-Chlor-p-phenylendiamin, 2,5-Dihydroxy-4- morpholinoanilin- dihydrobromid, 2-, 3-, 4-Aminophenol, 2-Aminomethyl-4-aminophenoi, 2- Hydroxymethyl- 4-aminophenoi, o-, p-Phenylendiamin, o-Toluyiendiamin, 2,5-Diaminotoluol, -phenot, - phenethol, 4-Amino-3-methylphenoi, 2-(2,5-Diaminophenyl)-ethanoi, 2,4- Diaminophen- oxyethanoi, 2-(2,5-Diaminophenoxy)-ethanol, 4-Methylamino-, 3-Amino-4- (2'- hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2- hydroxyethylamino)-, 6-Methyl-3-amino-2-chlor-, 2-Methyl-5-amino-4-chlor-, 3, 4-Methylendi- oxy-, 5-(2-Hydroxyethylamino)-4-methoxy-2-methyl-, 4-Amino-2- hydroxymethylphenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicyisäure, 3- Amino-4-hydroxy-, 4-Amino-3-hydroxybenzoesäure, 2-, 3-, 4-Aminobenzolsulfonsäure, 3-Amino- 4- hydroxybenzolsulfonsäure, 4-Amino-3-hydroxynaphthalin-1 -sulfonsäure, 6- Amino-7- hydroxynaphthalin-2-sulfonsäure, 7-Amino-4-hydroxynaphthalin-2- sulfonsäure, 4-Amino- 5-hydroxynaphthalin-2,7-disulfonsäure, 3-Amino-2-naphthoesäure, 3- Aminophthalsäure, 5-Aminoisophthalsäure, 1,3,5-, 1,2,4-Triaminobenzol, 1,2,4,5-Tetraaminobenzol, 2,4,5- Triaminophenol, Pentaaminobenzol, Hexaaminobenzol, 2,4,6-Triaminoresorcin, 4,5- Diaminobrenzcatechin, 4,6-Diaminopyrogaliol, 3,5-Diamino-4-hydroxybrenzcatechin, aromatische Aniline bzw. Phenole mit einem weiteren aromatischen Rest, wie sie in der nachstehenden Formel dargestellt sind 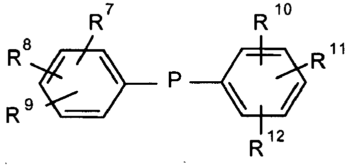 in der
- R⁷ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁸, R⁹, R¹⁰, R¹¹ und R¹² unabhängig voneinander für ein Wasserstoffatom, eine Hydroxy- oder eine Aminogruppe, die durch C₁-C₄-Alkyl-, C₁-C₄-Hydroxyalkyl, C₁-C₄-Alkoxy-, C₁-C₄-Aminoalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl- oder Iminogruppe, ein Sauerstoff- oder Schwefelatom, oder eine Gruppe mit der Formel

   -Q'-(CH₂-Q-CH₂-Q")ₒ-

   in der
   - Q eine direkte Bindung, eine CH₂- oder CHOH-Gruppe bedeutet,
   - Q' und Q" unabhängig voneinander für ein Sauerstoffatom, eine NR¹³-Gruppe, worin R¹³ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)_{p'}-O, worin p und p' 2 oder 3 sind, stehen und
   - o eine Zahl von 1 bis 4 bedeutet,
   wie beispielsweise 4,4'-Diaminostilben, 4,4'-Diaminostilben-2,2'- disulfonsäure-mono- oder -di-Na-Salz, 4-Amino-4'-dimethylaminostilben, 4,4'- Diaminodiphenylmethan, -sulfid, -sulfoxid, -amin, 4,4'-Diaminodiphenyiamin-2-sulfonsäure, 4,4'- Diaminobenzophenon, - diphenylether, 3,3',4,4'-Tetraaminodiphenyl, 3,3',4,4'-Tetraamino- benzophenon, 1,3-Bis- (2,4-diaminophenoxy)-propan, 1,8-Bis-(2,5-diaminophenoxy)-3,6-dioxaoctan, 1,3-Bis-(4- aminophenylamino)-propan, -2-propanoi, 1,3-Bis-[N-(4-aminophenyl)-2- hydroxyethyla- mino]-2-propanol, N,N-Bis-[2-(4-aminophenoxy)-ethyl]-methylamin, N- Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2- Amino-3-hydroxy-, 2,6-Diamino-, 2,5-Diamino-, 2,3-Diamino-, 2- Dimethylamino-5-amino-, 2-Methylamino-3-amino-6-methoxy-, 2,3-Diamino-6-methoxy-, 2,6-Dimethoxy- 3,5-dia- mino-, 2,4,5-Triamino-, 2,6-Dihydroxy-3,4-dimethylpyridin, 2,4-Dihydroxy- 5,6-diamino-, 4,5,6-Triamino-, 4-Hydroxy-2,5,6-triamino-, 2-Hydroxy-4,5,6-triamino-, 2, 4,5,6-Tetraamino-, 2-Methylamino-4,5,6-triamino-, 2,4-, 4,5-Diamino-, 2-Amino-4- methoxy-6-methyl- pyrimidin, 3,5-Diaminopyrazol, -1,2,4-triazol, 3-Amino-, 3-Amino-5- hydroxypyrazol, 2-,3-, 8-Aminochinolin, 4-Amino-chinaidin, 2-, 6-Aminonicotinsäure, 5-Aminoisochinolin, 5-, 6- Aminoindazol, 5-, 7-Amino-benzimidazol, -benzothiazol, 2,5-Dihydroxy-4-morpholinoani- lin sowie Indole und Indoline, sowie deren physiologisch verträgliche Salze, sein. Bevorzugte Beispiele für Indol- bzw. Indolinderivate 5,6-Dihydroxyindol, N- Methyl-5,6-dihydroxyindol, N-Ethyl-5,6-dihydroxyindol, N-Propyl-5,6-dihydroxyindol, N- Butyl-5,6-dihy- droxyindol, 5,6-Dihydroxyindol-2-carbonsäure, 6-Hydroxyindol, 6- Aminoindol und 4-Aminoindol. Weiterhin bevorzugt sind 5,6-Dihydroxyindolin, N-Methyl-5,6- dihydroxyindolin, N-Ethyl-5,6-dihydroxyindolin, N-Propyl-5,6-dihydroxyindolin, N-Butyl-5,6- dihydroxyindolin, 5,6-Dihydroxyindolin-2-carbonsäure. 6-Hydroxyindolin, 6-Aminoindolin und 4-Aminoindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen bevorzugt alle natürlich vorkommenden und synthetischen (alpha-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Es können aber auch andere Aminosäuren verwendet werden, wie z. B. 6- Aminocapronsäure.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5- Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2- Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4- Chlorresorcin, 1-Naphthol, 1,5-, 2,3-, 2,7-Dihydroxynaphthalin, 6- Dimethylamino-4- hydroxy-2-naphthalinsulfonsäure, 3,6-Dihydroxy-2,7-naphthalinsulfonsäure.

Als CH-aktive Verbindungen können beispielhaft genannt werden 1,2,3,3- Tetramethyl- 3H-indoliumiodid, 1,2,3,3-Tetramethyl-3H-indolium-p-toluoisulfonat, 1,2, 3,3-Tetramethyl- 3H-indoliummethansulfonat, Fischersche Base (1,3,3-Trimethyl-2- methylenindolin), 2,3- Dimethyl-benzothiazoliumiodid, 2,3-Dimethyl-benzothiazolium-p- toluolsulfonat, Rhodanin, Rhodanin-3-essigsäure, 1 -Ethyl-2-chinaldiniumiodid, 1 -Methyl-2- chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Bevorzugt werden die CH-aciden Verbindungen aus den Formeln (II) und/oder (III) und/oder (IV) ausgewählt 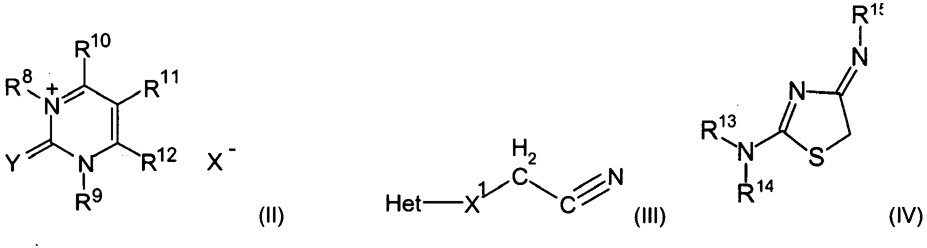 worin
- R⁸ und R⁹ stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine C₁-C₆-Alkylgruppe bedeutet,
- R¹¹ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
- R¹³ und R¹⁴ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6, und
- R¹⁵ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
- X⁻ steht für ein physiologisch verträgliches Anion,
- Het steht für einen gegebenenfalls substituierten Heteroaromaten,
- X¹ steht für eine direkte Bindung oder eine Carbonylgruppe.

Gleichwirkend zu den Verbindungen der Formel II sind deren Enaminformen. Es wird hier ausdrücklich auf die Druckschrift WO-A1-2004/022016 verwiesen, auf die vollinhaltlich Bezug genommen wird.

Mindestens eine Gruppe R¹⁰ oder R¹² gemäß Formel II steht zwingend für eine C₁-C₆-Alkylgruppe. Diese Alkylgruppe trägt an deren alpha-Kohlenstoffatom bevorzugt mindestens zwei Wasserstoffatome. Besonders bevorzugte Alkylgruppen sind die Methyl-, Ethyl-, Propyl-, n-Butyl-, isoButyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y für ein Sauerstoff- oder ein Schwefelatom, besonders bevorzugt für ein Sauerstoffatom.

Der Rest R⁸ wird bevorzugt ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

R¹¹ steht bevorzugt für ein Wasserstoffatom.

Besonders bevorzugt stehen die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

Vorzugsweise sind die Verbindungen gemäß Formel II ausgewählt aus einer oder mehrerer Verbindungen der Gruppe von Salzen mit physiologisch verträglichem Gegenion X⁻, die gebildet wird aus Salzen des
1 ,2-Dihydro-1 ,3,4,6-tetramethyl-2-oxo-pyrimidiniums,
1 ,2-Dihydro-1 ,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

Ganz besonders bevorzugte erfindungsgemäße Mittel sind dadurch gekennzeichnet, dass sie als CH-acide Verbindung
Salze des 1,2-Dihydro-1,3,4,6-tetramethyl-2-oxopyrimidiniums,
Salze des 1,2-Dihydro-1,3,4-trimethyl-2-oxopyrimidiniums,
Salze des 1,2-Dihydro-1,3,4,6-tetramethyl-2-thioxopyrimidiniums,
Salze des 1-Allyl-1,2-dihydro-3,4,6-trimethyl-2-oxopyrimidiniums,
Salze des 1,2-Dihydro-1-(2-hydroxyethyl)-3,4,6-trimethyl-2-oxopyrimidiniums,
2-(Cyanomethyl)benzimidazol,
4,5-Dihydro-4-imino-2-(1-piperidinyl)-thiazol und/oder dessen Hydrochlorid,
4,5-Dihydro-4-imino-2-(4-morpholinyl)-thiazol und/oder dessen Hydrochlorid,
4,5-Dihydro-4-imino-2-(1-pyrrolidinyl)-thiazol und/oder dessen Hydrochlorid
enthalten.

X⁻ steht in Formel (II) sowie in obigen Listen bevorzugt für Halogenid, Benzolsulfonat, p-Toluolsulfonat, C₁-C₄-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, lodid, Hydrogensulfat oder p-Toluolsulfonat als X⁻eingesetzt.

Der Rest Het gemäß Formel (III) steht bevorzugt für das Molekülfragment mit der Formel (V), 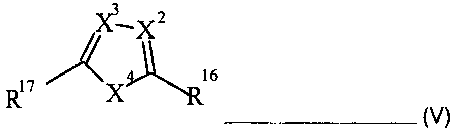 worin
- R¹⁶ und R¹⁷ stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{VIII}R^{IX}N-(CH₂)ₘ-, worin R^{VIII} und R^{IX} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{VIII} und R^{IX} gemeinsam mit dem Stickstoffatom einen 5-, 6- oder 7-gliedrigen Ring bilden können und m steht für eine Zahl 0, 1, 2, 3 oder 4, wobei R¹⁶ und/oder R¹⁷ einen an den Ring des Restmoleküls ankondensierten, gegebenenfalls substituierten aromatischen oder heteroaromatischen, 5- oder 6-Ring bilden können
- X² und X³ stehen unabhängig voneinander für ein Stickstoffatom oder eine Gruppe CR¹⁵, wobei R¹⁵ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{X}R^{XI}N-(CH₂)ₙ-, worin R^{X} und R^{XI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{X} und R^{XI} gemeinsam mit dem Stickstoffatom einen 5-, 6- oder 7-gliedrigen Ring bilden können und n steht für eine Zahl 0, 1, 2, 3 oder 4, wobei mindestens einer der Substituenten X² und X³ zusammen mit dem Restmolekül einen ankondensierten gegebenenfalls substituierten aromatischen 5- oder 6-Ring bilden kann,
- X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylguppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{XII}R^{XIII}N-(CH₂)ₚ- steht, worin R^{XII} und R^{XIII} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄₋alkylgruppe, wobei R^{XII} und R^{XIII} gemeinsam mit dem Stickstoffatom einen 5-, 6- oder 7-gliedrigen Ring bilden können und p steht für eine Zahl 0, 1, 2, 3 oder 4, substituiert sein können,

mit der Maßgabe, daß, wenn X⁴ für eine Vinylengruppe steht, mindestens eine der Gruppen X² oder X³ ein Stickstoffatom bedeutet.

Die Bindung des heterozyklischen Rings gemäß Formel (V) zum Molekülfragment -X¹-CH₂-C≡N unter Erhalt der erfindungsgemäßen Verbindung gemäß Formel (III) erfolgt an den Ring des Heterozyklusses und ersetzt ein an diesen Ring gebundenes Wasserstoffatom. Folglich ist es zwingend notwendig, daß die Substituenten R¹⁶, R¹⁷, X², X³ und X⁴ derart gewählt werden müssen, daß mindestens einer dieser Substituenten eine entsprechende Bindungsbildung ermöglicht. Folglich ist es zwingend, daß mindestens einer der Reste R¹⁶ oder R¹⁷ die Bindung zum Molekülfragment -X¹-CH₂-C≡N ausbildet, wenn X⁴ ein Sauerstoffatom oder ein Schwefelatom ist und X² und X³ ein Stickstoffatom bedeuten.

Der Rest Het gemäß Formel (III) wird besonders bevorzugt abgeleitet von den Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Benzothiazol, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hydroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise sind die Verbindungen gemäß Formel (III) ausgewählt aus der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril. Besonders bevorzugt ist 1*H*-Benzimidazol-2-ylacetonitril [2-(Cyanomethyl)benzimidazol].

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer CH-acider Verbindung(en) enthalten.

Es hat sich gezeigt, daß insbesondere der Einsatz bestimmter nichtionischer Verbindungen die aktivierende Wirkung der Dione synergistisch unterstützen kann. In erfindungsgemäß bevorzugten Mitteln zum Färben und/oder Aufhellen keratinischer Fasern sind daher zusätzlich - bezogen auf das Gewicht des Mittels - 0,1 bis 20 Gew.-%, vorzugsweise 1 bis 10 Gew.-% und insbesondere 2,5 bis 12,5 Gew.-% mindestens einer nichtionischen Verbindung der allgemeinen Formel (Ni-I) 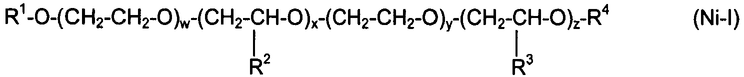 enthalten, worin
- R¹: steht für -H oder einen geradkettigen oder verzweigten, gesättigten oder ein-bzw. mehrfach ungesättigten C₆₋₂₄-Alkyl- oder -Alkenylrest,
- R² und R³: stehen unabhängig voneinander für -CH₃; -CH₂CH₃, -CH₂CH₂-CH₃, CH(CH₃)₂
- R⁴: steht für -H oder einen geradkettigen oder verzweigten, gesättigten oder ein-bzw. mehrfach ungesättigten C₁₋₄-Alkyl- oder -Alkenylrest, vorzugsweise für-H, -CH₃; -CH₂CH₃, -CH₂CH₂CH₃, CH(CH₃)₂, -(CH₂)₃CH₃, -CH₂-CH(CH₃)₂, CH(CH₃)CH₂CH₃, -C(CH₃)₃
- w, x, y, z: stehen unabhängig voneinander für Zahlen von 0 bis 50, vorzugsweise von 0 bis 20, weiter bevorzugt von 0 bis 10 und insbesondere für 0, 1, 2, 3, 4, 5, 6

In der vorstehend genannten Formel (Ni-I) steht R¹ für ein Wasserstoffatom oder einen geradkettigen oder verzweigten, gesättigten oder ein- bzw. mehrfach ungesättigten C₆₋₂₄-Alkyl- oder -Alkenylrest. Mit besonderem Vorzug steht R¹ für eine C₆₋₂₄-Alkylgruppe, oder eine C₆₋₂₄-Alkylengruppe. Unter diesen Gruppen haben sich die Reste, die sich von Fettsäuren nativen Ursprungs ableiten, als besonders geeignet erwiesen.

Bevorzugte Carbonsäuren, von denen sich der Rest R¹ ableiten kann, sind beispielsweise Hexansäure (Capronsäure), Heptansäure (Önanthsäure), Octansäure (Caprylsäure), Nonansäure (Pelargonsäure), Decansäure (Caprinsäure), Undecansäure usw., wie dies aus den vorstehend genannten bevorzugten Verbindungen hervorgeht. Bevorzugt ist im Rahmen der vorliegenden Verbindung der Einsatz von Fettsäuren wie Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure), Tetracosansäure (Lignocerinsäure), Hexacosansäure (Cerotinsäure), Triacotarsäure (Melissinsäure) sowie der ungesättigten Sezies 9c-Hexadecensäure (Palmitoleinsäure), 6c-Octadecensäure (Petroselinsäure), 6t-Octadecensäure (Petroselaidinsäure), 9c-Octadecensäure (Ölsäure), 9t-Octadecensäure ((Elaidinsäure), 9c,12c-Octadecadiensäure (Linolsäure), 9t,12t-Octadecadiensäure (Linolaidinsäure) und 9c,12c,15c-Octadecatreinsäure (Linolensäure). Aus Kostengründen ist es bevorzugt, nicht die reinen Spezies einzusetzen, sondern technische Gemische der einzelnen Säuren, wie sie aus der Fettspaltung zugänglich sind. Solche Gemische sind beispielsweise Koskosölfettsäure (ca. 6 Gew.-% C₈, 6 Gew.-% C₁₀, 48 Gew.-% C₁₂, 18 Gew.-% C₁₄, 10 Gew.-% C₁₆, 2 Gew.-% C₁₈, 8 Gew.-% C_{18'}, 1 Gew.-% C_{18"}), Palmkernölfettsäure (ca. 4 Gew.-% C₈, 5 Gew.-% C₁₀, 50 Gew.-% C₁₂, 15 Gew.-% C₁₄, 7 Gew.-% C₁₆, 2 Gew.-% C₁₈, 15 Gew.-% C_{18'}, 1 Gew.-% C_{18"}), Talgfettsäure (ca. 3 Gew.-% C₁₄, 26 Gew.-% C₁₆, 2 Gew.-% C_{16'}, 2 Gew.-% C₁₇, 17 Gew.-% C₁₈, 44 Gew.-% C_{18'}, 3 Gew.-% C_{18"}, 1 Gew.-% C_{18"'}), gehärtete Talgfettsäure (ca. 2 Gew.-% C₁₄, 28 Gew.-% C₁₆, 2 Gew.-% C₁₇, 63 Gew.-% C₁₈, 1 Gew.-% C_{18'}), technische Ölsäure (ca. 1 Gew.-% C₁₂, 3 Gew.-% C₁₄, 5 Gew.-% C₁₆, 6 Gew.-% C_{16'}, 1 Gew.-% C₁₇, 2 Gew.-% C₁₈, 70 Gew.-% C_{18'}, 10 Gew.-% C_{18"}, 0,5 Gew.-% C_{18"'}), technische Palmitin/Stearinsäure (ca. 1 Gew.-% C₁₂, 2 Gew.-% C₁₄, 45 Gew.-% C₁₆, 2 Gew.-% C₁₇, 47 Gew.-% C₁₈, 1 Gew.-% C_{18'}) sowie Sojabohnenölfettsäure (ca. 2 Gew.-% C₁₄, 15 Gew.-% C₁₆, 5 Gew.-% C₁₈, 25 Gew.-% C_{18'}, 45 Gew.-% C_{18"}, 7 Gew.-% C_{18"'}).

Besonders bevorzugte Reste R¹ sind ausgewählt aus -CH₃, -CH₂CH₃, -(CH)₂-CH₃, -CH(CH₃)₂, -(CH₂)₃CH₃, -(CH₂)₄CH₃, -(CH₂)₅CH₃, -(CH₂)₆CH₃, -(CH₂)₇CH₃, -(CH₂)₉CH₃, -(CH₂)₁₁CH₃, -(CH₂)₁₃CH₃, -(CH₂)₁₅CH₃, -(CH₂)₁₇CH₃, -(CH₂)₁₉CH₃, cis -(CH₂)₈-CH=CH-(CH₂)₅-CH₃, *cis* -(CH₂)₈-CH=CH-(CH₂)₇-CH₃, *cis* -(CH₂)₁₂-CH=CH-(CH₂)₇₋CH₃, cis,cis -(CH₂)₈-CH=CH-(CH₂)-CH=CH-(CH₂)₄-CH₃, *cis,cis,cis-*(CH₂)₈-CH=CH-(CH₂)-CH=CH-(CH₂)-CH=CH-(CH₂)-CH₃, *cis,cis,cis,cis* -(CH₂)₄-CH=CH-(CH₂)-CH=CH-(CH₂)-CH=CH-(CH₂)-CH=CH-(CH₂)₄-CH₃.

Die Reste R² und R³ stehen unabhängig voneinander für -CH₃,-CH₂CH₃, -CH₂CH₂CH₃, -CH(CH₃)₂. Besonders bevorzugte Reste R² und R³ stehen für -CH₃.

Der Rest R⁴ steht für -H oder einen geradkettigen oder verzweigten, gesättigten oder ein- bzw. mehrfach ungesättigten C₁₋₄-Alkyl- oder -Alkenylrest, vorzugsweise für -H, -CH₃; -CH₂CH₃,-CH₂CH₂CH₃, CH(CH₃)₂, -(CH₂)₃CH₃, -CH₂-CH(CH₃)₂, CH(CH₃)CH₂CH₃, -C(CH₃)₃.

Insbesondere im Falle der nichtionischen Tenside (R¹ ≠ -H) kann R⁴ für einen kurzkettigen Alkylrest, vorzugsweise für -CH₃; -CH₂CH₃, -CH₂CH₂CH₃, CH(CH₃)₂, -(CH₂)₃CH₃, -CH₂-CH(CH₃)₂, CH(CH₃)CH₂CH₃, -C(CH₃)₃, stehen, Formel (III) steht dann für so genannte "endgruppenverschlossene" Niotenside.

Erfindungsgemäß einsetzbar sind zum einen Polyalkylenglycole, d.h. Verbindungen der Formel (Ni-I), bei denen R¹ für -H steht. Je nach Wahl der Indices y, x, y, z gelangt man zu reinen Polethylenglycolen bzw. zu reinen Polypropylenglycolen bzw. zu gemischten Vertretern.

Als Polyalkylenglycole kommen insbesondere Polyethylenglycole und Polypropylenglycole in Betracht. Wenn neben R¹ auch R⁴ für -H steht, und die Indices x und z jeweils für 0 stehen, vereinfacht sich die allgemeine Formel (Ni-I) zu (Ni-Ia)

H-(O-CH₂-CH₂)_{(w+y)}-OH (Ni-Ia)

wobei (w+y) erfindungsgemäß Werte zwischen 1 (Ethylenglycol) und 100 annehmen kann. Für Polyethylenglycole existieren verschiedene Nomenklaturen, die zu Verwirrungen führen können. Technisch gebräuchlich ist die Angabe des mittleren relativen Molgewichts im Anschluß an die Angabe "PEG", so daß "PEG 200" ein Polyethylenglycol mit einer relativen Molmasse von ca. 190 bis ca. 210 charakterisiert. Für kosmetische Inhaltsstoffe wird eine andere Nomenklatur verwendet, in der das Kurzzeichen PEG mit einem Bindestrich versehen wird und direkt an den Bindestrich eine Zahl folgt, die der Zahl (w+y) in der oben genannten Formel (Ni-Ia) entspricht. Nach dieser Nomenklatur sind beispielsweise PEG-6, PEG-8, PEG-10, PEG-12, PEG-14, PEG-16, PEG-18, PEG-20, PEG-22, PEG-24, PEG-26, PEG-28 und PEG-30 einsetzbar. Kommerziell erhältlich sind Polyethylenglycole beispielsweise unter den Handelnamen Carbowax® (Union Carbide), Emkapol® (ICI Americas), Lipoxol® (HÜLS America), Polyglycol® (Dow Chemical), Alkapol® (Rhone-Poulenc), Lutrol® (BASF).

Besonders geeignet für die Aktivierung von Wasserstoffperoxid sind folgende Verbindungen: H-(O-CH₂-CH₂)-OH, H-(O-CH₂-CH₂)₂-OH, H-(O-CH₂-CH₂)₃-OH, H-(O-CH₂-CH₂)₄--OH, H-(O-CH₂-CH₂)₃-OH, H-(O-CH₂-CH₂)₆-OH, H-(O-CH₂-CH₂)₇-OH, H-(O-CH₂-CH₂)₆-OH, H-(O-CH₂-CH₂)₉-OH, H-(O-CH₂-CH₂)₁₀-OH, H-(O-CH₂-CH₂)₁₁-OH, H-(O-CH₂-CH₂)₁₂-OH, H-(O-CH₂-CH₂)₁₃-OH, H-(O-CH₂-CH₂)₁₄-OH, H-(O-CH₂-CH₂)₁₅-OH, H-(O-CH₂-CH₂)₁₆-OH, H-(O-CH₂-CH₂)₁₇-OH, H-(O-CH₂-CH₂)₁₈-OH, H-(O-CH₂-CH₂)₁₉-OH, H-(O-CH₂-CH₂)₂₆-OH, H-(O-CH₂-CH₂)₂₁-OH, H-(O-CH₂-CH₂)₂₂-OH, H-(O-CH₂-CH₂)₂₃-OH, H-(O-CH₂-CH₂)₂₄-OH, H-(O-CH₂-CH₂)₂₅-OH, H-(O-CH₂-CH₂)₂₆-OH, H-(O-CH₂-CH₂)₂₇-OH, H-(O-CH₂-CH₂)₂₈-OH, H-(O-CH₂CH₂)₂₉-OH, H-(O-CH₂-CH₂)₃₀-OH, H-(O-CH₂-CH₂)₃₁-OH, H-(O-CH₂-CH₂)₃₂-OH, H-(O-CH₂-CH₂)₃₃-OH, H-(O-CH₂-CH₂)₃₄-OH, H-(O-CH₂-CH₂)₃₆-OH, H-(O-CH₂-CH₂)₃₆-OH, H-(O-CH₂-CH₂)₃₇-OH, H-(O-CH₂-CH₂)₃₈-OH, H-(O-CH₂-CH₂)₃₉-OH, H-(O-CH₂-CH₂)₃₄-OH, H-(O-CH₂-CH₂)₄₁-OH, H-(O-CH₂-CH₂)₄₂-OH, H-(O-CH₂-CH₂)₄₃-OH, H-(O-CH₂-CH₂)₄₄-OH, H-(O-CH₂-CH₂)₄₆-OH, H-(O-CH₂-CH₂)₄₆-OH, H-(O-CH₂-CH₂)₄₇-OH, H(-O-CH₂-CH₂)₄₈-OH, H-(O-CH₂-CH₂)₄₉-OH, H-(O-CH₂-CH₂)₅₀-OH, H-(O-CH₂-CH₂)₅₁-OH, H-(O-CH₂-CH₂)₅₂-OH, H-(O-CH₂-CH₂)₅₃-OH, H-(O-CH₂-CH₂)₅₄-OH, H-(O-CH₂-CH₂)₅₅-OH, H-(O-CH₂-CH₂)₅₆-OH, H-(O-CH₂-CH₂)₅₇-OH, H-(O-CH₂-CH₂)₅₈-OH, H-(O-CH₂-CH₂)₅₉-OH, H-(O-CH₂-CH₂)₆₀-OH, H-(O-CH₂-CH₂)₆₁-OH, H-(O-CH₂-CH₂)₆₂-OH, H-(O-CH₂-CH₂)₆₃-OH, H-(O-CH₂-CH₂)₆₄-OH, H-(O-CH₂-CH₂)₆₅-OH, H-(O-CH₂-CH₂)₆₆-OH, H-(O-CH₂-CH₂)₆₇-OH, H-(O-CH₂-CH₂)₆₈-OH, H-(O-CH₂-CH₂)₆₉-OH, H-(O-CH₂-CH₂)₇₀-OH, H-(O-CH₂-CH₂)₇₁-OH, H-(O-CH₂-CH₂)₇₂-OH, H-(O-CH₂-CH₂)₇₃-OH, H-(O-CH₂-CH₂)₇₄-OH, H-(O-CH₂-CH₂)₇₅-OH, H-(O-CH₂-CH₂)₇₆-OH, H-(O-CH₂-CH₂)₇₇-OH, H-(O-CH₂-CH₂)₇₈-OH, H-(O-CH₂-CH₂)₇₉-OH, H-(O-CH₂-CH₂)₈₀-OH, H-(O-OH₂-CH₂)₈₁-OH, H-(O-CH₂-CH₂)₈₂-OH, H-(O-CH₂-CH₂)-OH.

Wenn neben R¹ auch R⁴ für -H steht, die Indices w und y jeweils für 0 stehen und R² bzw. R³ für Methyl stehen, vereinfacht sich die allgemeine Formel (Ni-I) zu (Ni-Ib) und man gelangt zu den Polypropylenglycolen (Kurzzeichen PPG) 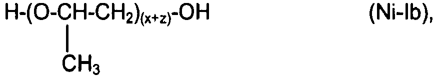 wobei (x+z) erfindungsgemäß Werte zwischen 1 (Propylenglycol) und 100 annehmen kann. Technisch bedeutsam sind hier insbesondere Di-, Tri- und Tetrapropylenglycol, d.h. die Vertreter mit n=2, 3 und 4 in Formel Ni-Ib.

Besonders geeignet für die Aktivirung von Wasserstoffperoxid sind folgende Verbindungen: H-(O-CH(CH₃)-CH₂)-OH, H-(O-CH(CH₃)-CH₂)₂-OH, H-(O-CH(CH₃)-CH₂)₃-OH, H-(O-CH(CH₃)-CH₂)₄--OH, H-(O-CH(CH₃)-CH₂)₅-OH, H-(O-CH(CH₃)-CH₂)₆-OH, H-(O-CH(CH₃)-CH₂)₇-OH, H-(O-CH(CH₃)-CH₂)₈-OH, H-(O-CH(CH₃)-CH₂)₉-OH, H-(O-CH(CH₃)-CH₂)₁₀-OH, H-(O-CH(CH₃)-CH₂)₁₁-OH, H-(O-CH(CH₃)-CH₂)₁₂-OH, H-(O-CH(CH₃)-CH₂)₁₃-OH, H-(O-CH(CH₃)-CH₂)₁₄-OH, H-(O-CH(CH₃)-CH₂)₁₅-OH, H-(O-CH(CH₃)-CH₂)₁₆-OH, H-(O-CH(CH₃)-CH₂)₁₇-OH, H-(O-CH(CH₃)-CH₂)₁₈-OH, H-(O-CH(CH₃)-CH₂)₁₉-OH, H-(O-CH(CH₃)-CH₂)₂₀-OH, H-(O-CH(CH₃)-CH₂)₂₁-OH, H-(O-CH(CH₃)-CH₂)₂₂-OH, H-(O-CH(CH₃)-CH₂)₂₃-OH, H-(O-CH(CH₃)-CH₂)₂₄-OH, H-(O-CH(CH₃)-CH₂)₂₅-OH, H-(O-CH(CH₃)-CH₂)₂₆-OH, H-(O-CH(CH₃)-CH₂)₂₇-OH, H-(O-CH(CH₃)-CH₂)₂₈-OH, H-(O-CH(CH₃)-CH₂)₂₉-OH, H-(O-CH(CH₃)-CH₂)₃₀-OH, H-(O-CH(CH₃)-CH₂)₃₁-OH, H-(O-CH(CH₃)-CH₂)₃₂-OH, H-(O-CH(CH₃)-CH₂)₃₃-OH, H-(O-CH(CH₃)-CH₂)₃₄-OH, H-(O-CH(CH₃)-CH₂)₃₅-OH, H-(O-CH(CH₃)-CH₂)₃₆-OH, H-(O-CH(CH₃)-CH₂)₃₇-OH, H-(O-CH(CH₃)-CH₂)₃₈-OH, H-(O-CH(CH₃)-CH₂)₃₉-OH, H-(O-CH(CH₃)-CH₂)₃₄-OH, H-(O-CH(CH₃)-CH₂)₄₁-OH, H-(O-CH(CH₃)-CH₂)₄₂-OH, H-(O-CH(CH₃)-CH₂)₄₃-OH, H-(O-CH(CH₃)-CH₂)₄₄-OH, H-(O-CH(CH₃)-CH₂)₄₅-OH. H-(O-CH(CH₃)-CH₂)₄₆-OH, H-(O-CH(CH₃)-CH₂)₄₇-OH, H-(O-CH(CH₃)-CH₂)₄₈-OH, H-(O-CH(CH₃)-CH₂)₄₉-OH, H-(O-CH(CH₃)-CH₂)₅₀-OH, H-(O-CH(CH₃)-CH₂)₅₁-OH, H-(O-CH(CH₃)-CH₂)₅₂-OH, H-(O-CH(CH₃)-CH₂)₅₃-OH, H-(O-CH(CH₃)-CH₂)₅₄-OH, H-(O-CH(CH₃)-CH₂)₅₅-OH, H-(O-CH(CH₃)-CH₂)₅₆-OH, H-(O-CH(CH₃)-CH₂)₅₇-OH, H-(O-CH(CH₃)-CH₂)₅₈-OH, H-(O-CH(CH₃)-CH₂)₅₉-OH, H-(O-CH(CH₃)-CH₂)₆₀-OH, H-(O-CH(CH₃)-CH₂)₆₁-OH, H-(O-CH(CH₃)-CH₂)₆₂-OH, H-(O-CH(CH₃)-CH₂)₆₃-OH, H-(O-CH(CH₃)-CH₂)₆₄-OH, H-(O-CH(CH₃)-CH₂)₆₅-OH, H-(O-CH(CH₃)-CH₂)₆₆-OH, H-(O-CH(CH₃)-CH₂)₆₇-OH, H-(O-CH(CH₃)-CH₂)₆₈-OH, H-(O-CH(CH₃)-CH₂)₆₉-OH, H-(O-CH(CH₃)-CH₂)₇₀-OH, H-(O-CH(CH₃)-CH₂)₇₁-OH, H-(O-CH(CH₃)-CH₂)₇₂-OH, H-(O-CH(CH₃)-CH₂)₇₃-OH, H-(O-CH(CH₃)-CH₂)₇₄-OH, H-(O-CH(CH₃)-CH₂)₇₅-OH, H-(O-CH(CH₃)-CH₂)₇₆-OH, H-(O-CH(CH₃)-CH₂)₇₇-OH, H-(O-CH(CH₃)-CH₂)₇₈-OH, H-(O-CH(CH₃)-CH₂)₇₉-OH, H-(O-CH(CH₃)-CH₂)₈₀-OH, H-(O-CH(CH₃)-CH₂)₈₁-OH, H-(O-CH(CH₃)-CH₂)₈₂-OH, H-(O-CH(CH₃)-CH₂)-OH.

Zusammenfassend sind besonders bevorzugte erfindungsgemäße Mittel dadurch gekennzeichnet, daß zusätzlich zu den Dionen nichtionische Verbindungen der Formel (Ni-I) verwendet werden, worin
- R¹: steht für -H
- R⁴: steht für -H
- x, z: stehen für 0
- w, y: stehen unabhängig voneinander für Zahlen von 0 bis 50, wobei die Summe (w + y) für Zahlen von 3 bis 80, vorzugsweise von 4 bis 60, weiter bevorzugt von 5 bis 40 und insbesondere von 6 bis 30 steht.

Steht R¹ in der allgemeinen Formel (Ni-I) nicht für -H, so repräsentiert die Formel alkoxylierte Alkohole. Wie bereits weiter oben erwähnt, haben sich die Reste, die sich von Fettsäuren nativen Ursprungs ableiten, als besonders geeignet erwiesen.

Ähnlich wie bei den Polalkylenglycolen können auch bei den Niotensiden rein ethoxylierte Vertreter, rein propoxylierte Vertreter, rein anders alkoxylierte Vertreter oder gemischt alkoxylierte Vertreter eingesetzt werden. Zudem kann je nach Wahl des Restes R⁴ eine "offene" oder endgruppenverschlossene Verbindung eingesetzt werden.

Mit besonderem Vorzug lassen sich ethoxylierte Fettalkohole einsetzen. Demnach sind erfindungsgemäß bevorzugte Mittel dadurch gekennzeichnet, daß sie zusätzlich nichtionische Verbindungen der Formel (Ni-I) enthalten, worin
- R¹: steht für für eine C₆₋₂₈-Alkylgruppe, vorzugsweise eine C₆₋₂₂-Alkylgruppe, weiter bevorzugt eine C₁₀₋₂₀-Alkylgruppe und insbesondere eine C₁₂₋₁₈-Alkylgruppe, oder eine C₆₋₂₈-Alkylengruppe, vorzugsweise eine C₈₋₂₂-Alkylengruppe, weiter bevorzugt eine C₁₀₋₂₀-Alkylengruppe und insbesondere eine C₁₂₋₁₈-Alkylengruppe, wobei bevorzugte Reste R¹ ausgewählt sind aus H₃C-(CH₂)₆-, H₃C-(CH₂)₇-, H₃C-(CH₂)₉-, H₃C-(CH₂)₁₁-, H₃C-(CH₂)₁₃-, H₃C-(CH₂)₁₅-, H₃C-(CH₂)₁₇-, H₃C-(CH₂)₁₉-, H₃C-(CH₂)₂₁-, *cis*-H₃C-(CH₂)₅-CH=CH-(CH₂)₈-, *cis*-H₃C-(CH₂)₇-CH=CH-(CH₂)₈-, *cis*-H₃C-(CH₂)₇-CH=CH-(CH₂)₁₂-, *cis,cis*-H₃C-(CH₂)₄-CH=CH-(CH₂)-CH=CH-(CH₂)₈-, *cis,cis,cis*-H₃C-(CH₂)-CH=CH-(CH₂)-CH=CH-(CH₂)-CH=CH-(CH₂)₈-, *cis,cis,cis,cis*-H₃C-(CH₂)₄-CH=CH-(CH₂)-CH=CH-(CH₂)-CH=CH-(CH₂)-CH=CH-(CH₂)₄-,
- R⁴: steht für -H oder einen geradkettigen oder verzweigten, gesättigten oder ein-bzw. mehrfach ungesättigten C₁₋₄-Alkyl- oder -Alkenylrest, vorzugsweise für-H, -CH₃; -CH₂CH₃, -CH₂CH₂CH₃, CH(CH₃)₂, -(CH₂)₃CH₃, -CH₂-CH(CH₃)₂, CH(CH₃)CH₂CH₃, -C(CH₃)₃
- x, z: stehen für 0
- w, y: stehen unabhängig voneinander für Zahlen von 0 bis 50, wobei die Summe (w + y) für Zahlen von 5 bis 90, vorzugsweise von 10 bis 80, weiter bevorzugt von 15 bis 70 und insbesondere von 30 bis 60 steht.

Die angegebenen C-Kettenlängen sowie Ethoxylierungsgrade bzw. Alkoxylierungsgrade stellen statistische Mittelwerte dar, die für ein spezielles Produkt eine ganze oder eine gebrochene Zahl sein können. Aufgrund der Herstellverfahren bestehen Handelsprodukte der genannten Formeln zumeist nicht aus einem individuellen Vertreter, sondern aus Gemischen, wodurch sich sowohl für die C-Kettenlängen als auch für die Ethoxylierungsgrade bzw. Alkoxylierungsgrade Mittelwerte und daraus folgend gebrochene Zahlen ergeben können.

Wie bereits erwähnt, lassen sich auch so genannte "endgruppenverschlossene" Niotenside erfindungsgemäß zur Aktivierung von Wasserstoffperoxid einsetzen. Als "Endgruppenverschluß" eignen sich insbesondere Methyl- oder Ethyl- oder tert-Butylreste.

Es hat sich gezeigt, daß der gleichzeitige Einsatz von Azolen zu einer Wirkungssteigerung der erfindungsgemäßen Mittel führt. Insbesondere die Aktivierung von Wasserstoffperoxid und die Verringerung der Gelbfärbung beim Blondieren werden durch den gleichzeitigen Einsatz von Azolen weiter verstärkt. Erfindungsgemässe Mittel zum Färben und Aufhellen keratinischer Fasern sind daher dadurch gekennzeichnet, daß sie - bezogen auf ihr Gewicht - 1 bis 20 Gew.-% mindestens Imidazolverbindung gemäß Formel (VII) und/oder deren physiologisch verträglichen Salzen 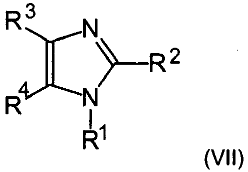 enthalten, worin
- R¹: steht für ein Wasserstoffatom, eine gegebenenfalls substituierte Arylgruppe oder eine (C₁-C₆)-Alkylgruppe,
- R²: steht für ein Wasserstoffatom, eine Carboxaldehydgruppe, eine (C₁-C₆)-Alkylgruppe oder eine Nitrogruppe,
- R³: steht für ein Wasserstoffatom, eine Carboxy-(C₁-C₆)-alkylgruppe, eine Amino-(C₁-C₆)-alkylgruppe, eine Carboxylgruppe, eine Carboxaldehydgruppe, eine (C₁-C₆)-Alkylgruppe, eine Nitrogruppe, eine 2-Amino-3-hydroxypropylgruppe oder eine Gruppe -CH₂-CH(NH₂)-COOH,
- R⁴: steht für ein Wasserstoffatom, eine Carboxaldehydgruppe oder eine Carboxylgruppe.

Vorzugsweise wird/werden die Imidazolverbindung(en) der Formel (VII) innerhalb engerer Mengenbereiche eingesetzt. Hier sind erfindungsgemäße Mittel zum Färben und/oder Aufhellen keratinischer Fasern bevorzugt, die mindestens eine Imidazolverbindung gemäß Formel (VII) und/oder deren physiologisch verträgliche Salze in Mengen von 2 bis 15 Gew.-%, vorzugsweise von 2,5 bis 12,5 Gew.-%, besonders bevorzugt von 3 bis 10 Gew.-% und insbesondere von 4 bis 8 Gew.-% enthalten.

Bevorzugt werden die Imidazolverbindungen gemäß Formel (VII) ausgewählt aus mindestens einem Vertreter aus einer Gruppe, die gebildet wird, aus Histamin, D-Histidin, L-Histidin, DL-Histidin, D-Histidinol, L-Histidinol, DL-Histidinol, Imidazol, Imidazol-4-essigsäure, Imidazol-4-carbonsäure, lmidazol-4,5-dicarbonsäure, Imidazol-2-carboxaldehyd, Imidazol-4-carboxaldehyd, Imidazol-5-carboxaldehyd, 2-Nitroimidazol, 4-Nitroimidazol, 4-Methylimidazol-5-carboxaldehyd, N-Methylimidazol-2-carboxaldehyd, 4-Methylimidazol, 2-Methylimidazol, N-Methylimidazol, N-(4-Aminophenyl)-imidazol, sowie den physiologisch verträglichen Salzen der vorgenannten Verbindungen. Erfindungsgemäß besonders bevorzugt wird Imidazol eingesetzt.

Zusammenfassend sind erfindungsgemäß bevorzugte Mittel dadurch gekennzeichnet, daß sie mindestens eine Imidazolverbindung gemäß Formel (VII) und/oder deren physiologisch verträgliche Salze in Mengen von 2 bis 15 Gew.-%, vorzugsweise von 2,5 bis 12,5 Gew.-%, besonders bevorzugt von 3 bis 10 Gew.-% und insbesondere von 4 bis 8 Gew.-% enthalten, wobei bevorzugte Imidazolverbindungen gemäß Formel (VII) und/oder deren physiologisch verträgliche Salze ausgewählt ist/sind aus Histamin, D-Histidin, L-Histidin, DL-Histidin, D-Histidinol, L-Histidinol, DL-Histidinol, Imidazol, Imidazol-4-essigsäure, lmidazol-4-carbonsäure, Imidazol-4,5-dicarbonsäure, Imidazol-2-carboxaldehyd, Imidazol-4-carboxaldehyd, Imidazol-5-carboxaldehyd, 2-Nitroimidazol, 4-Nitroimidazol, 4-Methylimidazol-5-carboxaldehyd, N-Methylimidazol-2-carboxaldehyd, 4-Methylimidazol, 2-Methylimidazol, N-Methylimidazol, N-(4-Aminophenyl)-imidazol, sowie den physiologisch verträglichen Salze der vorgenannten Verbindungen.

Da die "Booster" hydrolytisch leicht zersetzt werden, ist es bevorzugt, diese der Oxidationsmittelzubereitung bzw. der Anwendungsmischung erst unmittelbar vor der Anwendung zuzugeben.

Als zusätzliches Alkalisierungsmittel können die erfindungsgemäßen Mittel mindestens eine Verbindung aus Aminosäuren und/oder Oligopeptiden enthalten, wobei diese jeweils mindestens zwei Aminogruppen und mindestens eine -COOH- oder -SO₃H-Gruppe aufweisen und deren 2,5-%ige Lösung in Wasser jeweils einen pH-Wert von größer 9,0 aufweist.

Bevorzugte Alkalisierungsmittel sind Aminocarbonsäuren, insbesondere α-Aminocarbonsäuren und omega-Aminocarbonsäuren. Unter den α-Aminocarbonsäuren sind wiederum Lysin und insbesondere Arginin besonders bevorzugt.

Besagte Aminosäuren können den erfindungsgemäßen Mitteln bevorzugt in freier Form zugegeben werden. In einer Reihe von Fällen ist es jedoch auch möglich, die Aminosäuren in Salzform einzusetzen. Bevorzugte Salze sind dann die Verbindungen mit Halogenwasserstoffsäuren, insbesondere die Hydrochloride und die Hydrobromide.

Weiterhin können die Aminosäuren auch in Form von Oligopeptiden und Proteinhydrolysaten eingesetzt werden, wenn sichergestellt ist, daß die erforderlichen Mengen der erfindungsgemäß eingesetzten Aminosäuren darin enthalten sind.

Ein besonders bevorzugtes Alkalisierungsmittel ist Arginin.

Erfindungsgemäß bevorzugte Mittel sind dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanin und/oder Valin und/oder Lysin und/oder Leucin und/oder Threonin und/oder Serin enthalten.

Die erfindungsgemäßen wasserfreien Zusammensetzungen können zusätzlich mindestens einen weiteren Bleichverstärker, der von den anorganischen Persalzen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Aufhellleistung können erfindungsgemäße wasserfreie Mittel zusätzlich als Bleichverstärker mindestens eine gegebenenfalls hydratisierte SiO₂-Verbindung enthalten. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Erfindungsgemäß bevorzugte Mittel sind dadurch gekennzeichnet, daß sie zusätzlich 0,05 bis 15 Gew.-%, besonders bevorzugt 0,15 bis 10 Gew.-% und insbesondere 0,2 bis 5 Gew.-% mindestens einer gegebenenfalls hydratisierte SiO₂-Verbindung enthalten, wobei bevorzugte Mittel Wassergläser enthalten, die aus einem Silikat der Formel (SiO₂)ₙ(Na₂O)ₘ(K₂O)ₚ gebildet werden, worin n steht für eine positive rationale Zahl und m und p unabhängig voneinander für eine positive rationale Zahl oder für 0 stehen, mit den Maßgaben, daß mindestens einer der Parameter m oder p von 0 verschieden ist und das Verhältnis zwischen n und der Summe aus m und p zwischen 1:4 und 4:1 liegt.

Zusätzlich zu den genannten Inhaltsstoffen hat sich der Einsatz von Carbonaten und Carbonatanaloga in den erfindungsgemäßen Mitteln als bevorzugt herausgestellt. Erfindungsgemäß bevorzugte Mittel zum Färben und/oder Aufhellen keratinischer Fasern enthalten zusätzlich 0,1 bis 25 Gew.%, vorzugsweise 0,5 bis 20 Gew.%, besonders bevorzugt 1 bis 15 Gew.% und insbesondere 1,5 bis 10 Gew.% mindestens eines Stoffes aus den Gruppen der
i. Carbonate und/oder
ii. Monoalkylcarbonate (Kohlensäuremonoester) und/oder
iii. Kohlensäuremonoamide und/oder
iv. Silylcarbonate und/oder
v. Silylcarbamate
Als Carbonate bieten sich insbesondere Ammonium-, Alkalimetall- und Erdalkalimetallcarbonate und -hydrogencarbonate an. Besonders bevorzugt sind beispielsweise Natriumhydrogencarbonat, Ammoniumhydrogencarbonat, Natriumcarbonat, Ammoniumcarbonat und Ammoniumcarbamat.

Zusätzlich zu Carbonaten oder an deren Stelle können die erfindungsgemäßen Mittel vorzugsweise auch mindestens einen Kohlensäuremonoester und/oder mindestens ein Kohlensäuremonoamid enthalten. Diese Substanzen werden nachfolgend detailliert beschrieben.

Erfindungsgemäß bevorzugte Mittel sind dadurch gekennzeichnet, daß sie mindestens einen Kohlensäuremonoester der Formel (C-I)

R-O-C(O)-O-H (C-I)

enthalten, in der R für einen gesättigten oder ungesättigten, geradkettigen, verzweigten, oder cyclischen, substituierten oder unsubstituierten Kohlenwasserstoffrest, oder eine substituierte oder unsubstiuierte Arylgruppe bzw. einen substituierten oder unsubstituierten Heterocyclus steht.

In Formel (C-I) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Mittel sind dadurch gekennzeichnet, daß der Rest R in Formel (C-I) ausgewählt ist aus Methyl-, Ethyl-, n-Propyl-, iso-Propyl-, n-Butyl-, iso-Butyl-, *tert*-Butylsowie Hydroxymethyl- und Hydroxyethyl-Resten.

Alternativ zum Kohlensäuremonoester oder in Verbindung mit ihm können die erfindungsgemäßen Mittel Kohlensäuremonoamide enthalten. Hier sind erfindungsgemäß bevorzugte Mittel dadurch gekennzeichnet, daß sie mindestens ein Kohlensäuremonoamid der Formel (C-II)

R-NH-C(O)-O-H (C-II)

enthält, in der R für einen gesättigten oder ungesättigten, geradkettigen, verzweigten, oder cyclischen, substituierten oder unsubstituierten Kohlenwasserstoffrest, oder eine substituierte oder unsubstiuierte Arylgruppe bzw. einen substituierten oder unsubstituierten Heterocyclus steht.

In Formel (C-II) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Mittel sind dadurch gekennzeichnet, daß der Rest R in Formel (C-I) ausgewählt ist aus Methyl-, Ethyl-, n-Propyl-, iso-Propyl-, n-Butyl-, iso-Butyl-, *tert*-Butylsowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäße Mittel bevorzugt, die den Kohlensäuremonoester bzw. das Kohlensäuremonoamid in ganz oder teilweise neutralisierter Form, vorzugsweise in Form seines Alkalimetall-, Ammonium-, Erdalkalimetall- oder Aluminiumsalzes und insbesondere in Form seines Natriumsalzes, enthalten.

Unabhängig davon, ob die erfindungsgemäßen Mittel einen oder mehrere Kohlensäuremonoester und/oder ein oder mehrere Kohlensäuremonoamide enthalten, sind erfindungsgemäße Mittel bevorzugt, die den bzw. die Kohlensäuremonoester und/oder Kohlensäuremonoamide in Mengen von 0,1 bis 20 Gew.%, vorzugsweise von 0,5 bis 18 Gew.%, besonders bevorzugt von 2 bis 15 Gew.% und insbesondere von 5 bis 12 Gew.%, jeweils bezogen auf das gesamte Mittel, enthalten.

Zusätzlich zu Carbonaten und/oder Kohlensäuremonoestern und/oder Kohlensäuremonoamiden oder an deren Stelle können die erfindungsgemäßen Mittel vorzugsweise auch mindestens ein Silylcarbonat und/oder mindestens ein Silylcarbamat enthalten. Diese Substanzen werden nachfolgend detailliert beschrieben.

Erfindungsgemäß bevorzugte Mittel sind dadurch gekennzeichnet, daß sie mindestens ein Silylcarbonat der Formel (C-III) 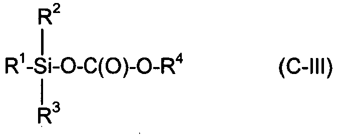 enthalten, in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Amino-, Iminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstiuierte Arylgruppe bzw. einen substituierten oder unsubstituierten Heterocyclus steht.

Bevorzugte Reste R¹, R² und R³ in der oben genannten Formel (C-III) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße Mittel dadurch gekennzeichnet sind, daß die Reste R¹, R² und R³ in Formel (C-III) ausgewählt sind aus Methyl-, Ethyl-, n-Pröpyl-, iso-Propyl-, n-Butyl-, iso-Butyl-, tert-Butyl- sowie Hydroxymethyl- und Hydroxyethyl-Resten.

Bevorzugte Reste R⁴ in der oben genannten Formel (C-III) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Das acide H-Atom des Silylcarbonats (R⁴ = -H in Formel C-III) kann auch in neutralisierter Form vorliegen, d.h. es können erfindungsgemäß auch Salze von Silylcarbonaten eingesetzt werden. Hier sind erfindungsgemäße Mittel bevorzugt, die mindestens ein Silylcarbonat in ganz oder teilweise neutralisierter Form, vorzugsweise in Form seines Alkalimetall-, Ammonium-, Erdalkalimetall- oder Aluminiumsalzes und insbesondere in Form seines Natriumsalzes, enthalten.

Zusätzlich zu den Siliylcarbonaten oder an ihrer Stelle können die erfindungsgemäßen Mittel Silylcarbamate enthalten.

Erfindungsgemäß bevorzugte Mittel sind dadurch gekennzeichnet, daß sie ein Silylcarbamat der Formel C-IV) 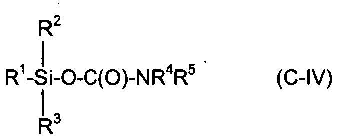 enthalten, in der enthält, in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstiuierte Arylgruppe bzw. einen substituierten oder unsubstituierten Heterocyclus stehen.

Bevorzugte Reste R¹, R² und R³ in der oben genannten Formel (C-IV) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße Mittel dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (C-IV) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Unabhängig davon, ob die erfindungsgemäßen Mittel ein oder mehrere Silylcarbonate und/oder ein oder mehrere Silylcarbamate enthalten, sind erfindungsgemäße Mittel bevorzugt, die das bzw. die Silylcarbonat(e) und/oder Silylcarbamat(e) in Mengen von 0,1 bis 25 Gew.%, vorzugsweise von 0,5 bis 20 Gew.%, besonders bevorzugt von 1 bis 15 Gew.% und insbesondere von 1,5 bis 10 Gew.%, jeweils bezogen auf das gesamte Mittel, enthalten.

Eine weitere Steigerung der Auhelleistung läßt sich durch den Zusatz von Cyanaten bzw. "Cyanatanaloga" erreichen, die eine Wirkungssteigerung ermöglichen. Bevorzugte erfindungsgemäße Mittel können daher 0,05 bis 5 Gew.-% mindestens eines Cyanats der Formel

**M⁺ [OCN]⁻**

in der M⁺ für K⁺, Na⁺, Li⁺ oder NH₄⁺ oder ½ Ca²⁺, ½ Mg²⁺ oder ½ Zn²⁺ steht und/oder
- 0,05 bis 5 Gew.-% mindestens einer Verbindung der Formel

   **H₂N-C(O)-L**

   in der L für eine durch das Anion ⁻OOH verdrängbare Gruppe steht,
enthalten

Als Cyanate können erfindungsgemäß Kaliumcyanat, KOCN, und/oder Natriumcyanat, NaOCN, und/oder Lithiumcyanat, LiOCN, und/oder Ammoniumcyanat, NH₄OCN, und/oder Calciumcyanat, Ca(OCN)₂, und/oder Magnesiumcyanat, Mg(OCN)₂, und/oder Zinkcyanat, Zn(OCN)₂, eingesetzt werden.

Erfindungsgemäß bevorzugte Mittel sind dadurch gekennzeichnet, daß sie 0,075 bis 4 Gew.-%, vorzugsweise 0,1 bis 2,5 Gew.-% und insbesondere 0,2 bis 1 Gew.-% Natriumcyanat und/oder Kaliumcyanat und/oder Ammoniumcyanat enthalten, wobei Natriumcyanat besonders bevorzugt ist.

Anstelle von Cyanaten oder in Kombination mit ihnen können auch Verbindungen der Formel H₂N-C(O)-L, in der L für eine durch das Anion ⁻OOH verdrängbare Gruppe steht, in den erfindungsgemäßen Mitteln enthalten sein. Hier sind erfindungsgemäß bevorzugte Mittel dadurch gekennzeichnet, daß sie 0,075 bis 4 Gew.-%, vorzugsweise 0,1 bis 3,5 Gew.-% und insbesondere 0,2 bis 3 Gew.-% mindestens einer Verbindung der Formel

**H₂N-C(O)-L**

enthalten, in der L ausgewählt ist aus -S-CH₂-COOH, -S-(CH₂)₃SO₃H, -S-(CH₂)₂-NH₂, -OCH₃, -O-(C₆H₄)-SO₃M, -Q⁺, -NH-OH, -O-C(O)-R¹, -O-C(O)-H, -O-CH(OH)₂, -SO₃M, -PH(O)OH, -P(O)(OH)₂, -(NH-Glucosamin)ₙ, -NH-C(O)-NH₂, -NH-NH₂, -NH-CN, -P(OH)(O)-C(O)NH₂, -SCN, -OCN, -S-C(NH)NH₂, 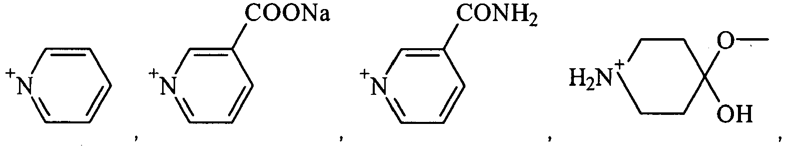 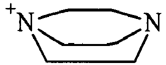 und M für -H, Na, K, NH4 steht, Q ausgewählt ist aus den aus tertiären Aminen gebildeten quartären Ammoniumresten, Heteroarylresten oder nicht-aromatischen Heterocyclen und R¹ für -H, -CH₃, - CH₂CH₃, -(CH₂)₂CH₃, -CH(CH₃)₂ oder eine andere Alkylgruppe steht.

Erfindungsgemäß besonders bevorzugte Mittel enthalten 0,075 bis 4 Gew.-%, vorzugsweise 0,1 bis 3,5 Gew.-% und insbesondere 0,2 bis 3 Gew.-% mindestens einer der nachstehend aufgeführten Verbindungen:
- H₂N-C(O)-S-CH₂-COOH
- H₂N-C(O)-S-(CH₂)₂-NH₂
- H₂N-C(O)-S-(CH₂)₃SO₃Na
- H₂N-C(O)-S-(CH₂)₃SO₃K
- H₂N-C(O)-S-(CH₂)₃SO₃NH₄
- H₂N-C(O)-SO₃H
- H₂N-C(O)-SO₃Na
- H₂N-C(O)-SO₃K
- H₂N-C(O)-SO₃NH₄
- H₂N-C(O)-O-(C₃H₄)-SO₃H
- H₂N-C(O)-O-(C₆H₄)-SO₃Na
- H₂N-C(O)-O-(C₆H₄)-SO₃K
- H₂N-C(O)-O-(C₆H₄)-SO₃NH₄
- H₂N-C(O)-O-C(O)-H
- H₂N-C(O)-O-C(O)-CH₃
- H₂N-C(O)-O-C(O)-CH₂CH₃
- H₂N-C(O)-O-C(O)-(CH₂)₂CH₃
- H₂N-C(O)-O-C(O)-CH(CH₃)₂
- H₂N-C(O)-OCH₃
- H₂N-C(O)-NH-OH
- H₂N-C(O)-O-CH(OH)₂
- H₂N-C(O)-PH(O)OH
- H₂N-C(O)-P(O)(OH)₂
- H₂N-C(O)-NH-C(O)-NH₂
- H₂N-C(O)-NH-NH₂
- H₂N-C(O)-NH-CN
- H₂N-C(O)-P(OH)(O)-C(O)NH₂
- H₂N-C(O)-SCN
- H₂N-C(O)-OCN
- H₂N-C(O)-S-C(NH)NH₂
- H₂N-C(O)-N(CH₃)₃⁺ Cl⁻
- H₂N-C(O)-N(CH₂CH₃)₃⁺ Cl⁻
- H₂N-C(O)-N((CH₂)₂CH₃)₃⁺ Cl⁻
- H₂N-C(O)-N(CH(CH₃)₂)₃⁺ Cl⁻
- H₂N-C(O)-N(C₆H₅)₃⁺ Cl⁻
- H₂N-C(O)-N(CH₂-Ph)₃⁺ Cl⁻
- H₂N-C(O)-N(CH₃)₃⁺ Br⁻
- H₂N-C(O)-N(CH₂CH₃)₃⁺ Br⁻
- H₂N-C(O)-N((CH₂)₂CH₃)₃⁺ Br⁻
- H₂N-C(O)-N(CH(CH₃)₂)₃⁺ Br⁻
- H₂N-C(O)-N(C₆H₅)₃⁺ Br⁻
- H₂N-C(O)-N(CH₂-Ph)₃⁺ Br⁻
- H₂N-C(O)-N(CH₃)₃⁺ H₃C-OSO₃⁻
- H₂N-C(O)-N(CH₂CH₃)₃⁺ H₃C-OSO₃⁻
- H₂N-C(O)-N((CH₂)₂CH₃)₃⁺ H₃C-OSO₃⁻
- H₂N-C(O)-N(CH(CH₃)₂)₃⁺ H₃C-OSO₃⁻
- H₂N-C(O)-N(C₆H₅)₃⁺ H₃C-OSO₃⁻
- H₂N-C(O)-N(CH₂-Ph)₃⁺ H₃C-OSO₃⁻
- H₂N-C(O)-N(CH₃)₃⁺ H₃C-CH₂-OSO₃⁻
- H₂N-C(O)-N(CH₂CH₃)₃⁺ H₃C-CH₂-OSO₃⁻
- H₂N-C(O)-N((CH₂)₂CH₃)₃⁺ H₃C-CH₂-OSO₃⁻
- H₂N-C(O)-N(CH(CH₃)₂)₃⁺ H₃C-CH₂-OSO₃⁻
- H₂N-C(O)-N(C₆H₅)₃⁺ H₃C-CH₂-OSO₃⁻
- H₂N-C(O)-N(CH₂-Ph)₃⁺ H₃C-CH₂-OSO₃⁻

Auch Piperidone bzw. ihre Derivate können die Effekte verstärken, so daß ein Einsatz von 0,1 bis 20 Gew.-% mindestens eines Piperidons und/oder Piperidonderivats in den erfindungsgemäßen Mitteln bevorzugt ist. Vorzugsweise enthalten die erfindungsgemäßen Mittel 0,5 bis 15 Gew.-%, besonders bevorzugt 1 bis 12,5 Gew.-% und insbesondere 2,5 bis 10 Gew.-% mindestens einer Verbindung aus der Gruppe
- 2-Piperidon und/oder
- der Derivate von 2-Piperidon, insbesondere der 2-Piperidiniumsalze und/oder
- 3-Piperidon und/oder
- der Derivate von 3-Piperidon, insbesondere der 3-Piperidiniumsalze und/oder
- 4-Piperidon und/oder
- der Derivate von 4-Piperidon, insbesondere der 4-Piperidiniumsalze

Piperidone sind Verbindungen, die eine Ketofunktion an einem Sechsring aufweisen, der aus fünf Kohlenstoffatomen und einem Stickstoffatom gebildet wird. Je nach Stellung des Stickstoffatomes zur Ketogruppe bezeichnet man den unsubstituierten Grundkörper als 2-Piperidon (Piperidin-2-on, δ-Valerolactam, alpha-Piperidon), 3-Piperidon (Piperidin-3-on, beta-Piperidon) oder 4-Piperidon (Piperidin-4-on, gamma-Piperidon).

Diese drei Piperidone sind erfindungsgemäß bevorzugt einsetzbar. Als zusätzlich zu den Piperidonen oder an ihrer Stelle erfindungsgemäß einsetzbare Piperidonderivate kommen sowohl am C-Gerüst, als auch am Stickstoffatom substituierte Piperidone in Frage, wobei die N-substituierten Piperidonderviate eine große Bedeutung haben, da sich das H-Atom am Stickstoff leicht austauschen läßt. Unter diesen Piperidonderviaten haben sich erfindungsgemäß folgende als besonders bevorzugt erwiesen:
- 1-Methyl-Piperidin-4-on (N-Methyl-4-piperidon)
- 1-Methyl-Piperidin-3-on (N-Methyl-3-piperidon)
- 1-Methyl-Piperidin-2-on (N-Methyl-2-piperidon)
- 1-Ethyl-Piperidin-4-on (N-Ethyl-4-piperidon)
- 1-Ethyl-Piperidin-3-on (N-Ethyl-3-piperidon)
- 1-Ethyl-Piperidin-2-on (N-Ethyl-2-piperidon)
- 1-Propyl-Piperidin-4-on (N-Propyl-4-piperidon)
- 1-Propyl-Piperidin-3-on (N-Propyl-3-piperidon)
- 1-Propyl-Piperidin-2-on (N-Propyl-2-piperidon)
- 1-Isopropyl-Piperidin-4-on (N-Isopropyl-4-piperidon)
- 1-Isopropyl-Piperidin-3-on (N-Isopropyl-3-piperidon)
- 1-Isopropyl-Piperidin-2-on (N-Isopropyl-2-piperidon)
- 1-Butyl-Piperidin-4-on (N-Butyl-4-piperidon)
- 1-Butyl-Piperidin-3-on (N-Butyl-3-piperidon)
- 1-Butyl-Piperidin-2-on (N-Butyl-2-piperidon)
- 1-Pentyl-Piperidin-4-on (N-Pentyl-4-piperidon)
- 1-Pentyl-Piperidin-3-on (N-Pentyl-3-piperidon)
- 1-Pentyl-Piperidin-2-on (N-Pentyl-2-piperidon)
- 1-Hexyl-Piperidin-4-on (N-Hexyl-4-piperidon)
- 1-Hexyl-Piperidin-3-on (N-Hexyl-3-piperidon)
- 1-Hexyl-Piperidin-2-on (N-Hexyl-2-piperidon)
- 1-Heptyl-Piperidin-4-on (N-Heptyl-4-piperidon)
- 1-Heptyl-Piperidin-3-on (N-Heptyl-3-piperidon)
- 1-Heptyl-Piperidin-2-on (N-Heptyl-2-piperidon)
- 1-Octyl-Piperidin-4-on (N-Octyl-4-piperidon)
- 1-Octyl-Piperidin-3-on (N-Octyl-3-piperidon)
- 1-Octyl-Piperidin-2-on (N-Octyl-2-piperidon)
- 1-Nonyl-Piperidin-4-on (N-Nonyl-4-piperidon)
- 1-Nonyl-Piperidin-3-on (N-Nonyl-3-piperidon)
- 1-Nonyl-Piperidin-2-on (N-Nonyl-2-piperidon)
- 1-Decyl-Piperidin-4-on (N-Decyl-4-piperidon)
- 1-Decyl-Piperidin-3-on (N-Decyl-3-piperidon)
- 1-Decyl-Piperidin-2-on (N-Decyl-2-piperidon)

Das N-Atom in den erfindungsgemäß vorzugsweise eingesetzten Piperidonen kann auch quaterniert vorliegen, so daß erfindungsgemäß bevorzugt auch Piperidiniumsalze eingesetzt werden können. Hier sind erfindungsgemäß besonders bevorzugte Mittel, dadurch gekennzeichnet, daß sie 3-Piperidiniumsalze der Formel (PIP-1) und/oder 4-Piperidiniumsalze der Formel (PIP-2) enthalten 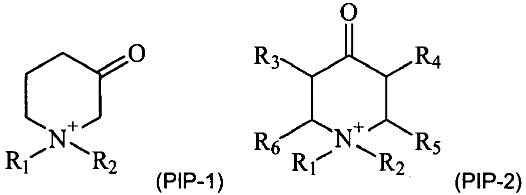 in denen die Reste R¹ bzw. R² bzw. R³ bzw. R⁴ bzw. R⁵ bzw. R⁶ unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, oder anderen Alkylresten, Alkenylresten, gegebenenfalls substituierten Arylresten und bei denen das Gegenion vorzugsweise ausgewählt ist aus Chlorid, Bromid, Methosulfat, Toluolsulfonat.

Vorzugsweise können die Reste bzw. R³ bzw. R⁴ bzw. R⁵ bzw. R⁶ ein Wasserstoffatom sein. Unter diesen Piperidiniumsalzen haben sich erfindungsgemäß folgende als besonders bevorzugt erwiesen:
- 3-oxo-Piperidinium-Chlorid (R¹ = R² = H in Formel PIP-1)
- 4-oxo-Piperidinium-Chlorid (R¹ = R² = H in Formel PIP-2)
- 3-oxo-Piperidinium-Bromid (R¹ = R² = H in Formel PIP-1)
- 4-oxo-Piperidinium-Bromid (R¹ = R² = H in Formel PIP-2)
- 3-oxo-Piperidinium-Methosulfat (R¹ = R² = H in Formel PIP-1)
- 4-oxo-Piperidinium-Methosulfat (R¹ = R² = H in Formel PIP-2)
- 3-oxo-Piperidinium-Tosylat (R¹ = R² = H in Formel PIP-1)
- 4-oxo-Piperidinium-Tosylat (R¹ = R² = H in Formel PIP-2)
- N-Methyl-3-oxo-piperidinium-Chlorid (R¹ = -CH₃, R² = H in Formel PIP-1)
- N-Methyl-4-oxo-piperidinium-Chlorid (R¹ = -CH₃, R² = H in Formel PIP-2)
- N-Methyl-3-oxo-piperidinium-Bromid (R¹ = -CH₃, R² = H in Formel PIP-1)
- N-Methyl-4-oxo-piperidinium-Bromid (R¹ = -CH₃, R² = H in Formel PIP-2)
- N-Methyl-3-oxo-piperidinium-Methosulfat (R¹ = -CH₃, R² = H in Formel PIP-1)
- N-Methyl-4-oxo-piperidinium-Methosulfat (R¹ = -CH₃, R² = H in Formel PIP-2)
- N-Methyl-3-oxo-piperidinium-Tosylat (R¹ = -CH₃, R² = H in Formel PIP-1)
- N-Methyl-4-oxo-piperidinium-Tosylat (R¹ = -CH₃, R² = H in Formel PIP-2)
- N-Ethyl-3-oxo-piperidinium-Chlorid (R¹ = -CH₂CH₃, R² = H in Formel PIP-1)
- N-Ethyl-4-oxo-piperidinium-Chlorid (R¹ = -CH₂CH₃, R² = H in Formel PIP-2)
- N-Ethyl-3-oxo-piperidinium-Bromid (R¹ = -CH₂CH₃, R² = H in Formel PIP-1)
- N-Ethyl-4-oxo-piperidinium-Bromid (R¹ = -CH₂CH₃, R² = H in Formel PIP-2)
- N-Ethyl-3-oxo-piperidinium-Methosulfat (R¹ = -CH₂CH₃, R² = H in Formel PIP-1)
- N-Ethyl-4-oxo-piperidinium-Methosulfat (R¹ = -CH₂CH₃, R² = H in Formel PIP-2)
- N-Ethyl-3-oxo-piperidinium-Tosylat (R¹ = -CH₂CH₃, R² = H in Formel PIP-1)
- N-Ethyl-4-oxo-piperidinium-Tosylat (R¹ = -CH₂CH₃, R² = H in Formel PIP-2)
- N-Propyl-3-oxo-piperidinium-Chlorid (R¹ = -CH₂CH₂CH₃, R² = H in Formel PIP-1)
- N-Propyl-4-oxo-piperidinium-Chlorid (R¹ = -CH₂CH₂CH₃, R² = H in Formel PIP-2)
- N-Propyl-3-oxo-piperidinium-Bromid (R¹ = -CH₂CH₂CH₃, R² = H in Formel PIP-1)
- N-Propyl-4-oxo-piperidinium-Bromid (R¹ = -CH₂CH₂CH₃, R² = H in Formel PIP-2)
- N-Propyl-3-oxo-piperidinium-Methosulfat (R¹ = -CH₂CH₂CH₃, R² = H in Formel PIP-1)
- N-Propyl-4-oxo-piperidinium-Methosulfat (R¹ = -CH₂CH₂CH₃, R² = H in Formel PIP-2)
- N-Propyl-3-oxo-piperidinium-Tosylat (R¹ = -CH₂CH₂CH₃, R² = H in Formel PIP-1)
- N-Propyl-4-oxo-piperidinium-Tosylat (R¹ = -CH₂CH₂CH₃, R² = H in Formel PIP-2)
- N-lsopropyl-3-oxo-piperidinium-Chlorid (R¹ = -CH(CH₃)₂, R² = H in Formel PIP-1)
- N-lsopropyl-4-oxo-piperidinium-Chlorid (R¹ = -CH(CH₃)₂, R² = H in Formel PIP-2)
- N-lsopropyl-3-oxo-piperidinium-Bromid (R¹ = -CH(CH₃)₂R² = H in Formel PIP-1)
- N-lsopropyl-4-oxo-piperidinium-Bromid (R¹ = -CH(CH₃)₂, R² = H in Formel PIP-2)
- N-Isopropyl-3-oxo-piperidinium-Methosulfat (R¹ = -CH(CH₃)₂, R² = H in Formel PIP-1)
- N-Isopropyl-4-oxo-piperidinium-Methosulfat (R¹ = -CH(CH₃)₂, R² = H in Formel PIP-2)
- N-Isopropyl-3-oxo-piperidinium-Tosylat (R¹ = -CH(CH₃)₂, R² = H in Formel PIP-1)
- N-Isopropyl-4-oxo-piperidinium-Tosylat (R¹ = -CH(CH₃)₂, R² = H in Formel PIP-2)
- N-Butyl-3-oxo-piperidinium-Chlorid (R¹ = -(CH₂)₃CH₃, R² = H in Formel PIP-1)
- N-Butyl-4-oxo-piperidinium-Chlorid (R¹ = -(CH₂)₃CH₃, R² = H in Formel PIP-2)
- N-Butyl-3-oxo-piperidinium-Bromid (R¹ = -(CH₂)₃CH₃, R² = H in Formel PIP-1)
- N-Butyl-4-oxo-piperidinium-Bromid (R¹ = -(CH₂)₃CH₃, R² = H in Formel PIP-2)
- N-Butyl-3-oxo-piperidinium-Methosulfat (R¹ = -(CH₂)₃CH₃, R² = H in Formel PIP-1)
- N-Butyl-4-oxo-piperidinium-Methosulfat (R¹ = -(CH₂)₃CH₃, R² = H in Formel PIP-2)
- N-Butyl-3-oxo-piperidinium-Tosylat (R¹ = -(CH₂)₃CH₃, R² = H in Formel PIP-1)
- N-Butyl-4-oxo-piperidinium-Tosylat (R¹ = -(CH₂)₃CH₃, R² = H in Formel PIP-2)
- N-lsobutyl-3-oxo-piperidinium-Chlorid (R¹ = -CH₂CH(CH₃)₂, R² = H in Formel PIP-1)
- N-lsobutyl-4-oxo-piperidinium-Chlorid (R¹ = -CH₂CH(CH₃)₂, R² = H in Formel PIP-2)
- N-lsobutyl-3-oxo-piperidinium-Bromid (R¹ = -CH₂CH(CH₃)₂, R² = H in Formel PIP-1)
- N-lsobutyl-4-oxo-piperidinium-Bromid (R¹ = -CH₂CH(CH₃)₂, R² = H in Formel PIP-2)
- N-lsobutyl-3-oxo-piperidinium-Methosulfat (R¹ = -CH₂CH(CH₃)₂, R² = H in Formel PIP-1)
- N-lsobutyl-4-oxo-piperidinium-Methosulfat (R¹ = -CH₂CH(CH₃)₂, R² = H in Formel PIP-2)
- N-lsobutyl-3-oxo-piperidinium-Tosylat (R¹ = -CH₂CH(CH₃)₂, R² = H in Formel PIP-1)
- N-Isobutyl-4-oxo-piperidinium-Tosylat (R¹ = -CH₂CH(CH₃)₂, R² = H in Formel PIP-2)
- N-sec-Butyl-3-oxo-piperidinium-Chlorid (R¹ = -CH₂CH(CH₃)₂, R² = H in Formel PIP-1)
- N-sec-Butyl-4-oxo-piperidinium-Chlorid (R¹ = -CH₂CH(CH₃)₂, R² = H in Formel PIP-2)
- N-sec-Butyl-3-oxo-piperidinium-Bromid (R¹ = -CH₂CH(CH₃)₂, R² = H in Formel PIP-1)
- N-sec-Butyl-4-oxo-piperidinium-Bromid (R¹ = -CH₂CH(CH₃)₂, R² = H in Formel PIP-2)
- N-sec-Butyl-3-oxo-piperidinium-Methosulfat (R¹ = -CH₂CH(CH₃)₂, R² = H in Formel PIP-1)
- N-sec-Butyl-4-oxo-piperidinium-Methosulfat (R¹ = -CH₂CH(CH₃)₂, R² = H in Formel PIP-2)
- N-sec-Butyl-3-oxo-piperidinium-Tosylat (R¹ = -CH₂CH(CH₃)₂, R² = H in Formel PIP-1)
- N-sec-Butyl-4-oxo-piperidinium-Tosylat (R¹ = -CH₂CH(CH₃)₂, R² = H in Formel PIP-2)
- N,N-Dimethyl-3-oxo-piperidinium-Chlorid (R¹ = R² = -CH₃ in Formel PIP-1)
- N,N-Dimethyl-4-oxo-piperidinium-Chlorid (R¹ = R² = -CH₃ in Formel PIP-2)
- N,N-Dimethyl-3-oxo-piperidinium-Bromid (R¹ = R² = -CH₃ in Formel PIP-1)
- N,N-Dimethyl-4-oxo-piperidinium-Bromid (R¹ = R² = -CH₃ in Formel PIP-2)
- N,N-Dimethyl-3-oxo-piperidinium-Methosulfat (R¹ = R² = -CH₃ in Formel PIP-1)
- N,N-Dimethyl-4-oxo-piperidinium-Methosalufat (R¹ = R² = -CH₃ in Formel PIP-2)
- N,N-Dimethyl-3-oxo-piperidinium-Tosylat (R¹ = R² = -CH₃ in Formel PIP-1)
- N,N-Dimethyl-4-oxo-piperidinium-Tosylat (R¹ = R² = -CH₃ in Formel PIP-2)
- N,N-Diethyl-3-oxo-piperidinium-Chlorid (R¹ = -CH₂CH₃, R² = H in Formel PIP-1)
- N,N-Diethyl-4-oxo-piperidinium-Chlorid (R¹ = -CH₂CH₃, R² = H in Formel PIP-2)
- N,N-Diethyl-3-oxo-piperidinium-Bromid (R¹ = -CH₂CH₃. R² = H in Formel PIP-1)
- N,N-Diethyl-4-oxo-piperidinium-Bromid (R¹ = -CH₂CH₃, R² = H in Formel PIP-2)
- N,N-Diethyl-3-oxo-piperidinium-Methosulfat (R¹ = -CH₂CH₃, R² = H in Formel PIP-1)
- N,N-Diethyl-4-oxo-piperidinium-Methosulfat (R¹ = -CH₂CH₃, R² = H in Formel PIP-2)
- N,N-Diethyl-3-oxo-piperidinium-Tosylat (R¹ = -CH₂CH₃, R² = H in Formel PIP-1)
- N,N-Diethyl-4-oxo-piperidinium-Tosylat (R¹ = -CH₂CH₃, R² = H in Formel PIP-2)
- N,N-Dipropyl-3-oxo-piperidinium-Chlorid (R¹ = R² = -CH₂CH₂CH₃ in Formel PIP-1)
- N,N-Dipropyl-4-oxo-piperidinium-Chlorid (R¹ = R² = -CH₂CH₂CH₃ in Formel PIP-2)
- N,N-Dipropyl-3-oxo-piperidinium-Bromid (R¹ = R² = -CH₂CH₂CH₃ in Formel PIP-1)
- N,N-Dipropyl-4-oxo-piperidinium-Bromid (R¹ = R² = -CH₂CH₂CH₃ in Formel PIP-2)
- N,N-Dipropyl-3-oxo-piperidinium-Methosulfat (R¹ = R² = -CH₂CH₂CH₃ in Formel PIP-1)
- N,N-Dipropyl-4-oxo-piperidinium-Methosulfat (R¹ = R² = -CH₂CH₂CH₃ in Formel PIP-2)
- N,N-Dipropyl-3-oxo-piperidinium-Tosylat (R¹ = R² = -CH₂CH₂CH₃ in Formel PIP-1)
- N,N-Dipropyl-4-oxo-piperidinium-Tosylat (R¹ = R² = -CH₂CH₂CH₃ in Formel PIP-2)
- N,N-Diisopropyl-3-oxo-piperidinium-Chlorid (R¹ = R² = -CH(CH₃)₂ in Formel PIP-1)
- N,N-Diisopropyl-4-oxo-piperidinium-Chlorid (R¹ = R² = -CH(CH₃)₂ in Formel PIP-2)
- N,N-Diisopropyl-3-oxo-piperidinium-Bromid (R¹ = R² = -CH(CH₃)₂ in Formel PIP-1)
- N,N-Diisopropyl-4-oxo-piperidinium-Bromid (R¹ = R² = -CH(CH₃)₂ in Formel PIP-2)
- N,N-Diisopropyl-3-oxo-piperidinium-Methosulfat (R¹ = R² = -CH(CH₃)₂ in Formel PIP-1)
- N,N-Diisopropyl-4-oxo-piperidinium-Methosulfat (R¹ = R² = -CH(CH₃)₂ in Formel PIP-2)
- N,N-Diisopropyl-3-oxo-piperidinium-Tosylat (R¹ = R² = -CH(CH₃)₂ in Formel PIP-1)
- N,N-Diisopropyl-4-oxo-piperidinium-Tosylat (R¹ = R² = -CH(CH₃)₂ in Formel PIP-2)
- N,N-Dibutyl-3-oxo-piperidinium-Chlorid (R¹ = R² = -(CH₂)₃CH₃ in Formel PIP-1)
- N,N-Dibutyl-4-oxo-piperidinium-Chlorid (R¹ = R² = -(CH₂)₃CH₃ in Formel PIP-2)
- N,N-Dibutyl-3-oxo-piperidinium-Bromid (R¹ = R² = -(CH₂)₃CH₃ in Formel PIP-1)
- N,N-Dibutyl-4-oxo-piperidinium-Bromid (R¹ = R² = -(CH₂)₃CH₃ in Formel PIP-2)
- N,N-Dibutyl-3-oxo-piperidinium-Methosulfat (R¹ = R² = -(CH₂)₃CH₃ in Formel PIP-1)
- N,N-Dibutyl-4-oxo-piperidinium-Methosulfat (R¹ = R² = -(CH₂)₃CH₃ in Formel PIP-2)
- N,N-Dibutyl-3-oxo-piperidinium-Tosylat (R¹ = R² = -(CH₂)₃CH₃ in Formel PIP-1)
- N,N-Dibutyl-4-oxo-piperidinium-Tosylat (R¹ = R² = -(CH₂)₃CH₃ in Formel PIP-2)
- N,N-Diisobutyl-3-oxo-piperidinium-Chlorid (R¹ = -CH₂CH (CH₃)₂, R² = H in Formel PIP-1)
- N,N-Diisobutyl-4-oxo-piperidinium-Chlorid (R¹ = -CH₂CH (CH₃)₂, R² = H in Formel PIP-2)
- N,N-Diisobutyl-3-oxo-piperidinium-Bromid (R¹ = -CH₂CH (CH₃)₂, R² = H in Formel PIP-1)
- N,N-Diisobutyl-4-oxo-piperidinium-Bromid (R¹ = -CH₂CH (CH₃)₂, R² = H in Formel PIP-2)
- N,N-Diisobutyl-3-oxo-piperidinium-Methosulfat (R¹ = -CH₂CH (CH₃)₂, R² = H in Formel PIP-1)
- N,N-Diisobutyl-4-oxo-piperidinium-Methosulfat (R¹ = -CH₂CH (CH₃)₂, R² = H in Formel PIP-2)
- N,N-Diisobutyl-3-oxo-piperidinium-Tosylat (R¹ = -CH₂CH (CH₃)₂, R² = H in Formel PIP-1)
- N,N-Diisobutyl-4-oxo-piperidinium-Tosylat (R¹ = -CH₂CH (CH₃)₂, R² = H in Formel PIP-2)
- N,N-Di(sec-Butyl)-3-oxo-piperidinium-Chlorid (R¹ = R² = -CH₂CH(CH₃)₂ in Formel PIP-1)
- N,N-Di(sec-Butyl)-4-oxo-piperidinium-Chlorid (R¹ = R² = -CH₂CH(CH₃)₂ in Formel PIP-2)
- N,N-Di(sec-Butyl)-3-oxo-piperidinium-Bromid (R¹ = R² = -CH₂CH(CH₃)₂ in Formel PIP-1)
- N,N-Di(sec-Butyl)-4-oxo-piperidinium-Bromid (R¹ = R² = -CH₂CH(CH₃)₂ in Formel PIP-2))
- N,N-Di(sec-Butyl)-3-oxo-piperidinium-Methosulfat (R¹ = R² = -CH₂CH(CH₃)₂ in Formel PIP-1)
- N,N-Di(sec-Butyl)-4-oxo-piperidinium-Methosulfat (R¹ = R² = -CH₂CH(CH₃)₂ in Formel PIP-2)
- N,N-Di(sec-Butyl)-3-oxo-piperidinium-Tosylat (R¹ = R² = -CH₂CH(CH₃)₂ in Formel PIP-1))
- N,N-Di(sec-Butyl)-4-oxo-piperidinium-Tosylat (R¹ = R² = -CH₂CH(CH₃)₂ in Formel PIP-2)
- N,N-3,5-Tetramethyl-4-oxo-piperidinium-lodid (R¹ = R² = R³ = R⁴ = -CH₃ in Formel PIP-2)
- N,N-2,6-Tetramethyl-4-oxo-piperidinium-lodid (R¹ = R² = R⁵ = R⁶ = -CH₃ in Formel PIP-2)

Weiterhin hat es sich als vorteilhaft erwiesen, wenn die erfindungsgemäßen Färbe- und/oder Aufhellmittel nichtionogene grenzflächenaktive Stoffe enthalten.

Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxystearat (Handels-produkt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Färbe- und/oder Aufhellmittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrotidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropyl methacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel können die Inhaltsstoffe in einem geeigneten wäßrigen, alkoholischen oder wäßrig-alkoholischen Träger enthalten. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1-20 Gewichtsprozent, ganz besonders bevorzugt in einer Konzentration von 2-10 Gewichtsprozent, jeweils bezogen auf das Mittel, enthalten.

In weiter bevorzugten erfindungsgemäßen Mitteln ist das Lösungsmittel ausgewählt aus Ethanol, n-Propanol, Isoropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Der pH-Wert der erfindungsgemäßen Mittel kann durch geeignete Inhaltstoffe wie Acidifizierungsmittel oder Alkalisierungsmittel in einem weiten Bereich eingestellt werden.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Für die Anwendung der erfindungsgemäßen Mittel auf dem Haar werden die Färbe- und/oder Aufhellmittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Färbe- und/oder Aufhellmittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Mittel zum Färben und/oder Aufhellen keratinischer Fasern enthalten zusätzlich Cu-, Fe-, Mn-, Co-, Ce-, V-, Ru-Ionen oder Komplexe dieser Ionen, wobei bevorzugte Mittel 0,0001 bis 2,5 Gew.-%, vorzugsweise 0,001 bis 1 Gew.-% mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Mangandioxid (MnO₂).

Erfindungsgemäß bevorzugt ist auch der Einsatz von so genannten Komplexbildnern. Komplexbilder sind Stoffe, die Metallionen komplexieren können. Bevorzugte Komplexbildner sind so genannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten MetallAtomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Komplexierende Gruppen (Liganden) üblicher komplexbildender Polymere sind Iminodi-essigsäure-, Hydroxychinolin-, Thioharnstoff-, Guanidin-, Dithiocarbamat-, Hydroxamsäure-, Amidoxim-, Aminophosphorsäure-, (cycl.) Polyamino-, Mercapto-, 1,3-Dicarbonyl- und Kronenether-Reste mit z. T. sehr spezif. Aktivitäten gegenüber Ionen unterschiedlicher Metalle. Basispolymere vieler auch kommerziell bedeutender komplexbildender Polymere sind Polystyrol, Polyacrylate, Polyacrylnitrile, Polyvinylalkohole, Polyvinylpyridine und Polyethylenimine. Auch natürliche Polymere wie Cellulose, Stärke od. Chitin sind komplexbildende Polymere. Darüber hinaus können diese durch polymeranaloge Umwandlungen mit weiteren Ligand-Funktionalitäten versehen werden.

Besonders bevorzugt ist im Rahmen der vorliegenden Erfindung der Einsatz eines oder mehrerer Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine.

Im Rahmen der vorliegenden Erfindung können alle Komplexbildner des Standes der Technik eingesetzt werden. Diese können unterschiedlichen chemischen Gruppen angehören. Vorzugsweise werden einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure,
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)glycin, N-(1,2-Dicarboxy-2-hydroxyethyl)-glycin, N-(1,2-Dicarboxy-2-hydroxyethyl)asparaginsäure oder Nitrilotriessigsäure (NTA),
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate hiervon,
d) Aminophosphonsäuren wie Ethylendiamintetra(methylenphosphonsäure), Diethylentriaminpenta(methylenphosphonsäure) oder Nitrilotri(methylenphosphonsäure),
e) Phosphonopolycarbonsäuren wie 2-Phosphonobutan-1,2,4-tricarbonsäure sowie
f) Cyclodextrine.

Als Polycarbonsäuren a) werden im Rahmen dieser Patentanmeldung Carbonsäuren -auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Ebenso sind als weitere bevorzugte Komplexbildner polymere Aminodicarbonsäuren, deren Salze oder deren Vorläufersubstanzen zu nennen. Besonders bevorzugt sind Polyasparaginsäuren bzw. deren Salze und Derivate, die neben Cobuilder-Eigenschaften auch eine bleichstabilisierende Wirkung aufweisen.

Weitere geeignete Komplexbildner sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und OctaNatriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Erfindungsgemäß besonders bevorzugte Mittel enthalten einen oder mehrere Stoffe aus der Gruppe
(a) Nitrilotriessigsäure (NTA),
(b) Diethylenetriaminpentaesigsäure (DTPA),
(c) Ethylendiamindibernsteinsäure (EDDS),
(d) Ethylenediamindiglutarsäure (EDGA),
(e) 2- Hydroxypropylendiamindibernsteinsäure (HPDS),
(f) Glycinamid-N,N'- dibernsteinsäure (GADS),
(g) Ethylendiamin-N-N'-diglutarsäure (EDDG),
(h) 2-Hydroxypropylendiamin-N-N'-dibernsteinsäure (HPDDS),
(i) Ethylendiamintetraessigsäure (EDTA),
(j) Ethylendicysteinsäure (EDC),
(k) Diaminoalkyldi(sulfobernsteinsäure) (DDS),
(l) Ethylendiamine-N-N'-bis(ortho-hydroxyphenylesigsäure (EDDHA),
(m) N-2-hydroxyethyl-N,N-diessigsäure,
(n) Glyceryliminodiessigsäure,
(o) Iminodiessigsäure-N-2-hydroxypropylsulfonsäure,
(p) Asparaginsäure-N-carboxymethyl-N-2,5-hydroxypropyl-3-sulfonsäure,
(q) 8-Alanin-N,N'-diessigsäure,
(r) Asparaginsäure-N,N'-diessigsäure,
(s) Asparaginsäure-N-monoessigsäure,
(t) Dipicolinsäure,
(u) sowie deren Salze und/oder Derivate

Aus den vorstehend genannten Stoffgruppen sind einige Vertreter im Rahmen der vorliegenden Erfindung besonders bevorzugt. Erfindungsgemäß besonders bevorzugte Mittel zum Färben und/oder Aufhellen keratinischer Fasern enthalten zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine,
wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

Bevorzugte erfindungsgemäße Mittel werden wasserarm bzw. wasserfrei formuliert. Erfindungsgemäß besonders bevorzugte Mittel sind dadurch gekennzeichnet, daß sie weniger als 5 Gew.-%, vorzugsweise weniger als 2 Gew.-%, besonders bevorzugt weniger als 1 Gew.-% und insbesondere weniger als 0,5 Gew.-% Wasser enthalten, wobei bevorzugte Mittel wasserfrei sind. Der Wassergehalt der Mittel läßt sich beispielsweise mittels Titration nach Karl Fischer bestimmen.

Ein zweiter Gegenstand der vorliegenden Erfindung ist Verfahren zum Färben und Aufhellen von Keratinfasern, insbesondere menschlichen Haaren, bei dem
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein erfindungsgemäßes Mittel ist.

Die erfindungsgemäßen Mittel können demnach als Einkomponentenmittel (Färbe- und Aufhellmittel M2 bzw. Nachbehandlungsmittel M4), als Zweikomponenten Mittel (M2 + M3) oder als Dreikomponentenmittel (M2 + M3 + M4) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und Aufhellungsverfahren, bei dem die Aufhellcreme und das Oxidationsmittel zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem erfindungsgemäßen Mittel zu einer homogenen Zusammensetzung vermischt, und diese auf das Haar aufgebracht wird.

In bevorzugten erfindungsgemäßen Verfahren dieser Art enthält die Zusammensetzung auf wäßriger Grundlage bezogen auf ihr Gewicht 1 bis 20 Gew.%, vorzugsweise 2 bis 10 Gew.% und insbesondere 3 bis 6 Gew.% Wasserstoffperoxid, berechnet als 100%iges H₂O₂.

Weiter bevorzugte erfindungsgemäße Verfahren dieser Art sind dadurch gekennzeichnet, daß die Zusammensetzung auf wäßriger Grundlage, enthaltend Wasserstoffperoxid, mit einem erfindungsgemäßen Mittel im Gewichtsverhältnis 1:5 bis 10:1, vorzugsweise 1:2 bis 5:1 und insbesondere 1:2 bis 2:1 zu einer homogenen Zusammensetzung vermischt, und diese auf das Haar aufgebracht wird.

Alternativ kann - wie vorstehend erwähnt - auch ein Dreikomponentensystem zur Anwendung gelangen. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem erfindungsgemäßen Mittel zu einer homogenen Zusammensetzung vermischt, und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.

Beispiele:
Die Untersuchungen wurden in wässrigen Lösungen durchgeführt. Strähnen aus dunkelblonden Haaren (Code: Kerling 7/0) von ca. 0,5 g Gewicht wurden in Bechergläsern mit der 15 fachen Menge an Wirkstofflösung übergossen und 30 Minuten bei 32 ° C blondiert. Der pH-Wert wurde auf ca. 10,0 bzw. 8,0 eingestellt.
Die Konzentrationen an Wasserstoffperoxid betrugen jeweils 6,0 %, die Konzentrationen an 2,3-Butandion (Fa. Aldrich) 5,0%. Die Lösungen wurden zusätzlich mit 1,5% Turpinal (60%ige Lösung von Hydroxyethandiphosphonsäure) und 0,1 % Dipicolinsäure stabilisiert.

### Farbmetrische Resultate:

| **Versuch** | **L** | **a** | **b** | **dE** |
|---|---|---|---|---|
| Ausgangshaar dunkelblond | 31,17 | 6,97 | 13,66 | |
| Dunkelblond + Wasserstoffperoxid pH 10 | 41,37 | 9,14 | 21,88 | 13,28 |
| Dunkelblond + Wasserstoffperoxid + 5% 2,3-Butandion pH 10 | 43,64 | 8,79 | 22,46 | **15,37** |
| | | | | |
| Dunkelblond + Wasserstoffperoxid pH 8 | 33,26 | 7,69 | 15,60 | 2,94 |
| Dunkelblond + Wasserstoffperoxid + 5% 2,3-Butandion pH 8 | 35,54 | 8,44 | 17,83 | **6,22** |

Die Messwerte zeigen, dass durch den Zusatz von 2,3-Butandion zu den Wasserstoffperoxidlösungen eine Verbesserung der Aufhellung erzielt wird.

## Patentansprüche

1. Mittel zum Färben und Aufhellen von Keratinfasern, insbesondere menschlichen Haaren, enthaltend - bezogen auf sein Gewicht - 0,001 bis 15 Gew.-% mindestens einer Verbindung der Formel (la): 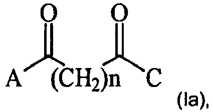 in der
A, C stehen unabhängig voneinander für eine gegebenenfalls substituierte, insbesondere halogenierte, C₁₋₂₈-Alkylgruppe, vorzugsweise eine gegebenenfalls substituierte, insbesondere halogenierte, C₁₋₆-Alkylgruppe, oder eine C₂₋₂₈-Alkylengruppe, vorzugsweise eine C₂₋₆-Alkylengruppe, wobei die Reste A und C miteinander einen Ring bilden können und wobei bevorzugte Reste A bzw. C ausgewählt sind aus -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃, -CF₃, -CCl₃,-CBr₃, -Cl₃, -CF₂CF₃, -CCl₂CCl₃
n steht für 0,1, oder 2, vorzugsweise für 0,
0,5 bis 15 Gew.-% Wasserstoffperoxid (berechnet als 100%iges H₂O₂),
zusätzlich 0,001 bis 5 Gew.-% eines oder mehrerer Oxidationsfarbstoffvorprodukte und/oder direktziehender Farbstoffe, und
1 bis 20 Gew.-% mindestens einer Imidazolverbindung gemäß Formel (VII) und/oder deren physiologisch verträglichen Salzen 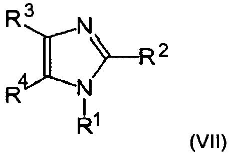 worin
R¹ steht für ein Wasserstoffatom, eine gegebenenfalls substituierte Arylgruppe oder eine (C₁-C₆)-Alkylgruppe,
R² steht für ein Wasserstoffatom, eine Carboxaldehydgruppe, eine (C₁-C₆)-Alkylgruppe oder eine Nitrogruppe,
R³ steht für ein Wasserstoffatom, eine Carboxy-(C₁-C₆)-alkylgruppe, eine Amino-(C₁-C₆)-alkylgruppe, eine Carboxylgruppe, eine Carboxaldehydgruppe, eine (C₁-C₆)-Alkylgruppe, eine Nitrogruppe, eine 2-Amino-3-hydroxypropylgruppe oder eine Gruppe -CH₂-CH(NH₂)-COOH,
R⁴ steht für ein Wasserstoffatom, eine Carboxaldehydgruppe oder eine Carboxylgruppe,
wobei die Imidazolverbindung gemäß Formel (VII) und/oder deren physiologisch verträgliche Salze ausgewählt ist/sind aus Histamin, D-Histidin, L-Histidin, DL-Histidin, D-Histidinol, L-Histidinol, DL-Histidinol, Imidazol, Imidazol-4-essigsäure, Imidazol-4-carbonsäure, Imidazol-4,5-dicarbonsäure, Imidazol-2-carboxaldehyd, Imidazol-4-carboxaldehyd, Imidazol-5-carboxaldehyd, 2-Nitroimidazol, 4-Nitroimidazol, 4-Methylimidazol-5-carboxaldehyd, N-Methylimidazol-2-carboxaldehyd, 4-Methylimidazol, 2-Methylimidazol, N-Methylimidazol, N-(4-Aminophenyl)-imidazol, sowie den physiologisch verträglichen Salze der vorgenannten Verbindungen.

2. Mittel zum Färben und Aufhellen keratinischer Fasern nach Anspruch 1, **dadurch gekennzeichnet, daß** es ein oder mehrere Verbindungen aus der Gruppe H₃C-C(O)-C(O)-CH₃, H₃C-CH₂-C(O)-C(O)-CH₃, H₃C-(CH₂)₂-C(O)-C(O)-CH₃, (H₃C)₂CH-C(O)-C(O)-CH₃, H₃C-(CH₂)₃-C(O)-C(O)-CH₃, H₃C-CH₂-CH(CH₃)-C(O)-C(O)-CH₃, (H₃C)₂CH-CH₂-C(O)-C(O)-CH₃, (H₃C)₃C-C(O)-C(O)-CH₃, F₃C-C(O)-C(O)-CH₃, Cl₃C-C(O)-C(O)-CH₃, Br₃C-C(O)-C(O)-CH₃, I₃C-C(O)-C(O)-CH₃, F₃C-CF₂-C(O)-C(O)-CH₃, Cl₃C-CCl₂-C(O)-C(O)-CH₃, H₃C-CH₂-C(O)-C(O)-CH₂CH₃, H₃C-(CH₂)₂-C(O)-C(O)-CH₂CH₃, (H₃C)₂CH-C(O)-C(O)-CH₂CH₃, H₃C-(CH₂)₃-C(O)-C(O)-CH₂CH₃, H₃C-CH₂-CH(CH₃)-C(O)-C(O)-CH₂CH₃, (H₃C)₂CH-CH₂-C(O)-C(O)-CH₂CH₃, (H₃C)₃C-C(O)-C(O)-CH₂CH₃, F₃C-C(O)-C(O)-CH₂CH₃, Cl₃C-C(O)-C(O)-CH₂CH₃, Br₃C-C(O)-C(O)-CH₂CH₃, I₃C-C(O)-C(O)-CH₂CH₃, F₃C-CF₂-C(O)-C(O)-CH₂CH₃, Cl₃C-CCl₂-C(O)-C(O)-CH₂CH₃, H₃C-(CH₂)₂-C(O)-C(O)-(CH₂)₂CH₂, (H₃C)₂CH-C(O)-C(O)-(CH₂)₂CH₂, H₃C-(CH₂)₃-C(O)-C(O)-(CH₂)₂CH₂, H₃C-CH₂-CH(CH₃)-C(O)-C(O)-(CH₂)₂CH₂, (H₃C)₂CH-CH₂-C(O)-C(O)-(CH₂)₂CH₂, (H₃C)₃C-C(O)-C(O)-(CH₂)₂CH₂, F₃C-C(O)-C(O)-(CH₂)₂CH₂, Cl₃C-C(O)-C(O)-(CH₂)₂CH₂, Br₃C-C(O)-C(O)-(CH₂)₂CH₂, I₃C-C(O)-C(O)-(CH₂)₂CH₂, F₃C-CF₂-C(O)-C(O)-(CH₂)₂CH₂, Cl₃C-CCl₂-C(O)-C(O)-(CH₂)₂CH₂, (H₃C)₂CH-C(O)-C(O)-CH(CH₃)₂, H₃C-(CH₂)₃-C(O)-C(O)-CH(CH₃)₂, H₃C-CH₂-CH(CH₃)-C(O)-C(O)-CH(CH₃)₂, (H₃C)₂CH-CH₂-C(O)-C(O)-CH(CH₃)₂ (H₃C)₃C-C(O)-C(O)-CH(CH₃)₂, F₃C-C(O)-C(O)-CH(CH₃)₂, C)₃C-C(O)-C(O)-CH(CH₃)₂, Br₃C-C(O)-C(O)-CH(CH₃)₂, I₃C-C(O)-C(O)-CH(CH₃)₂, F₃C-CF₂-C(O)-C(O)-CH(CH₃)₂, Cl₃C-CCl₂-C(O)-C(O)-CH(CH₃)₂, H₃C-(CH₂)₃-C(O)-C(O)-(CH₂)₃CH₃, H₃C-CH₂-CH(CH₃)-C(O)-C(O)-(CH₂)₃CH₃, (H₃C)₂CH-CH₂-C(O)-C(O)-(CH₂)₃CH₃, (H₃C)₃C-C(O)-C(O)-(CH₂)₃CH₃, F₃C-C(O)-C(O)-(CH₂)₃CH₃, Cl₃C-C(O)-C(O)-(CH₂)₃CH₃, Br₃C-C(O)-C(O)-(CH₂)₃CH₃, I₃C-C(O)-C(O)-(CH₂)₃CH₃, F₃C-CF₂-C(O)-C(O)-(CH₂)₃CH₃, Cl₃C-CCl₂-C(O)-C(O)-(CH₂)₃CH₃, H₃C-CH₂-CH(CH₃)-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH(CH₃)CH₂CH₃, F₃C-C(O)-C(O)-CH(CH₃)CH₂CH₃, Cl₃C-C(O)-C(O)-CH(CH₃)CH₂CH₃, Br₃C-C(O)-C(O)-CH(CH₃)CH₂CH₃, I₃C-C(O)-C(O)-CH(CH₃)CH₂CH₃, F₃C-CF₂-C(O)-C(O)-CH(CH₃)CH₂CH₃, Cl₃C-CCl₂-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH₂CH(CH₃)₂, F₃C-C(O)-C(O)-CH₂CH(CH₃)₂, Cl₃C-C(O)-C(O)-CH₂CH(CH₃)₂, Br₃C-C(O)-C(O)-CH₂CH(CH₃)₂, I₃C-C(O)-C(O)-CH₂CH(CH₃)₂, F₃C-CF₂-C(O)-C(O)-CH₂CH(CH₃)₂, Cl₃C-CCl₂-C(O)-C(O)-CH₂CH(CH₃)₂, (H₃C)₃C-C(O)-C(O)-C(CH₃)₃, F₃C-C(O)-C(O)-C(CH₃)₃, Cl₃C-C(O)-C(O)-C(CH₃)₃, Br₃C-C(O)-C(O)-C(CH₃)₃, I₃C-C(O)-C(O)-C(CH₃)₃, F₃C-CF₂-C(O)-C(O)-C(CH₃)₃, Cl₃C-CCl₂-C(O)-C(O)-C(CH₃)₃, F₃C-C(O)-C(O)-CF₃, Cl₃C-C(O)-C(O)-CF₃, Br₃C-C(O)-C(O)-CF₃, I₃C-C(O)-C(O)-CF₃, F₃C-CF₂-C(O)-C(O)-CF3, Cl₃C-CCl₂-C(O)-C(O)-CF₃, Cl₃C-C(O)-C(O)-CCl₃, Br₃C-C(O)-C(O)-CCl₃, I₃C-C(O)-C(O)-CCl₃, F₃C-CF₂-C(O)-C(O)-CCl₃, Cl₃C-CCl₂-C(O)-C(O)-CCl₃, Br₃C-C(O)-C(O)-CBr₃, I₃C-C(O)-C(O)-CBr₃, F₃C-CF₂-C(O)-C(O)-CBr₃, Cl₃C-CCl₂-C(O)-C(O)-CBr₃, I₃C-C(O)-C(O)-Cl₃, F₃C-CF₂-C(O)-C(O)-Cl₃, Cl₃C-CCl₂-C(O)-C(O)-Cl₃, CF₂CF₃-C(O)-C(O)-CF₂CF₃, CCl₂CCl₃-C(O)-C(O)-CF₂CF₃, CCl₂CCl₃-C(O)-C(O)-CCl₂CCl₃, H₃C-C(O)-(CH₂)-C(O)-CH₃, H₃C-CH₂-C(O)-(CH₂)-C(O)-CH₃, H₃C-(CH₂)₂-C(O)-(CH₂)-C(O)-CH₃, (H₃C)₂CH-C(O)-(CH₂)-C(O)-CH₃, H₃C-(CH₂)a-C(O)-(CH₂)-C(O)-CH₃, H₃C-CH₂-CH(CH₃)-C(O)-(CH₂)-C(O)-CH₃, (H₃C)₂CH-CH₂-C(O)-(CH₂)-C(O)-CH₃, (H₃C)₃C-C(O)-(CH₂)-C(O)-CH₃, F₃C-C(O)-(CH₂)-C(O)-CH₃, Cl₃C-C(O)-(CH₂)-C(O)-CH₃, Br₃C-C(O)-(CH₂)-C(O)-CH₃, I₃C-C(O)-(CH₂)-C(O)-CH₃, F₃C-CF₂-C(O)-(CH₂)-C(O)-CH₃, Cl₃C-CCl₂-C(O)-(CH₂)-C(O)-CH₃, H₃C-CH₂-C(O)-(CH₂)-C(O)-CH₂CH₃, H₃C-(CH₂)₂-C(O)-(CH₂)-C(O)-CH₂CH₃, (H₃C)₂CH-C(O)-(CH₂)-C(O)-CH₂CH₃, H₃C-(CH₂)₃-C(O)-(CH₂)-C(O)-CH₂CH₃, H₃C-CH₂-CH(CH₃)-C(O)-(CH₂)-C(O)-CH₂CH₃, (H₃C)₂CH-CH₂-C(O)-(CH₂)-C(O)-CH₂CH₃, (H₃C)₃C-C(O)-(CH₂)-C(O)-CH₂CH₃, F₃C-C(O)-(CH₂)-C(O)-CH₂CH₃, Cl₃C-C(O)-(CH₂)-C(O)-CH₂CH₃, Br₃C-C(O)-(CH₂)-C(O)-CH₂CH₃, I₃C-C(O)-(CH₂)-C(O)-CH₂CH₃, F₃C-CF₂-C(O)-(CH₂)-C(O)-CH₂CH₃, Cl₃C-CCl₂-C(O)-(CH₂)-C(O)-CH₂CH₃, H₃C-(CH₂)₂-C(O)-(CH₂)-C(O)-(CH₂)₂CH₂, (H₃C)₂CH-C(O)-(CH₂)-C(O)-(CH₂)₂CH₂, H₃C-(CH₂)₃-C(O)-(CH₂)-C(O)-(CH₂)₂CH₂, H₃C-CH₂-CH(CH₃)-C(O)-(CH₂)-C(O)-(CH₂)₂CH₂, (H₃C)₂CH-CH₂-C(O)-(CH₂)-C(O)-(CH₂)₂CH₂, (H₃C)₃C-C(O)-(CH₂)-C(O)-(CH₂)₂CH₂, F₃C-C(O)-(CH₂)-C(O)-(CH₂)₂CH₂, Cl₃C-C(O)-(CH₂)-C(O)-(CH2)2CH2, Br₃C-C(O)-(CH₂)-C(O)-(CH₂)₂CH₂, I₃C-C(O)-(CH₂)-C(O)-(CH₂)₂CH₂, F₃C-CF₂-C(O)-(CH₂)-C(O)-(CH₂)₂CH₂, Cl₃C-CCl₂-C(O)-(CH₂)-C(O)-(CH₂)₂CH₂, (H₃C)₂CH-C(O)-(CH₂)-C(O)-CH(CH₃)₂, H₃C-(CH₂)₃-C(O)-(CH₂)-C(O)-CH(CH₃)₂, H₃C-CH₂-CH(CH₃)-C(O)-(CH₂)-C(O)-CH(CH₃)₂, (H₃C)₂CH-CH₂-C(O)-(CH₂)-C(O)-CH(CH₃)₂, (H₃C)₃C-C(O)-(CH₂)-C(O)-CH(CH₃)₂, F₃C-C(O)-(CH₂)-C(O)-CH(CH₃)₂, Cl₃C-C(O)-(CH₂)-C(O)-CH(CH₃)₂, Br₃C-C(O)-(CH₂)-C(O)-CH(CH₃)₂, I₃C-C(O)-(CH₂)-C(O)-CH(CH₃)₂, F₃C-CF₂-C(O)-(CH₂)-C(O)-CH(CH₃)₂, Cl₃C-CCl₂-C(O)-(CH₂)-C(O)-CH(CH₃)₂, H₃C-(CH₂)₃-C(O)-(CH₂)-C(O)-(CH₂)₃CH₃, H₃C-CH₂-CH(CH₃)-C(O)-(CH₂)-C(O)-(CH₂)₃CH₃, (H₃C)₂CH-CH₂-C(O)-(CH₂)-C(O)-(CH₂)₃CH₃, (H₃C)₃C-C(O)-(CH₂)-C(O)-(CH₂)₃CH₃, F₃C-C(O)-(CH₂)-C(O)-(CH₂)₃CH₃, Cl₃C-C(O)-(CH₂)-C(O)-(CH₂)₃CH₃, Br₃C-C(O)-(CH₂)-C(O)-(CH₂)₃CH₃, I₃C-C(O)-(CH₂)-C(O)-(CH₂)₃CH₃, F₃C-CF₂-C(O)-(CH₂)-C(O)-(CH₂)₃CH₃, Cl₃C-CCl₂-C(O)-(CH₂)-C(O)-(CH₂)₃CH₃, H₃C-CH₂-CH(CH₃)-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH(CH₃)CH₂CH₃, F₃C-C(O)-(CH₂)-C(O)-CH(CH₃)CH₂CH₃, Cl₃C-C(O)-(CH₂)-C(O)-CH(CH₃)CH₂CH₃, Br₃C-C(O)-(CH₂)-C(O)-CH(CH₃)CH₂CH₃, I₃C-C(O)-(CH₂)-C(O)-CH(CH₃)CH₂CH₃, F₃C-CF₂-C(O)-(CH₂)-C(O)-CH(CH₃)CH₂CH₃, Cl₃C-CCl₂-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH₂CH(CH₃)₂, F₃C-C(O)-(CH₂)-C(O)-CH₂CH(CH₃)₂, Cl₃C-C(O)-(CH₂)-C(O)-CH₂CH(CH₃)₂, Br₃C-C(O)-(CH₂)-C(O)-CH₂CH(CH₃)₂, I₃C-C(O)-(CH₂)-C(O)-CH₂CH(CH₃)₂, F₃C-CF₂-C(O)-(CH₂)-C(O)-CH₂CH(CH₃)₂, Cl₃C-CCl₂-C(O)-(CH₂)-C(O)-CH₂CH(CH₃)₂, (H₃C)₃C-C(O)-(CH₂)-C(O)-C(CH₃)₃, F₃C-C(O)-(CH₂)-C(O)-C(CH₃)₃, Cl₃C-C(O)-(CH₂)-C(O)-C(CH₃)₃, Br₃C-C(O)-(CH₂)-C(O)-C(CH₃)₃, I₃C-C(O)-(CH₂)-C(O)-C(CH₃)₃, F₃C-CF₂-C(O)-(CH₂)-C(O)-C(CH₃)₃, Cl₃C-CCl₂-C(O)-(CH₂)-C(O)-C(CH₃)₃, F₃C-C(O)-(CH₂)-C(O)-CF₃, Cl₃C-C(O)-(CH₂)-C(O)-CF₃, Br₃C-C(O)-(CH₂)-C(O)-CF₃, I₃C-C(O)-(CH₂)-C(O)-CF₃, F₃C-CF₂-C(O)-(CH₂)-C(O)-CF₃, Cl₃C-CCl₂-C(O)-(CH₂)-C(O)-CF₃, Cl₃C-C(O)-(CH₂)-C(O)-CCl₃, Br₃C-C(O)-(CH₂)-C(O)-CCl₃, I₃C-C(O)-(CH₂)-C(O)-CCl₃, F₃C-CF₂-C(O)-(CH₂)-C(O)-CCl₃, Cl₃C-CCl₂-C(O)-(CH₂)-C(O)-CCl₃, Br₃C-C(O)-(CH₂)-C(O)-CBr₃, I₃C-C(O)-(CH₂)-C(O)-CBr₃, F₃C-CF₂-C(O)-(CH₂)-C(O)-CBr₃, Cl₃C-CCl₂-C(O)-(CH₂)-C(O)-CBr₃, I₃C-C(O)-(CH₂)-C(O)-Cl₃, F₃C-CF₂-C(O)-(CH₂)-C(O)-Cl₃, Cl₃C-CCl₂-C(O)-(CH₂)-C(O)-C)₃, CF₂CF₃-C(O)-(CH₂)-C(O)-CF₂CF₃, CCl₂CCl₃-C(O)-(CH₂)-C(O)-CF₂CF₃, CCl₂CCl₃-C(O)-(CH₂)-C(O)-CC₂CCl₃, H₃C-C(O)-(CH₂)₂-C(O)-CH₃, H₃C-CH₂-C(O)-(CH₂)₂-C(O)-CH₃, H₃C-(CH₂)₂-C(O)-(CH₂)₂-C(O)-CH₃, (H₃C)₂CH-C(O)-(CH₂)₂-C(O)-CH₃, H₃C-(CH₂)₃-C(O)-(CH₂)₂-C(O)-CH₃, H₃C-CH₂-CH(CH₃)-C(O)-(CH₂)₂-C(O)-CH₃, (H₃C)₂CH-CH₂-C(O)-(CH₂)₂-C(O)-CH₃, (H₃C)₃C-C(O)-(CH₂)₂-C(O)-CH₃, F₃C-C(O)-(CH₂)₂-C(O)-CH₃, Cl₃C-C(O)-(CH₂)₂-C(O)-CH₃, Br₃C-C(O)-(CH₂)₂-C(O)-CH₃, I₃C-C(O)-(CH₂)₂-C(O)-CH₃, F₃C-CF₂-C(O)-(CH₂)₂-C(O)-CH₃, Cl₃C-CCl₂-C(O)-(CH₂)₂-C(O)-CH₃, H₃C-CH₂-C(O)-(CH₂)₂-C(O)-CH₂CH₃, H₃C-(CH₂)₂-C(O)-(CH₂)₂-C(O)-CH₂CH₃, (H₃C)₂CH-C(O)-(CH₂)₂-C(O)-CH₂CH₃, H₃C-(CH₂)₃-C(O)-(CH₂)₂-C(O)-CH₂CH₃, H₃C-CH₂-CH(CH₃)-C(O)-(CH₂)₂-C(O)-CH₂CH₃, (H₃C)₂CH-CH₂-C(O)-(CH₂)₂-C(O)-CH₂CH₃, (H₃C)₃C-C(O)-(CH₂)₂-C(O)-CH₂CH₃, F₃C-C(O)-(CH₂)₂-C(O)-CH₂CH₃, Cl₃C-C(O)-(CH₂)₂-C(O)-CH₂CH₃, Br₃C-C(O)-(CH₂)₂-C(O)-CH₂CH₃, I₃C-C(O)-(CH₂)₂-C(O)-CH₂CH₃, F₃C-CF₂-C(O)-(CH₂)₂-C(O)-CH₂CH₃, Cl₃C-CCl₂-C(O)-(CH₂)₂-C(O)-CH₂CH₃, H₃C-(CH₂)₂-C(O)-(CH₂)₂-C(O)-(CH₂)₂CH₂, (H₃C)₂CH-C(O)-(CH₂)₂-C(O)-(CH₂)₂CH₂, H₃C-(CH₂)₃-C(O)-(CH₂)₂-C(O)-(CH₂)₂CH₂, H₃C-CH₂-CH(CH₃)-C(O)-(CH₂)₂-C(O)-(CH₂)₂CH₂, (H₃CH-CH-CH₂-C(O)-(CH₂)₂-C(O)-(CH₂)₂CH₂, (H₃C)₃C-C(O)-(CH₂)₂-C(O)-(CH₂)₂CH₂, F₃C-C(O)-(CH₂)₂-C(O)-(CH₂)₂CH₂, Cl₃C-C(O)-(CH₂)₂-C(O)-(CH₂)₂CH₂, Br₃C-C(O)-(CH₂)₂-C(O)-(CH₂)₂CH₂, I₃C-C(O)-(CH₂)₂-C(O)-(CH₂)₂CH₂, F₃C-CF₂-C(O)-(CH₂)₂-C(O)-(CH₂)₂CH₂, Cl₃C-CCl₂-C(O)-(CH₂)₂-C(O)-(CH₂)₂CH₂, (H₃C)₂CH-C(O)-(CH₂)₂-C(O)-CH(CH₃)₂, H₃C-(CH₂)₃-C(O)-(CH₂)₂-C(O)-CH(CH₃)₂, H₃C-CH₂-CH(CH₃)-C(O)-(CH₂)₂-C(O)-CH(CH₃)₂, (H₃C)₂CH-CH₂-C(O)-(CH₂)₂-C(O)-CH(CH₃)₂, (H₃C)₃C-C(O)-(CH₂)₂-C(O)-CH(CH₃)₂, F₃C-C(O)-(CH₂)₂-C(O)-CH(CH₃)₂, Cl₃C-C(O)-(CH₂)₂-C(O)-CH(CH₃)₂, Br₃C-C(O)-(CH₂)₂-C(O)-CH(CH₃)₂, I₃C-C(O)-(CH₂)₂-C(O)-CH(CH₃)₂, F₃C-CF₂-C(O)-(CH₂)₂-C(O)-CH(CH₃)₂, Cl₃C-CCl₂-C(O)-(CH₂)₂-C(O)-CH(CH₃)₂, H₃C-(CH₂)₃-C(O)-(CH₂)₂-C(O)-(CH₂)₃CH₃, H₃C-CH₂-CH(CH₃)-C(O)-(CH₂)₂-C(O)-(CH₂)₃CH₃, (H₃C)₂CH-CH₂-C(O)-(CH₂)-C(O)-(CH₂)CH₃, (H₃C)₃C-C(O)-(CH₂)₂-C(O)-(CH₂)₃CH₃, F₃C-C(O)-(CH₂)₂-C(O)-(CH₂)₃CH₃, Cl₃C-C(O)-(CH₂)₂-C(O)-(CH₂)₃CH₃, Br₃C-C(O)-(CH₂)₂-C(O)-(CH₂)₃CH₃, I₃C-C(O)-(CH₂)₂-C(O)-(CH₂)₃CH₃, F₃C-CF₂-C(O)-(CH₂)₂-C(O)-(CH₂)₃CH₃, Cl₃C-CCl₂-C(O)-(CH₂)₂-C(O)-(CH₂)₃CH₃, H₃C-CH₂-CH(CH₃)-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH(CH₃)CH₂CH₃, F₃C-C(O)-(CH₂)₂-C(O)-CH(CH₃)CH₂CH₃, Cl₃C-C(O)-(CH₂)₂-C(O)-CH(CH₃)CH₂CH₃, Br₃C-C(O)-(CH₂)₂-C(O)-CH(CH₃)CH₂CH₃, I₃C-C(O)-(CH₂)₂-C(O)-CH(CH₃)CH₂CH₃, F₃C-CF₂-C(O)-(CH₂)₂-C(O)-CH(CH₃)CH₂CH₃, Cl₃C-CCl₂-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH₂CH(CH₃)₂, F₃C-C(O)-(CH₂)₂-C(O)-CH₂CH(CH₃)₂, Cl₃C-C(O)-(CH₂)₂-C(O)-CH₂CH(CH₃)₂, Br₃C-C(O)-(CH₂)₂-C(O)-CH₂CH(CH₃)₂, I₃C-C(O)-(CH₂)₂-C(O)-CH₂CH(CH₃)₂, F₃C-CF₂-C(O)-(CH₂)₂-C(O)-CH₂CH(CH₃)₂, Cl₃C-CCl₂-C(O)-(CH₂)₂-C(O)-CH₂CH(CH₃)₂, (H₃C)₃C-C(O)-(CH₂)₂-C(O)-C(CH₃)₃, F₃C-C(O)-(CH₂)₂-C(O)-C(CH₃)₃, Cl₃C-C(O)-(CH₂)₂-C(O)-C(CH₃)₃, Br₃C-C(O)-(CH₂)₂-C(O)-C(CH₃)₃, I₃C-C(O)-(CH₂)₂-C(O)-C(CH₃)₃, F₃C-CF₂-C(O)-(CH₂)₂-C(O)-C(CH₃)₃, Cl₃C-CCl₂-C(O)-(CH₂)₂-C(O)-C(CH₃)₃, F₃C-C(O)-(CH₂)₂-C(O)-CF₃, Cl₃C-C(O)-(CH₂)₂-C(O)-CF₃, Br₃C-C(O)-(CH₂)₂-C(O)-CF₃, I₃C-C(O)-(CH₂)₂-C(O)-CF₃, F₃C-CF₂-C(O)-(CH₂)₂-C(O)-CF₃, Cl₃C-CCl₂-C(O)-(CH₂)₂-C(O)-CF₃, Cl₃C-C(O)-(CH₂)₂-C(O)-CCl₃, Br₃C-C(O)-(CH₂)₂-C(O)-CCl₃, I₃C-C(O)-(CH₂)₂-C(O)-CCl₃, F₃C-CF₂-C(O)-(CH₂)₂-C(O)-CCl₃, Cl₃C-CCl₂-C(O)-(CH₂)₂-C(O)-CCl₃, Br₃C-C(O)-(CH₂)₂-C(O)-CBr₃, I₃C-C(O)-(CH₂)₂-C(O)-CBr₃, F₃C-CF₂-C(O)-(CH₂)₂-C(O)-CBr₃, Cl₃C-CCl₂-C(O)-(CH₂)₂-C(O)-CBr₃, I₃C-C(O)-(CH₂)₂-C(O)-Cl₃, F₃C-CF₂-C(O)-(CH₂)₂-C(O)-Cl₃, Cl₃C-CCl₂-C(O)-(CH₂)₂-C(O)-Cl₃, CF₂CF₃-C(O)-(CH₂)₂-C(O)-CF₂CF₃, CCl₂CCl₃-C(O)-(CH₂)₂-C(O)-CF₂CF₃, CCl₂CCl₃-C(O)-(CH₂)₂-C(O)-CCl₂CCl₃, enthält.

3. Mittel zum Färben und Aufhellen keratinischer Fasern nach einem der Ansprüche 1 oder 2, **dadurch gekennzeichnet, daß** es die Verbindung(en) der Formel (I) in Mengen von 0,1 bis 12,5 Gew.-%, vorzugsweise von 0,25 bis 10 Gew.%, besonders bevorzugt von 0,5 bis 7,5 Gew.-% und insbesondere von 1 bis 5 Gew.-%, jeweils bezogen auf das gesamte Mittel, enthält.

4. Mittel zum Färben und Aufhellen keratinischer Fasern nach einem der Ansprüche 1 bis 3, **dadurch gekennzeichnet, daß** es 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthält.

5. Mittel zum Färben und Aufhellen keratinischer Fasern nach einem der Ansprüche 1 bis 4, **dadurch gekennzeichnet, daß** es als Oxidationsfarbstoffvorprodukt mindestens eine Entwicklerkomponente enthält, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol; 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol.

6. Mittel zum Färben und Aufhellen keratinischer Fasern nach einem der Ansprüche 1 bis 5, **dadurch gekennzeichnet, daß** es als Oxidationsfarbstoffvorprodukt mindestens eine Kupplerkomponente enthält, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

7. Mittel zum Färben und Aufhellen keratinischer Fasern nach einem der Ansprüche 1 bis 6, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Basic Yellow 87, Basic Orange 31 und Basic Red 51 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

8. Mittel zum Färben und Aufhellen keratinischer fasern nach einem der Ansprüche 1 bis 7, **dadurch gekennzeichnet, daß** es zusätzlich 0,1 bis 20 Gew.%, vorzugsweise 1 bis 10 Gew.-% und insbesondere 2,5 bis 12,5 Gew.% mindestens einer nichtionischen Verbindung der allgemeinen Formel (Ni-I) 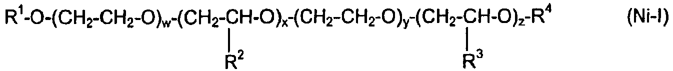 enthält, worin
R¹ steht für -H oder einen geradkettigen oder verzweigten, gesättigten oder ein-bzw. mehrfach ungesättigten C₆₋₂₄-Alkyl- oder -Alkenylrest,
R² und R³ stehen unabhängig voneinander für -CH₃; -CH₂CH₃, -CH₂CH₂-CH₃, CH(CH₃)₂
R⁴ 7 steht für -H oder einen geradkettigen oder verzweigten, gesättigten oder ein-bzw. mehrfach ungesättigten C₁₋₄-Alkyl- oder -Alkenylrest, vorzugsweise für-H, -CH₃; -CH₂CH₃, -CH₂CH₂CH₃, CH(CH₃)₂, -(CH₂)₃CH₃, -CH₂-CH(CH₃)₂, CH(CH₃)CH₂CH₃, -C(CH₃)₃
w, x, y, z stehen unabhängig voneinander für Zahlen von 0 bis 50, vorzugsweise von 0 bis 20, weiter bevorzugt von 0 bis 10 und insbesondere für 0, 1, 2, 3, 4, 5, 6.

9. Mittel zum Färben und Aufhellen keratinischer Fasern nach einem der vorangehenden Ansprüche, **dadurch gekennzeichnet, daß** es die mindestens eine Imidazolverbindung gemäß Formel (VII) und/oder deren physiologisch verträgliche Salze in Mengen von 2 bis 15 Gew.%, vorzugsweise von 2,5 bis 12,5 Gew.-%, besonders bevorzugt von 3 bis 10 Gew.% und insbesondere von 4 bis 8 Gew.-% enthält.

10. Mittel zum Färben und Aufhellen keratinischer Fasern nach einem der Ansprüche 1 bis 9, **dadurch gekennzeichnet, daß** es zusätzlich 0,01 bis 2 Gew.-% mindestens einer festen Peroxoverbindung, die ausgewählt ist aus Ammonium- und Alkalimetall-persulfaten undperoxidisulfaten enthält, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidislfate enthalten.

11. Mittel zum Färben und Aufhellen keratinischer Fasern nach einem der Ansprüche 1 bis 10, **dadurch gekennzeichnet, daß** es zusätzlich 0,1 bis 25 Gew.%, vorzugsweise 0,5 bis 20 Gew.%, besonders bevorzugt 1 bis 15 Gew.% und insbesondere 1,5 bis 10 Gew.% mindestens eines Stoffes aus den Gruppen der
- Carbonate und/oder
- Monoalkylcarbonate (Kohlensäuremonoester) und/oder
- Kohlensäuremonoamide und/oder
- Silylcarbonate und/oder
- Silylcarbamate
enthält.

12. Mittel zum Färben und Aufhellen keratinischer Fasern nach einem der Ansprüche 1 bis 11, **dadurch gekennzeichnet, daß** es zusätzlich Cu-, Fe-, Mn-, Co-, Ce-, V-, Ru-Ionen oder Komplexe dieser Ionen enthält, wobei bevorzugte Mittel 0,0001 bis 2,5 Gew.-%, vorzugsweise 0,001 bis 1 Gew.-% mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Mangandioxid (MnO₂) enthalten.

13. Mittel zum Färben und Aufhellen keratinischer Fasern nach einem der Ansprüche 1 bis 12, **dadurch gekennzeichnet, daß** es zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine
enthält, wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

14. Verfahren zum Färben und Aufhellen von Keratinfasern, insbesondere menschlichen Haaren, **dadurch gekennzeichnet, daß**
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser
aufgetragen und nach einer Einwirkzeit von einigen Minuten wieder abgespült wird, wobei mindestens eines der Mittel M1, M2 oder M3 ein Mittel nach einem der Ansprüche 1 bis 13 ist.

## Claims

1. An agent for coloring and lightening keratin fibers, in particular human hair, containing, based on its weight, from 0.001 to 15 wt.% of at least one compound of formula (Ia): 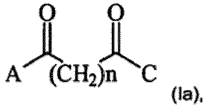 in which
A, C represent, independently of one another, an optionally substituted, in particular halogenated, C₁₋₂₈ alkyl group, preferably an optionally substituted, in particular halogenated, C₁₋₆ alkyl group,
or a C₂₋₂₈ alkylene group, preferably a C₂₋₆ alkylene group,
wherein the functional groups A and C can together form a ring, and wherein preferred functional groups A and C are selected from -CH₃, -CH₂CH₃, -(CH₂)₂CH₂, -CH(CH₃)₂, -(CH₂)₃CH₃, - CH(CH₃)CH₂CH₃, -CH₂CH(CH₃)₂, -C(CH₃)₃, -CF₃, -CCl₃, - CBr₃, -Cl₃, -CF₂CF₃, -CCl₂CCl₃
n represents 0, 1 or 2, preferably 0,
from 0.5 to 15 wt.% of hydrogen peroxide (calculated as 100% H₂O₂),
additionally from 0.001 to 5 wt.% of one or more oxidation dye precursors and/or direct dyes, and from 1 to 20 wt.% of at least one imidazole compound according to formula (VII) and/or the physiologically acceptable salts thereof, 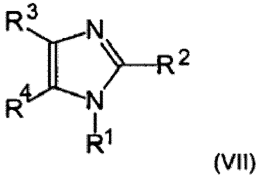 where
R¹ represents a hydrogen atom, an optionally substituted aryl group or a (C₁-C₆) alkyl group,
R² represents a hydrogen atom, a carboxaldehyde group, a (C₁-C₆) alkyl group or a nitro group,
R³ represents a hydrogen atom, a carboxy (C₁-C₆) alkyl group, an amino (C₁-C₆) alkyl group, a carboxyl group, a carboxaldehyde group, a (C₁-C₆) alkyl group, a nitro group, a 2-amino-3-hydroxypropyl group or a -CH₂-CH(NH₂)-COOH group,
R⁴ represents a hydrogen atom, a carboxaldehyde group or a carboxyl group,
wherein the imidazole compound according to formula (VII) and/or the physiologically acceptable salts thereof is/are selected from histamine, D-histidine, L-histidine, DL-histidine, D-histidinol, L-histidinol, DL-histidinol, imidazole, imidazole-4-acetic acid, imidazole-4-carboxylic acid, imidazole-4,5-dicarboxylic acid, imidazole-2-carboxaldehyde, imidazole-4-carboxaldehyde, imidazole-5-carboxaldehyde, 2-nitroimidazole, 4-nitroimidazole, 4-methylimidazole-5-carboxaldehyde, N-methylimidazole-2-carboxaldehyde, 4-methylimidazole, 2-methylimidazole, N-methylimidazole, N-(4-aminophenyl)-imidazole, and the physiologically acceptable salts of said compounds.

2. The agent for coloring and lightening keratin fibers according to claim 1, **characterized in that** it contains one or more compounds from the group
H₃C-C(O)-C(O)-CH₃, H₃C-CH₂-C(O)-C(O)-CH₃, H₃C-(CH₂)₂-C(O)-C(O)-CH₃, (H₃C)₂CH-C(O)-C(O)-CH₃, H₃C-(CH₂)₃-C(O)-C(O)-CH₃, H₃C-CH₂-CH(CH₃)-C(O)-C(O)-CH₃, (H₃C)₂CH-CH₂-C(O)-C(O)-CH₃, (H₃C)₃C-C(O)-C(O)-CH₃, F₃C-C(O)-C(O)-CH₃, Cl₃C-C(O)-C(O)-CH₃, Br₃C-C(O)-C(O)-CH₃, I₃C-C(O)-C(O)-CH₃, F₃C-CF₂-C(O)-C(O)-CH₃, Cl₃C-CCl₂-C(O)-C(O)-CH₃, H₃C-CH₂-C(O)-C(O)-CH₂CH₃, H₃C-(CH₂)₂-C(O)-C(O)-CH₂CH₃, (H₃C)₂CH-C(O)-C(O)-CH₂CH₃, H₃C-(CH₂)₃-C(O)-C(O)-CH₂CH₃, H₃C-CH₂-CH(CH₃)-C(O)-C(C)-CH₂CH₃, (H₃C)₂CH-CH₂-C(O)-C(O)-CH₂CH₃, (H₃C)₃C-C(O)-C(O)-CH₂CH₃, F₃C-C(O)-C(O)-CH₂CH₃, Cl₃C-C(O)-C(O)-CH₂CH₃, Br₃C-C(O)-C(O)-CH₂CH₃, I₃C-C(O)-C(C)-CH₂CH₃, F₃C-CF₂-C(O)-C(O)-CH₂CH₃, Cl₃C-CCl₂-C(O)-C(O)-CH₂CH₃, H₃C-(CH₂)₂-C(O)-C(O)-(CH₂)₂CH₂, (H₃C)₂CH-C(O)-C(O)-(CH₂)₂CH₂, H₃C-(CH₂)₃-C(O)-C(O)-(CH₂)₂CH₂, H₃C-CH₂-CH(CH₃)-C(O)-C(O)-(CH₂)₂CH₂, (H₃C)₂CH-CH₂-C(O)-C(O)-(CH₂)₂CH₂, (H₃C)₃C-C(O)-C(O)-(CH₂)₂CH₂, F₃C-C(O)-C(O)-(CH₂)₂CH₂, Cl₃C-C(O)-C(O)-(CH₂)₂CH₂, Br₃C-C(O)-C(O)-(CH₂)₂CH₂, I₃C-C(O)-C(O)-(CH₂)₂CH₂, F₃C-CF₂-C(O)-C(O)-(CH₂)₂CH₂, Cl₃C-CCl₂-C(O)-C(O)-(CH₂)₂CH₂, (H₃C)₂CH-C(C)-C(O)-CH(CH₃)₂, H₃C-(CH₂)₃-C(O)-C(O)-CH(CH₃)₂, H₃C-CH₂-CH(CH₃)-C(O)-C(O)-CH(CH₃)₂, (H₃C)₂CH-CH₂-C(O)-C(O)-CH(CH₃)₂, (H₃C)₃C-C(O)-C(O)-CH(CH₃)₂, F₃C-C(O)-C(O)-CH(CH₃)₂, Cl₃C-C(O)-C(O)-CH(CH₃)₂, Br₃C-C(O)-C(O)-CH(CH₃)₂, I₃C-C(O)-O(O)-CH(CH₃)₂, F₃C-CF₂₋C(O)-C(O)-CH(CH₃)₂, Cl₃C-CCl₂-C(O)-C(O)-CH(CH₃)₂, H₃C-(CH₂)₃-C(O)-C(OHCH₂)₃CH₃, H₃C-CH₂-CH(CH₃)-C(O)-C(O)-(CH₂)₃CH₃, (H₃C)₂CH-CH₂-C(O)-C(O)-(CH₂)₃CH₃, (H₃C)3C-C(O)-C(O)-(CH₂)₃CH₃, F₃C-C(O)-C(O)-(CH₂)₃CH₃, Cl₃C-C(O)-C(O)-(CH₂)₃CH₃, Br₃C-C(O)-C(O)-(CH₂)₃CH₃, I₃C-C(O)-C(O)-(CH₂)₃CH₃, F₃C-CF₂-C(O)-C(O)-(CH₂)₃CH₃, Cl₃C-CCl₂-C(O)-C(O)-(CH₂)₃CH₃, H₃C-CH₂-CH(CH₃)-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH(CH₃)CH₂CH₃, F₃C-C(O)-C(O)-CH(CH₃)CH₂CH₃, Cl₃C-C(O)-C(O)-CH(CH₃)CH₂CH₃, Br₃C-C(O)-C(O)-CH(CH₃)CH₂CH₃, I₃C-C(O)-C(O)-CH(CH₃)CH₂CH₃, F₃C-CF₂-C(O)-C(O)-CH(CH₃)CH₂CH₃, Cl₃C-CCl₂-C(O)-C(O)-CH(CH₃)CH₂CH₃, (H₃C)₂CH-CH₂-C(O)-C(O)-CH₂CH(CH₃)₂, (H₃C)₃C-C(O)-C(O)-CH₂CH(CH₃)₂, F₃C-C(O)-C(O)-CH₂CH(CH₃)₂, Cl₃C-C(O)-C(O)-CH₂CH(CH₃)₂, Br₃C-C(O)-C(O)-CH₂CH(CH₃)₂, I₃C-C(O)-C(O)-CH₂CH(CH₃)₂, F₃C-CF₂-C(O)-C(O)-CH₂CH(CH₃)₂, Cl₃C-CCl₂-C(O)-C(O)-CH₂CH(CH₃)₂, (H₃C)₃C-C(O)-C(O)-C(CH₃)₃, F₃C-C(O)-C(O)-C(CH₃)₃, Cl₃C-C(O)-C(O)-C(CH₃)₃, Br₃C-C(O)-C(O)-C(CH₃)₃, I₃C-C(O)-C(O)-C(CH₃)₃, F₃C-CF₂-C(O)-C(O)-C(CH₃)₃, Cl₃C-CCl₂-C(O)-C(O)-C(CH₃)₃, F₃C-C(O)-C(O)-CF₃, Cl₃C-C(O)-C(O)-CF₃, Br₃C-C(O)-C(O)-CF₃, I₃C-C(O)-C(O)-CF₃, F₃C-CF₂-C(O)-C(O)-CF₃, Cl₃C-CCl₂-C(O)-C(O)-CF₃, Cl₃C-C(O)-C(O)-CCl₃, Br₃C-C(O)-C(O)-CCl₃, I₃C-C(O)-C(O)-CCl₃, F₃C-CF₂-C(O)-C(O)-CCl₃, Cl₃C-CCl₂-C(O)-C(O)-CCl₃, Br₃C-C(O)-C(O)-CBr₃, I₃C-C(O)-C(O)-CBr₃, FaC-CF₂-C(O)-C(O)-CBr₃, Cl₃C-CCl₂-C(O)-C(O)-CBr₃, I₃C-C(O)-C(O)-Cl₃, F₃C-CF₂-C(O)-C(O)-Cl₃, Cl₃C-CCl₂-C(O)-C(O)-Cl₃, CF₂CF₃-C(O)-C(O)-CF₂CF₃, CCl₂CCl₃-C(O)-C(O)-CF₂CF₃, CCl₂CCl₃-C(O)-C(O)-CCl₂CCl₃, H₃C-C(O)-(CH₂)-C(O)-CH₃, H₃C-CH₂-C(O)-(CH₂)-C(O)-CH₃, H₃C-(CH₂)₂-C(O)-(CH₂)-C(O)-CH₃, (H₃C)₂CH-C(O)-(CH₂)-C(O)-CH₃, H₃C-(CH₂)a-C(O)-(CH₂)-C(O)-CH₃, H₃C-CH₂-CH(CH₃)-C(O)-(CH₂)-C(O)-CH₃, (H₃C)₂CH-CH₂-C(O)-(CH₂)-C(O)-CH₃, (H₃C)₃C-C(O)-(CH₂)-C(O)-CH₃, F₃C-C(O)-(CH₂)-C(O)-CH₃, Cl₃C-C(O)-(CH₂)-C(O)-CH₃, Br₃C-C(O)-(CH₂)-C(O)-CH₃, I₃C-C(O)-(CH₂)-C(O)-CH₃, F₃C-CF₂-C(O)-(CH₂)-C(O)-CH₃, Cl₃C-CCl₂-C(O)-(CH₂)-C(O)-CH₃, H₃C-CH₂-C(O)-(CH₂)-C(O)-CH₂CH₃, H₃C-(CH₂)₂-C(O)-(CH₂)-C(O)-CH₂CH₃, (H₃C)₂CH-C(O)-(CH₂)-C(O)-CH₂CH₃, H₃C-(CH₂)₃-C(O)-(CH₂)-C(O)-CH₂CH₃, H₃C-CH₂-CH(CH₃)-C(O)-(CH₂)-C(O)-CH₂CH₃, (H₃C)₂CH-CH₂-C(O)-(CH₂)-C(O)-CH₂CH₃, (H₃C)₃C-C(OHCH₂)-C(O)-CH₂CH₃, F₃C-C(O)-(CH₂)-C(O)-CH₂CH₃, Cl₃C-C(O)-(CH₂)-C(O)-CH₂CH₃, Br₃C-C(O)-(CH₂)-C(O)-CH₂CH₃, I₃C-C(O)-(CH₂)-C(O)-CH₂CH₃, F₃C-CF₂-C(O)-(CH₂)-C(O)-CH₂CH₃, Cl₃C-CCl₂-C(O)-(CH₂)-C(O)-CH₂CH₃, H₃C-(CH₂)₂-C(O)-(CH₂)-C(O)-(CH₂)₂CH₂, (H₃C)₂CH-C(O)-(CH₂)-C(O)-(CH₂)₂CH₂, H₃C-(CH₂)₃-C(O)-(CH₂)-C(O)-(CH₂)₂CH₂, H₃C-CH₂-CH(CH₃)-C(O)-(CH₂)-C(O)-(CH₂)₂CH₂, (H₃C)₂CH-CH₂-C(O)-(CH₂)-C(O)-(CH₂)₂CH₂, (H₃C)₃C-C(O)-(CH₂)-C(O)-(CH₂)₂CH₂, F₃C-C(O)-(CH₂)-C(O)-(CH₂)₂CH₂, Cl₃C-C(O)-(CH₂)-C(O)-(CH₂)₂CH₂, Br₃C-C(O)-(CH₂)-C(O)-(CH₂)₂CH₂, I₃C-C(O)-(CH₂)-C(O)-(CH₂)₂CH₂, F₃C-CF₂-C(O)-(CH₂)-C(O)-(CH₂)₂CH₂, Cl₃C-CCl₂-C(O)-(CH₂)-C(O)-(CH₂)₂CH₂, (H₃C)₂CH-C(O)-(CH₂)-C(O)-CH(CH₃)₂, H₃C-(CH₂)₃-C(O)-(CH₂)-C(O)-C H(CH₃)₂, H₃C-CH₂-CH(CH₃)-C(O)-(CH₂)-C(O)-CH(CH₃)₂, (H₃C)₂CH-CH₂-C(O)-(CH₂)-C(O)-CH(CH₃)₂, (H₃C)₃C-C(O)-(CH₂)-C(O)-CH(CH₃)₂, F₃C-C(O)-(CH₂)-C(O)-CH(CH₃)₂, Cl₃C-C(O)-(CH₂)-C(O)-CH(CH₃)₂, Br₃C-C(O)-(CH₂)-C(O)-CH(CH₃)₂, I₃C-C(O)-(CH₂)-C(O)-CH(CH₃)₂, F₃C-CF₂-C(O)-(CH₂)-C(O)-CH(CH₃)₂, Cl₃C-CCl₂-C(O)-(CH₂)-C(O)-CH(CH₃)₂, H₃C-(CH₂)₃-C(O)-(CH₂)-C(O)-(CH₂)₃CH₃, H₃C-CH₂-CH(CH₃)-C(O)-(CH₂)-C(O)-(CH₂)₃CH₃, (H₃C)₂CH-CH₂-C(O)-(CH₂)-C(O)-(CH₂)₃CH₃, (H₃C)₃C-C(O)-(CH₂)-C(O)-(CH₂)₃CH₃, F₃C-C(O)-(CH₂)-C(O)-(CH₂)₃CH₃, Cl₃C-C(O)-(CH₂)-C(O)-(CH₂)₃CH₃, Br₃C-C(O)-(CH₂)-C(O)-(CH₂)₃CH₃, I₃C-C(O)-(CH₂)-C(O)-(CH₂)₃CH₃, F₃C-CF₂-C(O)-(CH₂)-C(O)-(CH₂)₃CH₃, Cl₃C-CCl₂-C(O)-(CH₂)-C(O)-(CH₂)₃CH₃, H₃C-CH₂-CH(CH₃)-C(O)-(CH₂)-C(O)-CH(CH₃)CH₂CH₃. (H₃C)2CH-CH₂-C(O)-(CH₂)-C(O)-CH₂CH(CH₃)₂, (H₃C)₃C-C(O)-(CH₂)-C(O)-CH(CH₃)CH₂CH₃, F₃C-C(O)-(CH₂)-C(O)-CH(CH₃)CH₂CH₃, Cl₃C-C(O)-(CH₂)-C(O)-CH(CH₃)CH₂CH₃, Br₃C-C(O)-(CH₂)-C(O)-CH(CH₃)CH₂CH₃, I₃C-C(O)-(CH₂)-C(O)-CH(CH₃)CH₂CH₃, F₃C-CF₂-C(O)-(CH₂)-C(O)-CH(CH₃)CH₂CH₃, Cl₃C-CCl₂-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH₂CH(CH₃)₂, F₃C-C(O)-(CH₂)-C(O)-CH₂CH(CH₃)₂, Cl₃C-C(O)-(CH₂)-C(O)-CH₂CH(CH₃)₂, Br₃C-C(O)-(CH₂)-C(O)-CH₂CH(CH₃)₂, I₃C-C(O)-(CH₂)-C(O)-CH₂CH(CH₃)₂, F₃C-CF₂-C(O)-(CH₂)-C(O)-CH₂CH(CH₃)₂, Cl₃C-CCl₂-C(O)-(CH₂)-C(O)-CH₂CH(CH₃)₂, (H₃C)₃C-C(O)-(CH₂)-C(O)-C(CH₃)₃, F₃C-C(O)-(CH₂)-C(O)-C(CH₃)₃, Cl₃C-C(O)-(CH₂)-C(O)-C(CH₃)₃, Br₃C-C(O)-(CH₂)-C(O)-C(CH₃)₃, I₃C-C(O)-(CH₂)-C(O)-C(CH₃)₃, F₃C-CF₂-C(O)-(CH₂)-C(O)-C(CH₃)₃, Cl₃C-CCl₂-C(O)-(CH₂)-C(O)-C(CH₃)₃, F₃C-C(O)-(CH₂)-C(O)-CF₃, Cl₃C-C(O)-(CH₂)-C(O)-CF₃, Br₃C-C(O)-(CH₂)-C(O)-CF₃, I₃C-C(O)-(CH₂)-C(O)-CF₃, F₃C-CF₂-C(O)-(CH₂)-C(O)-CF₃, Cl₃C-CCl₂-C(O)-(CH₂)-C(O)-CF₃, Cl₃C-C(O)-(CH₂)-C(O)-CCl₃, Br₃C-C(O)-(CH₂)-C(O)-CCl₃, I₃C-C(O)-(CH₂)-C(O)-CCl₃, F₃C-CF₂-C(O)-(CH₂)-C(O)-CCl₃, Cl₃C-CCl₂-C(O)-(CH₂)-C(O)-CCl₃, Br₃C-C(O)-(CH₂)-C(O)-CBr₃, I₃C-C(O)-(CH₂)-C(O)-CBr₃, F₃C-CF₂-C(O)-(CH₂)-C(O)-CBr₃, Cl₃C-CCl₂-C(O)-(CH₂)-C(O)-CBr₃, I₃C-C(O)-(CH₂)-C(O)-Cl₃, F₃C-CF₂-C(O)-(CH₂)-C(O)-Cl₃, Cl₃C-CCl₂-C(O)-(CH₂)-C(O)-Cl₃, CF₂CF₃-C(O)-(CH₂)-C(O)-CF₂CF₃, CCl₂CCl₃-C(O)-(CH₂)-C(O)-CF₂CF₃, CCl₂CCl₃-C(O)-(CH₂)-C(O)-CCl₂CCl₃, H₃C-C(O)-(CH₂)₂-C(O)-CH₃, H₃C-CH₂-C(O)-(CH₂)₂-C(O)-CH₃, H₃C-(CH₂)₂-C(O)-(CH₂)₂-C(O)-CH₃, (H3C)2CH-C(O)-(CH₂)₂-C(O)-CH₃, H₃C-(CH₂)₃-C(O)-(CH₂)₂-C(O)-CH₃, H₃C-CH₂-CH(CH₃)-C(O)-(CH₂)₂-C(O)-CH₃, (H₃C)₂CH-CH₂-C(O)-(CH₂)₂-C(O)-CH₃, (H₃C)₃C-C(OHCH₂)₂-C(O)-CH₃, F₃C-C(O)-(CH₂)₂-C(O)-CH₃, Cl₃C-C(O)-(CH₂)₂-C(O)-CH₃, Br₃C-C(O)-(CH₂)₂-C(O)-CH₃, I₃C-C(O)-(CH₂)₂-C(O)-CH₃, F₃C-CF₂-C(O)-(CH₂)₂-C(O)-CH₃, Cl₃C-CCl₂-C(O)-(CH₂)₂-C(O)-CH₃, H₃C-CH₂-C(O)-(CH₂)2-C(O)-CH₂CH₃, H₃C-(CH₂)₂-C(O)-(CH₂)₂-C(O)-CH₂CH₃, (H₃C)₂CH-C(O)-(CH₂)₂-C(O)-CH₂CH₃, H₃C-(CH₂)₃-C(O)-(CH₂)₂-C(O)-CH₂CH₃, H₃C-CH₂-CH(CH₃)-C(O)-(CH₂)₂-C(O)-CH₂CH₃, (H₃C)₂CH-CH₂-C(O)-(CH₂)₂-C(O)-CH₂CH₃, (H₃C)₃C-C(O)-(CH₂)₂-C(O)-CH₂CH₃, F₃C-C(O)-(CH₂)₂-C(O)-CH₂CH₃, Cl₃C-C(O)-(CH₂)₂-C(O)-CH₂CH₃, Br₃C-C(O)-(CH₂)₂-C(O)-CH₂CH₃, I₃C-C(O)-(CH₂)₂-C(O)-CH₂CH₃, F₃C-CF₂-C(O)-(CH₂)₂-C(O)-CH₂CH₃, Cl₃C-CCl₂-C(O)-(CH₂)₂-C(O)-CH₂CH₃, H₃C-(CH₂)₂-C(O)-(CH₂)₂-C(O)-(CH₂)₂CH₂, (H₃C)₂CH-C(O)-(CH₂)₂-C(O)-(CH₂)₂CH₂, H₃C-(CH₂)₃-C(O)-(CH₂)₂-C(O)-(CH₂)₂CH₂, H₃C-CH₂-CH(CH₃)-C(O)-(CH₂)₂-C(O)-(CH₂)₂CH₂, (H₃CH-CH-CH₂-C(O)-(CH₂)₂-C(O)-(CH₂)₂CH₂, (H₃C)₃C-C(O)-(CH₂)₂-C(O)-(CH₂)₂CH₂, F₃C-C(O)-(CH₂)₂-C(O)-(CH₂)₂CH₂, Cl₃C-C(O)-(CH₂)₂-C(O)-(CH₂)₂CH₂, Br₃C-C(O)-(CH₂)₂-C(O)-(CH₂)₂CH₂, I₃C-C(O)-(CH₂)₂-C(O)-(CH₂)₂CH₂, F₃C-CF₂-C(O)-(CH₂)₂-C(O)-(CH₂)₂CH₂, Cl₃C-CCl₂-C(O)-(CH₂)₂-C(O)-(CH₂)₂CH₂, (H₃C)₂CH-C(O)-(CH₂)₂-C(O)-CH(CH₃)₂, H₃C-(CH₂)₃-C(O)-(CH₂)₂-C(O)-CH(CH₃)₂, H₃C-CH₂-CH(CH₃)-C(O)-(CH₂)₂-C(O)-CH(CH₃)₂, (H₃C)₂CH-CH₂-C(O)-(CH₂)₂-C(O)-CH(CH₃)₂, (H₃C)₃C-C(O)-(CH₂)₂-C(O)-CH(CH₃)₂, F₃C-C(O)-(CH₂)₂-C(O)-CH(CH₃)₂, Cl₃C-C(O)-(CH₂)₂-C(O)-CH(CH₃)₂, Br₃C-C(O)-(CH₂)₂-C(O)-CH(CH₃)₂, I₃C-C(O)-(CH₂)₂-C(O)-CH(CH₃)₂, F₃C-CF₂-C(O)-(CH₂)₂-C(O)-CH(CH₃)₂, Cl₃C-CCl₂-C(O)-(CH₂)₂-C(O)-CH(CH₃)₂, H₃C-(CH₂)₃-C(O)-(CH₂)₂-C(O)-(CH₂)₃CH₃, H₃C-CH₂-CH(CH₃)-C(O)-(CH₂)₂-C(O)-(CH2)3CH3, (H₃C)₂CH-CH₂-C(O)-(CH₂)-C(O)-(CH₂)CH₃, (H₃C)₃C-C(O)-(CH₂)₂-C(O)-(CH₂)₃CH₃, F₃C-C(O)-(CH₂)₂-C(O)-(CH₂)₃CH₃, Cl₃C-C(O)-(CH₂)₂-C(O)-(CH₂)₃CH₃, Br3C-C(O)-(CH₂)₂-C(O)-(CH₂)₃CH₃, I₃C-C(O)-(CH₂)₂-C(O)-(CH₂)₃CH₃, F₃C-CF₂-C(O)-(CH₂)₂-C(O)-(CH₂)₃CH₃, Cl₃C-CCl₂-C(O)-(CH₂)₂-C(O)-(CH₂)₃CH₃, H₃C-CH₂-CH(CH₃)-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH(CH₃)CH₂CH₃, F₃C-C(O)-(CH₂)₂-C(O)-CH(CH₃)CH₂CH₃, Cl₃C-C(O)-(CH₂)₂-C(O)-CH(CH₃)CH₂CH₃, Br₃C-C(O)-(CH₂)₂-C(O)-CH(CH₃)CH₂CH₃, I₃C-C(O)-(CH₂)₂-C(O)-CH(CH₃)CH₂CH₃, F₃C-CF₂-C(O)-(CH₂)₂-C(O)-CH(CH₃)-CH₂CH₃, Cl₃C-CCl₂-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH₂CH(CH₃)₂, F₃C-C(O)-(CH₂)₂-C(O)-CH₂CH(CH₃)₂, Cl₃C-C(O)-(CH₂)₂-C(O)-CH₂CH(CH₃)₂, Br₃C-C(O)-(CH₂)₂-C(O)-CH₂CH(CH₃)₂, I₃C-C(O)-(CH₃)₂-C(O)-CH₂CH(CH₃)₂, F₃C-CF₂-C(O)-(CH₂)₂-C(O)-CH₂CH(CH₃)₂, Cl₃C-CCl₂-C(O)-(CH₂)₂-C(O)-CH₂CH(CH₃)₂, (H₃C)₃C-C(O)-(CH₂)₂-C(O)-C(CH₃)₃. F₃C-C(O)-(CH₂)₂-C(O)-C(CH₃)₃, Cl₃C-C(O)-(CH₂)₂-C(O)-C(CH₃)₃ Br₃-C(O)-(CH₂)₂-C(O)-C(CH₃)₃, I₃C-C(O)-(CH₂)₂-C(O)-C(CH₃)₃, F₃C-CF₂-C(O)-(CH₂)₂-C(O)-C(CH₃)₃, Cl₃C-CCl₂-C(O)-(CH₂)₂-C(O)-C(CH₃)₃, F₃C-C(O)-(CH₂)₂-C(O)-CF₃, Cl₃C-C(O)-(CH₂)₂-C(O)-CF₃, Br₃C-C(O)-(CH₂)₂-C(O)-CF₃, I₃C-C(O)-(CH₂)₂-C(O)-CF₃, F₃C-CF₂-C(O)-(CH₂)₂-C(O)-CF₃, Cl₃C-CCl₂-C(O)-(CH₂)₂-C(O)-CF₃, Cl₃C-C(O)-(CH₂)₂-C(O)-CCl₃, Br₃C-C(O)-(CH₂)₂-C(O)-CCl₃. I₃C-C(O)-(CH₂)₂-C(O)-CCl₃, F₃C-CF₂-C(O)-(CH₂)₂-C(O)-CCl₃, Cl₃C-CCl₂-C(O)-(CH₂)₂-C(O)-CCl₃, Br₃C-C(O)-(CH₂)₂-C(O)-CBr₃, I₃C-C(O)-(CH₂)₂-C(O)-CBr₃, F₃C-CF₂-C(O)-(CH₂)₂-C(O)-CBr₃, Cl₃C-CCl₂-C(O)-(CH₂)₂-C(O)-CBr₃, I₃C-C(O)-(CH₂)₂-C(O)-Cl₃, F₃C-CF₂-C(O)-(CH₂)₂-C(O)-Cl₃, Cl₃C-CCl₂-C(O)-(CH₂)₂-C(O)-Cl₃, CF₂CF₃-C(O)-(CH₂)₂-C(O)-CF₂CF₃, CCl₂CCl₃-C(O)-(CH₂)₂-C(O)-CF₂CF₃, CCl₂CCl₃-C(O)-(CH₂)₂-C(O)-CCl₂CCl₃.

3. The agent for coloring and lightening keratin fibers according to one of claims 1 or 2, **characterized in that** it contains the compound(s) of formula (I) in amounts of from 0.1 to 12.5 wt.%, preferably from 0.25 to 10 wt.%, particularly preferably from 0.5 to 7.5 wt.%, and in particular from 1 to 5 wt.%, in each case based on the total agent.

4. The agent for coloring and lightening keratin fibers according to one of claims 1 to 3, **characterized in that** it contains from 1 to 12.5 wt.%, particularly preferably from 2.5 to 10 wt.%, and in particular from 3 to 6 wt.%, of hydrogen peroxide (calculated as 100% H₂0₂).

5. The agent for coloring and lightening keratin fibers according to one of claims 1 to 4, **characterized in that** it contains at least one developer component as an oxidation dye precursor, preferred developer components being selected from p-phenylenediamine, p-toluylenediamine, N,N-bis-(2-hydroxyethyl)amino-p-phenylenediamine, 1,3-bis-[(2-hydroxyethyl-4'-aminophenyl)amino]-propan-2-ol; 1,10-bis-(2',5'-diaminophenyl)-1,4,7,10-tetraoxadecane, 4-aminophenol, 4-amino-3-methylphenol, bis-(5-amino-2-hydroxyphenyl)methane, 2,4,5,6- tetraaminopyrimidine, 2-hydroxy-4,5,6- triaminopyrimidine, 4,5-diamino-1-(2-hydroxyethyl)-pyrazol.

6. The agent for coloring and lightening keratin fibers according to one of claims 1 to 5, **characterized in that** it contains at least one coupler component as an oxidation dye precursor, preferred coupler components being selected from resorcinol, 2-methylresorcinol, 5-methylresorcinol, 2,5-dimethylresorcinol, 4-chlororesorcinol, resorcinol monomethyl ether, 5-aminophenol, 5-amino-2-methylphenol, 5-(2-hydroxyethyl)amino-2-methylphenol, 3-amino-4-chloro-2-methylphenol, 3-amino-2-chloro-6-methylphenol, 3-amino-2,4-dichlorophenol, 2,4-diaminophenoxyethanol, 2-amino-4-(2'-hydroxyethyl)amino-anisole sulfate, 1, 3-bis-(2,4-diaminophenoxy)propane, 2-amino-3-hydroxypyridine, 2-methylamino-3-amino-6-methoxypyridine, 2,6-dihydroxy-3,4-dimethylpyridine, 3,5-diamino-2,6-dimethoxypyridine, 1-naphthol, 2-methyl-1-naphthol, 1,5-dihydroxynaphthaline, 2, 7-dihydroxynaphthaline, 1-pheny1-3-methylpyrazol-5-one, 2,6-bis-[(2'-hydroxyethyl)amine toluene, 4-hydroxyindole, 6-hydroxyindole, 6-hydroxybenzomorpholine.

7. The agent for coloring and lightening keratin fibers according to one of claims 1 to 6, **characterized in that** it contains at least one direct dye selected from nitrophenylenediamines, nitroaminophenols, azo dyes, anthraquinones or indophenols, preferably from the group of dyes known under the international names or trade names HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Basic Yellow 87, Basic Orange 31 and Basic Red 51 known compounds, and 1,4-diamino-2-nitrobenzene, 2-amino-4-nitrophenol, 1,4-bis-(β-hydroxyethyl)-amino-2-nitrobenzene, 3-nitro-4-β-hydroxyethyl)-aminophenol, 2-(2-hydroxyethyl)amino-4,6-dinitrophenol, 1-(2'-hydroxyethyl)amino-4-methyl-2-nitrobenzene, 1-amino-4-(2-hydroxyethyl)-amino-5-chloro-2-nitrobenzene, 4-amino-3-nitrophenol, 1-(2-ureidoethyl)amino-4-nitrobenzene, 4-amino-2-nitrodiphenylamine-2'-carboxylic acid, 6-nitro-1,2,3,4-tetrahydrochinoxaline, 2-hydroxy-1,4-naphthoquinone, picramatic acid and the salts thereof, 2-amino-6-chloro-4-nitrophenol, 4-ethylamino-3-nitrobenzoic acid and 2-chloro-6-ethylamino-1-hydroxy-4-nitrobenzene.

8. The agent for coloring and lightening keratin fibers according to one of claims 1 to 7, **characterized in that** it additionally contains from 0.1 to 20 wt.%, preferably from 1 to 10 wt.%, and in particular from 2.5 to 12.5 wt.%, of at least one non-ionic compound of general formula (Ni-I), where 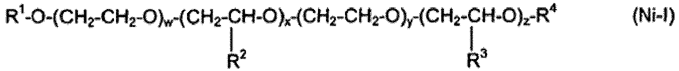
R¹ represents -H or a straight-chain or branched, saturated or mono or polyunsaturated C₆₋₂₄ alkyl or alkenyl functional group,
R² and R³ represent, independently of one another, -CH₃; -CH₂CH₃, -CH₂CH₂-CH₃, CH(CH₃)₂
R⁴ represents -H or a straight-chain or branched, saturated or mono or polyunsaturated C₁₋₄ alkyl or alkenyl functional group, preferably -H, -CH₃; -CH₂CH₃, -CH₂CH₂CH₃, CH(CH₃)₂, -(CH₂)₃CH₃, -CH₂-CH(CH₃)₂, CH(CH₃)CH₂CH₃, -C(CH₃)₃
w, x, y, z represent, independently of one another, numbers from 0 to 50, preferably from 0 to 20, more preferably from 0 to 10, and in particular 0, 1, 2, 3, 4, 5 or 6.

9. The agent for coloring and lightening keratin fibers according to one of the preceding claims, **characterized in that** it contains the at least one imidazole compound according to formula (VII) and/or the physiologically acceptable salts thereof in amounts of from 2 to 15 wt.%, preferably from 2.5 to 12.5 wt.%, particularly preferably from 3 to 10 wt.%, and in particular from 4 to 8 wt.%.

10. The agent for coloring and lightening keratin fibers according to one of claims 1 to 9, **characterized in that** it additionally contains from 0.01 to 2 wt.% of at least one solid peroxo compound selected from ammonium and alkali metal persulfates and peroxydisulfates, preferred agents containing peroxydisulfates preferably selected from sodium peroxydisulfate and/or potassium peroxydisulfate and/or ammonium peroxydisulfate, and particularly preferred agents containing at least two different peroxydisulfates.

11. The agent for coloring and lightening keratin fibers according to one of claims 1 to 10, **characterized in that** it additionally contains from 0.1 to 25 wt.%, preferably from 0.5 to 20 wt.%, particularly preferably from 1 to 15 wt.%, and in particular from 1.5 to 10 wt.%, of at least one substance from the groups of
- carbonates and/or
- monoalkyl carbonates (carbonic acid monoesters) and/or
- carbonic acid monoamides and/or
- silyl carbonates and/or
- silyl carbamates.

12. The agent for coloring and lightening keratin fibers according to one of claims 1 to 11, **characterized in that** it additionally contains Cu, Fe, Mn, Co, Ce, V, Ru ions or complexes of said ions, preferred agents containing from 0.0001 to 2.5 wt.%, preferably from 0.001 to 1 wt.%, of at least one compound from the group of copper chloride (CuCl₂), copper sulfate (CuSO₄), iron(II)sulfate, manganese(II)sulfate, manganese(II)chloride, cobalt(II)chloride, cerium sulfate, cerium chloride, vanadium sulfate, manganese dioxide (MnO₂).

13. The agent for coloring and lightening keratin fibers according to one of claims 1 to 12, **characterized in that** it additionally contains one or more chelate complexing agents from the groups of
(i) polycarboxylic acids in which the sum of the carboxyl and optionally hydroxyl groups is at least five,
(ii) nitrogen-containing mono or polycarboxylic acids,
(iii) geminal diphosphonic acids,
(iv) aminophosphonic acids,
(v) phosphonic polycarboxylic acids,
(vi) cyclodextrins,
preferred agents containing phosphonates, preferably hydroxyalkane or aminoalkane phosphonates and in particular 1-hydroxyethane-1,1-diphosphonate (HEDP) or the di- or tetrasodium salt thereof, and/or ethylenediamine tetramethylene phosphonate (EDTMP) or the hexasodium salt thereof, and/or diethylenetriamine pentamethylene phosphonate (DTPMP) or the hepta or octasodium salt thereof.

14. A method for coloring and lightening keratin fibers, in particular human hair, **characterized in that**
- a pretreatment agent M1 is applied to the fiber if desired, then
- an agent M2 is used on the fiber, an additional agent M3 being added, if desired, to the agent M2 before said agent is used,
- said agent M2 is rinsed off the fiber after 5-30 minutes,
- and a post-treatment agent M4 is optionally applied to the fiber after treatment and is rinsed off again after a contact time of several minutes, at least one of the agents M1, M2 or M3 being an agent according to one of claims 1 to 13.

## Revendications

1. Agent de coloration et d'éclaircissement de fibres de kératine, en particulier de cheveux humains, contenant par rapport à son poids
de 0,001 à 15 % en poids d'au moins un composé de la formule (la) : 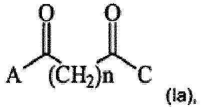 dans laquelle
A, C représentent indépendamment l'un de l'autre un groupe alkyle en C₁ à C₂₈, notamment halogéné, éventuellement substitué, de préférence un groupe alkyle en C₁ à C₆, notamment halogéné, éventuellement substitué,
ou un groupe alkylène en C₁ à C₂₈, de préférence un groupe alkylène en C₂ à C₆,
les radicaux A et C pouvant former conjointement un cycle et
les radicaux A ou C préférés étant choisis parmi -CH₃, -CH₂CH₃, -(CH₂)₂CH₃, -CH(CH₃)₂, - (CH₂)₃CH₃, -CH(CH₃)CH₂CH₃, -CH₂CH(CH₃)₂, -C(CH₃)₃, -CF₃, -CCl₃, -CBr₃, -Cl₃, -CF₂CF₃, - CCl₂CCl₃
n est 0, 1 ou 2, de préférence 0,
0,5 à 15 % en poids de peroxyde d'hydrogène (calculé en tant que H₂O₂ à 100 %), en outre 0,001 à 5 % en poids d'un ou de plusieurs précurseurs de colorants d'oxydation et/ou de colorants directs, et
1 à 20 % en poids d'au moins un composé imidazole de la formule (VII) et/ou de ses sels physiologiquement acceptables 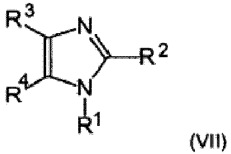 dans laquelle
R¹ représente un atome d'hydrogène, un groupe aryle éventuellement substitué ou un groupe alkyle en C₁ à C₆,
R² représente un atome d'hydrogène, un groupe carboxaldéhyde, un groupe alkyle en C₁ à C₆ ou un groupe nitro,
R³ représente un atome d'hydrogène, un groupe carboxyalkyle en C₁ à C₆, un groupe aminoalkyle en C₁ à C₆, un groupe carboxyle, un groupe carboxaldéhyde, un groupe alkyle en C₁ à C₆, un groupe nitro, un groupe 2-amino-3-hydroxypropyle ou un groupe -CH₂-CH(NH₂)-COOH,
R⁴ représente un atome d'hydrogène, un groupe carboxaldéhyde ou un groupe carboxyle,
le composé imidazole de la formule (VII) et/ou ses sels physiologiquement acceptables sont choisis parmi l'histamine, la D-histidine, la L-histidine, la DL-l'histidine, le D-histidinol, le L-histidinol, le DL-histidinol, l'imidazole, l'acide imidazole-4-acétique, l'acide imidazole-4-carboxylique, l'acide imidazole-4,5-dicarboxylique, l'imidazole-2-carboxaldéhyde, l'imidazole-4-carboxaldéhyde, l'imidazole-5-carboxaldéhyde, le 2-nitroimidazole, le 4-nitroimidazole, le 4-méthylimidazole-5-carboxaldéhyde, le N-méthylimidazole-2-carboxaldéhyde, le 4-méthylimidazole, le 2-méthylimidazole, le N-méthylimidazole, le N-(4-aminophényl)-imidazole, ainsi que les sels physiologiquement acceptables des composés susmentionnés.

2. Agent de coloration et d'éclaircissement de fibres de kératine selon la revendication 1, **caractérisé en ce qu'**il contient un ou plusieurs composés du groupe comportant H₃C-C(O)-C(O)-CH₃, H₃C-CH₂-C(O)-C(O)-CH₃, H₃C-(CH₂)₂-C(O)-C(O)-CH₃, (H₃C)₂CH-C(O)-C(O)-CH₃, H₃C-(CH₂)₃-C(O)-C(O)-CH₃, H₃C-CH₂-CH(CH₃)-C(O)-C(O)-CH₃, (H₃C)₂CH-CH₂-C(O)-C(O)-CH₃, (H₃C)₃C-C(O)-C(O)-CH₃, F₃C-C(O)-C(O)-CH₃, Cl₃C-C(O)-C(O)-CH₃, Br₃C-C(O)-C(O)-CH₃, I₃C-C(O)-C(O)-CH₃, F₃C-CF₂-C(O)-C(O)-CH₃, Cl₃C-CCl₂-C(O)-C(O)-CH₃, H₃C-CH₂-C(O)-C(O)-CH₂CH₃, H₃C-(CH₂)₂-C(O)-C(O)-CH₂CH₃, (H₃C)₂CH-C(O)-C(O)-CH₂CH₃, H₃C-(CH₂)₃-C(O)-C(O)-CH₂CH₃, H₃C-CH₂-CH(CH₃)-C(O)-C(O)-CH₂CH₃, (H₃C)₂CH-CH₂-C(O)-C(O)-CH₂CH₃, (H₃C)₃C-C(O)-C(O)-CH₂CH₃, F₃C-C(O)-C(O)-CH₂CH₃, Cl₃C-C(O)-C(O)-CH₂CH₃, Br₃C-C(O)-C(O)-CH₂CH₃, I₃C-C(O)-C(O)-CH₂CH₃, F₃C-CF₂-C(O)-C(O)CH₂CH₃, Cl₃C-CCl₂-C(O)-C(O)-CH₂CH₃, H₃C-(CH₂)₂-C(O)-C(O)-(CH₂)₂CH₂, (H₃C)₂CH-C(O)-C(O)-(CH₂)₂CH₂, H₃C-(CH₂)₃-C(O)-C(O)-(CH₂)₂CH₂, H₃C-CH₂-CH(CH₃)-C(O)-C(O)-(CH₂)₂CH₂, (H₃C)₂CH-CH₂-C(O)-C(O)-(CH₂)₂CH₂, (H₃C)₃C-C(O)-C(O)-(CH₂)₂CH₂, F₃C-C(O)-C(O)-(CH₂)₂CH₂, Cl₃C-C(O)-C(O)-(CH₂)₂CH₂, Br₃C-C(O)-C(O)-(CH₂)₂CH₂, I₃C-C(O)-C(O)-(CH₂)₂CH₂, F₃C-CF₂-C(O)-C(O)-(CH₂)₂CH₂, Cl₃C-CCl₂-C(O)-C(O)-(CH₂)₂CH₂, (H₃C)₂CH-C(O)-C(O)-CH(CH₃)₂, H₃C-(CH₂)₃-C(O)-C(O)-CH(CH₃)₂, H₃C-CH₂-CH(CH₃)-C(O)-C(O)-CH(CH₃)₂, (H₃C)₂CH-CH₂-C(O)-C(O)-CH(CH₃)₂, (H₃C)₃C-C(O)-C(O)-CH(CH₃)₂, F₃C-C(O)-C(O)-CH(CH₃)₂, Cl₃C-C(O)-C(O)-CH(CH₃)₂, Br₃C-C(O)-C(O)-CH(CH₃)₂, I₃C-C(O)-C(O)-CH(CH₃)₂, F₃C-CF₂-C(O)-C(O)-CH(CH₃)₂, Cl₃C-CCl₂-C(O)-C(O)-CH(CH₃)₂, H₃C-(CH₂)₃-C(O)-C(OHCH₂)₃CH₃, H₃C-CH₂-CH(CH₃)-C-(O)-C(O)-(CH₂)₃CH₃, (H₃C)₂CH-CH₂-C(O)-C(O)-(CH₂)₃CH₃, (H₃C)₃C-C(O)-C(O)-(CH₂)₃CH₃, F₃C-C(O)-C(O)-(CH₂)₃CH₃, Cl₃C-C(O)-C(O)-(CH₂)₃CH₃, Br₃C-C(O)-C(O)-(CH₂)₃CH₃, I₃C-C(O)-C(O)-(CH₂)₃CH₃, F₃C-CF₂-C(O)-C(O)-(CH₂)₃CH₃. Cl₃C-CCl₂-C(O)-C(O)-(CH₂)₃CH₃, H₃C-CH₂-CH(CH₃)-C(O)-C-(O)-CH(CH₃)CH₂CH₃, (H₃C)₂CH-CH₂-C(O)-C(O)-CH₂CH(CH₃)₂, (H₃C)₃C-C(O)-C(O)-CH(CH₃)CH₂CH₃, F₃C-C(O)-C(O)-CH(CH₃)CH₂CH₃, Cl₃C-C(O)-C(O)-CH(CH₃)CH₂CH₃, Br₃C-C(O)-C(O)-CH(CH₃)CH₂CH₃, I₃C-C(O)-C(O)-CH(CH₃)CH₂CH₃, F₃C-CF₂-C(O)-C(O)-CH(CH₃)CH₂CH₃, Cl₃C-CCl₂-C(O)-C(O)-CH(CH₃)CH₂CH₃, (H₃C)₂CH-CH₂-C(O)-C(O)-CH₂CH(CH₃)₂, (H₃C)₃,C-C(O)-C(O)-CH₂CH(CH₃)₂, F₃C-C(O)-C(O)-CH₂CH(CH₃)₂, Cl₃C-C(O)-C(O)-CH₂CH(CH₃)₂, Br₃C-C(O)-C(O)-CH₂CH(CH₃)₂, I₃C-C(O)-C(O)-CH₂CH(CH₃)₂, F₃C-CF₂-C(O)-C(O)-CH₂CH(CH₃)₂, Cl₃C-CCl₂-C(O)-C(O)-CH₂CH(CH₃)₂, (H₃C)₃C-C(O)-C(O)-C(CH₃)₃, F₃C-C(O)-C(O)-C(CH₃)₃, Cl₃C-C(O)-C(O)-C(CH₃)₃, Br₃C-C(O)-C(O)-C(CH₃)₃, I₃C-C(O)-C(O)-C(CH₃)₃. F₃C-CF₂-C(O)-C(O)-C(CH₃)₃, Cl₃C-CCl₂-C(O)-C(O)-C(CH₃)₃, F₃C-C(O)-C(O)-CF₃, Cl₃C-C(O)-C(O)-CF₃, Br₃C-C(O)-C(O)-CF₃, I₃C-C(O)-C(O)-CF₃, F₃C-CF₂-C(O)-C(O)-CF₃. Cl₃C-CCl₂-C(O)-C(O)-CF₃, Cl₃C-C(O)-C(O)-CCl₃, Br₃C-C(O)-C(O)-CCl₃, I₃C-C(O)-C(O)-CCl₃, F₃C-CF₂-C(O)-C(O)-CCl₃, Cl₃C-CCl₂-C(O)-C(O)-CCl₃, Br₃C-C(O)-C(O)-CBr₃, I₃C-C(O)-C(O)-CBr₃, F₃C-CF₂-C(O)-C(O)-CBr₃, Cl₃C-CCl₂-C(O)-C(O)-CBr₃, I₃C-C(O)-C(O)-Cl₃, F₃C-CF₂-C(O)-C(O)-Cl₃, Cl₃C-CCl₂-C(O)-C(O)-Cl₃, CF₂CF₃-C(O)-C(O)-CF₂CF₃, CCl₂CCl₃-C(O)-C(O)-CF₂CF₃, CCl₂CCl₃-C(O)-C(O)-CCl₂CCl₃, H₃C-C(O)-(CH₂)-C(O)-CH₃, H₃C-CH₂-C(O)-(CH₂)-C(O)-CH₃, H₃C-(CH₂)₂-C(O)-(CH₂)-C(O)-CH₃, (H₃C)₂CH-C(O)-(CH₂)-C(O)-CH₃, H₃C-(CH₂)₃-C(O)-(CH₂)-C(O)-CH₃, H₃C-CH₂-CH(CH₃)-C(O)-(CH₂)-C(O)-CH₃, (H₃C)₂CH-CH₂-C(O)-(CH₂)-C(O)-CH₃, (H₃C)₃C-C(O)-(CH₂)-C(O)-CH₃, F₃C-C(O)-(CH₂)-C(O)-CH₃, Cl₃C-C(O)-(CH₂)-C(O)-CH₃, Br₃C-C(O)-(CH₂)-C(O)-CH₃, I₃C-C(O)-(CH₂)-C(O)-CH₃, F₃C-CF₂-C(O)-(CH₂)-C(O)-CH₃, Cl₃C-CCl₂-C(O)-(CH₂)-C(O)-CH3, H₃CH₂-C(O)-(CH₂)-C(O)-CH₂CH₃, H₃C-(CH₂)₂-C(O)-(CH₂)-C(O)-CH₂CH₃, (H₃C)₂CH-C(O)-(CH₂)-C(O)-CH₂CH₃, H₃C-(CH₂)₃-C(O)-(CH₂)-C(O)-CH₂CH₃, H₃C-CH₂-CH(CH₃)-C(O)-(CH₂)-C(O)-CH₂CH₃, (H₃C)₂CH-CH₂-C(O)-(CH₂)-C(O)-CH₂CH₃, (H₃C)₃C-C(O)-(CH₂)-C(O)-CH₂CH₃, F₃C-C(O)-(CH₂)-C(O)-CH₂CH₃, Cl₃C-C(O)-(CH₂)-C(O)-CH₂CH₃, Br₃C-C(O)-(CH₂)-C(O)-CH₂CH₃, I₃C-C(O)-(CH₂)-C(O)-CH₂CH₃, F₃C-CF₂-C(O)-(CH₂)-C(O)-CH₂CH₃, Cl₃C-CCl₂-C(O)-(CH₂)-C(O)-CH₂CH₃, H₃C-(CH₂)₂-C(O)-(CH₂)-C(O)(CH₂)₂CH₂, (H₃C)₂CH-C(O)-(CH₂)-C(O)-(CH₂)₂CH₂, H₃C-(CH₂)₃-C(O)-(CH₂)-C(O)-(CH₂)₂CH₂, H₃C-CH₂-CH(CH₃)-C(O)-(CH₂)-C(O)-(CH₂)₂CH₂, (H₃C)₂CH-CH₂-C(O)-(CH₂)-C(O)-(CH₂)₂CH₂, (H₃C)₃C-C(O)-(CH₂)-C(O)-(CH₂)₂CH₂, F₃C-C(O)-(CH₂)-C(O)-(CH₂)₂CH₂, Cl₃C-C(O)-(CH₂)-C(O)-(CH₂)₂CH₂, Br₃C-C(O)-(CH₂)-C(O)-(CH₂)₂CH₂, I₃C-C(O)-(CH₂)-C(O)-(CH₂)₂CH₂, F₃C-CF₂-C(O)-(CH₂)-C(O)-(CH₂)₂CH₂, Cl₃C-CCl₂-C(O)-(CH₂)-C(O)-(CH₂)₂CH₂, (H₃C)₂CH-C(O)-(CH₂)-C(O)-CH(CH₃)₂, H₃C-(CH₂)₃₋C(O)-(CH₂)-C(O)-CH(CH₃)₂, H₃C-CH₂-CH(CH₃)-C(O)-(CH₂)-C(O)-CH(CH₃)₂, (H₃C)₂CH-CH₂-C(O)-(CH₂)-C(O)-CH(CH₃)₂, (H₃C)₃C-C(O)-(CH₂)-C(O)-CH(CH₃)₂, F₃C-C(O)-(CH₂)-C(O)-CH(CH₃)₂, Cl₃C-C(O)-(CH₂)-C(O)-CH(CH₃)₂, Br₃C-C(O)-(CH₂)-C(O)-CH(CH₂)₂, I₃C-C(O)-(CH₂)-C(O)-CH(CH₃)₂, F₃C-CF₂-C(O)-(CH₂)-C(O)-CH(CH₃)₂, Cl₃C-CCl₂-C(O)-(CH₂)-C(O)-CH(CH₃)₂, H₃C-(CH₂)₃-C(O)-(CH₂)-C(O)-(CH₂)₃CH₃, H₃C-CH₂-CH(CH₃)-C(O)-(CH₂)-C(O)-(CH₂)₃CH₃, (H₃C)₂CH-CH₂-C(O)-(CH₂)-C(O)-(CH₂)₃CH₃, (H₃C)₃C-C(O)-(CH₂)-C(O)-(CH₂)₃CH₃, F₃C-C(O)-(CH₂)-C(O)-(CH₂)₃CH₃, Cl₃C-C(O)-(CH₂)-C(O)-(CH₂)₃CH₃, Br₃C-C(O)-(CH₂)-C(O)-(CH₂)₃CH₃, I₃C-C(O)-(CH₂)-C(O)-(CH₂)₃CH₃, F₃C-CF₂-C(O)-(CH₂)-C(O)-(CH₂)₃CH₃, Cl₃-C-CCl₂-C(O)-(CH₂)-C(O)-(CH₂)₃CH₃, H₃C-CH₂-CH(CH₃)-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH(CH₃)CH₂CH₃, F₃-C-C(O)-(CH₂)-C(O)-CH(CH₃)CH₂CH₃, Cl₃C-C(O)-(CH₂)-C(O)-CH(CH₃)CH₂CH₃, Br₃C-C(O)-(CH₂)-C(O)-CH(CH₃)CH₂CH₃, I₃C-C(O)-(CH₂)-C(O)-CH(CH₃)CH₂CH₃, F₃C-CF₂-C(O)-(CH₂)-C(O)-CH(CH₃)CH₂CH₃, Cl₃C-CCl₂-C(O)-(CH₂)-C(O)-CH(CH₃)CH₂CH₃, (H₃C)₂CH-CH₂-C(O)-(CH₂)-C(O)-CH₂CH(CH₃)₂, (H₃C)₃C-C(O)-(CH₂)-C(O)-CH₂CH(CH₃)₂, F₃C-C(O)-(CH₂)-C(O)-CH₂CH(CH₃)₂, Cl₃C-C(O)-(CH₂)-C(O)-CH₂CH(CH₃)₂, Br₃C-C(O)-(CH₂)-C(O)-CH₂CH(CH₃)₂, I₃C-C(O)-(CH₂)-C(O)-CH₂CH(CH₃)₂, F₃C-CF₂-C(O)-(CH₂)-C(O)-CH₂CH(CH₃)₂, Cl₃C-CCl₂-C(O)-(CH₂)-C(O)-CH₂CH(CH₃)₂, (H₃C)₃C-C(O)-(CH₂)-C(O)-C(CH₃)₃, F₃C-C(O)-(CH₂)-C(O)-C(CH₃)₃, Cl₃C-C(O)-(CH₂)-C(O)-C(CH₃)₃, Br₃C-C(O)-(CH₂)-C(O)-C(CH₃)₃, I₃C-C(O)-(CH₂)-C(O)-C(CH₃)₃, F₃C-CF₂-C(O)-(CH₂)-C(O)-C(CH₃)₃, Cl₃C-CCl₂-C(O)-(CH₂)-C(O)-C(CH₃)₃ F₃-C-C(O)-(CH₂)-C(O)-CF₃, Cl₃C-C(O)-(CH₂)-C(O)-CF₃, Br₃C-C-(O)-(CH₂)-C(O)-CF₃, I₃C-C(O)-(CH₂)-C(O)-CF₃, F₃C-CF₂-C(O)-(CH₂)-C(O)-CF₃, Cl₃C-CCl₂-C(O)-(CH₂)-C(O)-CF₃, Cl₃C-C(O)-(CH₂)-C(O)-CCl₃, Br₃C-C(O)-(CH₂)-C(O)-Cl₃, I₃C-C(O)-(CH₂)-C(O)-CCl₃, F₃C-CF₂-C(O)-(CH₂)-C(O)-CCl₃, Cl₃C-CCl₂-C(O)-(CH₂)-C(O)-CCl₃, Br₃C-C(O)(CH₂)-C(O)-CBr₃, I₃C-C(O)-(CH₂)-C(O)-CBr₃, F₃C-CF₂-C(O)-(CH₂)-C(O)-CBr₃, Cl₃C-CCl₂-C(O)-(CH₂)-C(O)-CBr₃, I₃C-C(O)-(CH₂)-C(O)-Cl₃, F₃C-CF₂-C(O)-(CH₂)-C(O)-Cl₃, Cl₃C-CCl₂-C(O)-(CH₂)-C(O)-Cl₃, CF₂CF₃-C(O)-(CH₂)-C(O)-CF₂CF₃, CCl₂CCl₃₋C(O)-(CH₂)-C(O)-CF₂CF₃, CCl₂CCl₃-C(O)-(CH₂)-C(O)-CCl₂CCl₃, H₃C-C(O)-(CH₂)₂-C(O)-CH₃, H₂C-CH₂-C(O)-(CH₂)₂-C(O)-CH₃, H₃C-(CH₂)₂-C(O)-(CH₂)₂-C(O)-CH₃, (H₃C)₂CH-C(O)-(CH₂)₂-C(O)-CH₃, H₃C-(CH₂)₃-C(O)(CH₂)₂-C(O)-CH₃, H₃C-CH₂-CH(CH₃)-C(O)-(CH₂)₂-C(O)-CH₃, (H₃C)₂CH-CH₂-C(O)(CH₂)₂-C(O)-CH₃, (H₃C)₃C-C(O)-(CH₂)₂-C(O)-CH₃, F₃C-C(O)-(CH₂)₂-C(O)-CH₃, Cl₃C-C(O)-(CH₂)₂-C(O)-CH₃, Br₃C-C(O)-(CH₂)₂₋C(O)-CH₃, I₃C-C(O)-(CH₂)₂-C(O)-CH₃, F₃C-CF₂-C(O)-(CH₂)₂-C(O)-CH₃, Cl₃C-CCl₂-C(O)-(CH₂)₂-C(O)-CH₃, H₃C-CH₃-C(O)-(CH₂)₂-C(O)-CH₂CH₃, H₃C-(CH₂)₂-C(O)-(CH₂)₂-C(O)-CH₂CH₃, (H₃C)₂CH-C(O)-(CH₂)₂-C(O)-CH₂CH₃, H₃C-(CH₂)₃-C(O)-(CH₂)₂-C(O)-CH₂CH₃, H₃C-CH₂-CH(CH₃)-C(O)-(CH₂)₂-C(O)-CH₂CH₃, (H₃C)₂CH-CH₃-C(O)-(CH₂)₂-C(O)-CH₂CH₃, (H₃C)₃C(O)-(CH₂)₂-C(O)-CH₂CH₃, F₃C-C(O)-(CH₂)₂-C(O)-CH₂CH₃, Cl₃C-C(O)-(CH₂)₂-C(O)-CH₂CH₃, Br₃C-C(O)-(CH₂)₂-C(O)-CH₂CH₃, I₃C-C(O)-(CH₂)₂-C(O)-CH₂CH₃, F₃C-CF₂-C(O)-(CH₂)₂-C(O)-CH₂H₃, Cl₃C-CCl₂-C(O)-(CH₂)₂-C(O)-CH₂CH₃, H₃C-(CH₂)₂-C(O)-(CH₂)₂-C(O)-(CH₂)₂CH₃, (H₃C)₂CH-C(O)-(CH₂)₂-C(O)-(CH₃)₂CH₂, H₃C-(CH₂)₃-C(O)-(CH₂)₂-C(O)-(CH₂)₂CH₂, H₃C-CH₂-CH(CH₃)-C(O)-(CH₂)₂-C(O)-(CH₂)₂CH₂, (H₃CH-CH-CH₂-C(O)-(CH₂)₂-C(O)-(CH₂)₂H₂, (H₃C)₃C-C(O)-(CH₂)₂-C(O)-(CH₂)₂H₂, F₃C-C(O)-CH₂)₂-C(O)-(CH₂)₂CH₂, Cl₃C-C(O)-(CH₂)₂-C(O)-(CH₂)₂CH₂, Br₃C-C(O)-(CH₂)₂-C(O)-(CH₂)₂CH₂, I₃C-C(O)-(CH₂)₂-C(O)-(CH₂)₂CH₂, F₃C-CF₃-C(O)-(CH₂)₂-C(O)-(CH₂)₂CH₂, Cl₃C-CCl₂-C(O)-(CH₂)₂-C(O)-(CH₂)₂CH₂, (H₃C)₂CH-C(O)-(CH₂)₂-C(O)-CH(CH₃)₂, H₃C-(CH₂)₃-C(O)-(CH₂)₂-C(O)-CH(CH₃)₂, H₃C-CH₂-CH(CH₃)-C(O)-(CH₂)₂-C(O)-CH(CH₃)₂, (H₃C)₂CH-CH₂-C(O)-(CH₂)₂-C(O)-CH(CH₃)₂, (H₃C)₃C-C(O)-(CH₂)₂-C(O)-CH(CH₃)₂, F₃C-C(O)-(CH₂)₂-C(O)-CH(CH₃)₂, Cl₃C-C(O)-(CH₂)₂-C(O)-CH(CH₃)₂, Br₃C-C(O)-(CH₂)₂-C(O)-CH(CH₃)₂, I₃C-C(O)-(CH₂)₂-C(O)-CH(CH₃)₂, F₃C-CF₂-C(O)-(CH₂)₂-C(O)-CH(CH₃)₂, Cl₃C-CCl₂-C(O)-(CH₂)₂-C(O)-CH(CH₃)₂, H₃C-(CH₂)₃-C(O)-(CH₂)₂-C(O)-(CH₂)₃CH₃, H₃C-CH₂-CH(CH₃)-C(O)-(CH₂)₂-C(O)-(CH₂)₃CH₃, (H₃C)₂CH-CH₂-C(O)-(CH₂)-C(O)-(CH₂)CH₃, (H₃C)₃C-C(O)-(CH₂)₂-C(O)-(CH₂)₃CH₃, F₃C-C(O)-(CH₂)₂-C(O)-(CH₂)₃CH₃, Cl₃C-C(O)-(CH₂)₂-C(O)-(CH₂)₃CH₃, Br₃C-C(O)-(CH₂)₂-C(O)-(CH₂)₃CH₃, I₃C-C(O)-(CH₂)₂-C(O)-(CH₂)₃CH₃, F₃C-CF₂-C(O)(CH₂)₂-C(O)-(CH₂)₃CH₃, Cl₃C-CCl₂-C(O)-(CH₂)₂-C(O)-(CH₂)₃CH₃, H₃C-CH₂-CH(CH₃)-C(O)-(CH₂)₂-C(O)-CH(CH₃)CH₂CH₃, (H₃C)₂CH-CH₂-C(O)-(CH₂)₂-C(O)-CH₂H(CH₃)₂, (H₃C)₃C-C(O)-(CH₂)₂-C(O)-CH(CH₃)CH₂CH₃, F₃C-C(O)-(CH₂)₂-C(O)-CH(CH₃)CH₂CH₃, Cl₃C-C(O)-(CH₂)₂-C(O)-CH(CH₃)CH₂CH₃, Br₃C-C(O)-(CH₂)₂-C(O)-CH(CH₃)CH₂CH₃, I₃C-C(O)-(CH₂)₂-C(O)-CH(CH₃)CH₂CH₃, F₃C-CF₂-C(O)-(CH₂)₂-C(O)-CH(CH₃)CH₂CH₃, Cl₃C-CCl₂-C(O)-(CH₂)₂-C(O)-CH(CH₃)CH₂CH₃, (H₃C)₂CH-CH₂-C(O)-(CH₂)₂-C(O)-CH₂CH(CH₃)₂, (H₃C)₃C-C(O)-(CH₂)₂-C(O)-CH₂CH(CH₃)₂, F₃C-C(O)-(CH₂)₂-C(O)-CH₂CH(CH₃)₂, Cl₃C-C(O)-(CH₂)₂-C(O)-CH₂CH(CH₃)₂, Br₃-C(O)-(CH₂)₂-C(O)-CH₂CH(CH₃)₂, I₃C-C(O)-(CH₂)₂-C(O)-CH₂CH(CH₃)₂, F₃C-CF₂-C(O)-(CH₂)₂-C(O)-CH₂CH(CH₃)₂, Cl₃C-CCl₂-C(O)-(CH₂)₂-C(O)-CH₂CH(CH₃)₂, (H₃C)₃C-C(O)-(CH₂)₂-C(O)-C(CH₃)₃, F₃C-C(O)-(CH₂)₂-C(O)-C(CH₃)₃, Cl₃C-C(O)-(CH₂)₂-C(O)-C(CH₃)₃, Br₃C-C(O)-(CH₂)₂-C(O)-C(CH₃)₃, I₃C-C(O)-(CH₂)₂-C(O)-C(CH₃)₃, F₃C-CF₂-C(O)-(CH₂)₂-C(O)-C(CH₃)₃, Cl₃C-CCl₂-C(O)-(CH₂)₂-C(O)-C(CH₃)₃, F₃C-C(O)-(CH₂)₂-C(O)-CF₃, Cl₃C-C(O)-(CH₂)₂-C(O)-CF₃, Br₃C-C(O)-(CH₂)₂-C(O)-CF₃, I₃C-C(O)-(CH₂)₂-C(O)-CF₃, F₃C-CF₂-C(O)-(CH₂)₂-C(O)-CF₃, Cl₃C-CCl₂-C(O)-(CH₂)₂-C(O)-CF₃, Cl₃C-C(O)-(CH₂)₂-C(O)-CCl₃, Br₃C-C(O)-(CH₂)₂-C(O)-CCl₃, I₃C-C(O)-(CH₂)₂-C(O)-CCl₃, F₃C-CF₂-C(O)-(CH₂)₂-C(O)-CCl₃, Cl₃C-CCl₂-C(O)-(CH₂)₂-C(O)-CCl₃, Br₃C-C(O)-(CH₂)₂-C(O)-CBr₃, I₃C-C(O)-(CH₂)₂-C(O)-CBr₃, F₃C-CF₂-C(O)-(CH₂)₂-C(O)-CBr₃, Cl₃C-CCl₂-C(O)-(CH₂)₂-C(O)-CBr₃, I₃C-C(O)-(CH₂)₂-C(O)-Cl₃, F₃C-CF₂-C(O)-(CH₂)₂-C(O)-Cl₃, Cl₃C-CCl₂-C(O)-(CH₂)₂-C(O)-Cl₃, CF₂CF₃-C(O)-(CH₂)₂-C(O)-CF₂CF₃, CCl₂-CCl₃-C(O)-(CH₂)₂-C(O)-CF₂CF₃, CCl₂CCl₃-C(O)-(CH₂)₂-C(O)-CCl₂CCl₃.

3. Agent de coloration et d'éclaircissement des fibres de kératine selon l'une des revendications 1 ou 2, **caractérisé en ce qu'**il contient le ou les composés de la formule (I) dans des quantités allant de 0,1 à 12,5 % en poids, de préférence de 0,25 à 10 % en poids, de manière particulièrement préférée de 0,5 à 7,5 % en poids et en particulier de 1 à 5 % en poids, respectivement rapportés au produit total.

4. Agent de coloration et d'éclaircissement de fibres de kératine selon l'une des revendications 1 à 3, **caractérisé en ce qu'**il contient de 1 à 12,5 % en poids, plus préférablement de 2,5 à 10 % en poids et en particulier de 3 à 6 % en poids de peroxyde d'hydrogène (calculé en tant que H₂O₂ à 100%).

5. Agent de coloration et d'éclaircissement de fibres de kératine selon l'une des revendications 1 à 4, **caractérisé en ce qu'**il contient comme précurseur de colorant d'oxydation au moins un composant développeur, les composants développeurs préférés étant choisis parmi la p-phénylènediamine, la p-toluènediamine, la N,N-bis-(2-hydroxyéthyl)amino-p-phénylènediamine, le 1,3-bis-[(2-hydroxyéthyl-4'-aminophényl)amino]-propan-2-ol ; le 1,10-bis(2',5'-diaminophényl)-1,4,7,10-tétraoxadécane, le 4-aminophénol, le 4-amino-3-méthylphénol, le bis-(5-amino-2-hydroxyphényl)méthane, la 2,4,5,6-tétraaminopyrimidine, la 2-hydroxy-4,5,6-triaminopyrimidine, le 4,5-diamino-1-(2-hydroxyéthyl)pyrazole.

6. Agent de coloration et d'éclaircissement de fibres de kératine selon l'une des revendications 1 à 5, **caractérisé en ce qu'**il contient comme précurseur de colorant d'oxydation au moins un composant copulateur, les composants copulateurs préférés étant choisis parmi le résorcinol, le 2-méthylrésorcinol, le 5-méthyl-résorcinol, le 2,5-diméthylrésorcinol, le 4-chlororésorcinol, l'éther monométhylique de résorcinol, le 5-aminophénol, le 5-amino-2-méthylphénol, le 5-(2-hydroxyéthyl)amino-2-méthylphénol, le 3-amino-4-chloro-2-méthylphénol, le 3-amino-2-chloro-6-méthylphénol, le 3-amino-2,4-dichlorophénol, le 2,4-diaminophénoxyéthanol, le sulfate de 2-amino-4-(2'-hydroxyéthyl)amino-anisol, le 1,3-bis-(2,4-diaminophénoxy)propane, la 2-amino-3-hydroxypyridine, la 2-méthylamino-3-amino-6-méthoxypyridine, la 2,6-dihydroxy-3,4-diméthylpyridine, la 3,5-diamino-2,6-diméthoxypyridine, le 1-naphtol, le 2-méthyl-1-naphtol, le 1,5-dihydroxynaphtalène, le 2,7-dihydroxynaphtalène, la 1-phényl-3-méthylpyrazol-5-one, le 2,6-bis-[(2'-hydroxyéthyl)amino]-toluène, le 4-hydroxyindole, le 6-hydroxyindole, la 6-hydroxybenzomorpholine.

7. Agent de coloration et d'éclaircissement de fibres de kératine selon l'une des revendications 1 à 6, **caractérisé en ce qu'**il comprend au moins un colorant direct choisi parmi les nitrophénylènediamines, les nitroaminophénols, les colorants azoïques, les anthraquinones ou les indophénols, de préférence dans le groupe des colorants connus sous les dénominations internationales ou noms commerciaux HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Basic Yellow 87, Basic Orange 31 et Basic Red 51, ainsi que le 1,4-diamino-2-nitrobenzène, le 2-amino-4-nitrophénol, le 1,4-bis-(β-hydroxyéthyl)-amino-2-nitrobenzène, le 3-nitro-4-(β-hydroxyéthyl)-aminophénol, le 2-(2-hydroxyéthyl)amino-4,6-dinitrophénol, le 1-(2'-hydroxyéthyl)amino-4-méthyl-2-nitrobenzène, le 1-amino-4-(2-hydroxyéthylamino-5-chloro-2-nitrobenzène, le 4-amino-3-nitrophénol, le 1-(2-uréidoéthyl)amino-4-nitrobenzène, l'acide 4-amino-2-nitrodiphénylamine-2'-carboxylique, la 6-nitro-1,2,3,4-tétrahydroquinoxaline, le 2-hydroxy-1,4-naphtoquinone, l'acide picramique et ses sels, le 2-amino-6-chloro-4-nitrophénol, l'acide 4-éthylamino-3-nitrobenzoïque et le 2-chloro-6-éthylamino-1-hydroxy-4-nitrobenzène.

8. Agent de coloration et d'éclaircissement de fibres de kératine selon l'une des revendications 1 à 7, **caractérisé en ce qu'**il contient en outre de 0,1 à 20 % en poids, de préférence de 1 à 10 % en poids et en particulier de 2,5 à 12,5 % en poids d'au moins un composé non ionique de la formule générale (Ni-I) 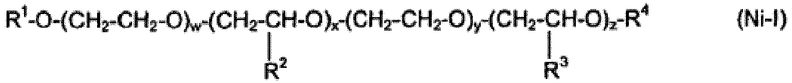 dans laquelle
R¹ représente -H ou un radical alkyle ou alcényle en C₆ à C₂₄ linéaire ou ramifié, saturé ou monoinsaturé ou polyinsaturé,
R² et R³ représentent indépendamment -CH₃, -CH₂CH₃, -CH₂CH₂-CH₃, CH(CH₃)₂
R⁴ représente -H ou un radical alkyle ou alcényle en C₁ à C₄ linéaire ou ramifié, saturé ou monoinsaturé ou polyinsaturé, de préférence -H, -CH₃, -CH₂CH₃, -CH₂CH₂CH₃, -CH(CH₃)₂, - (CH₂)₃CH₃, -CH₂-CH(CH₃)₂, -CH(CH₃)CH₂CH₃, -C(CH₃)₃
w, x, y, z représentent indépendamment les uns des autres des nombres de 0 à 50, de préférence de 0 à 20, plus préférablement de 0 à 10 et notamment 0, 1, 2, 3, 4, 5, 6.

9. Agent de coloration et d'éclaircissement de fibres de kératine selon l'une des revendications précédentes, **caractérisé en ce qu'**il contient au moins un composé imidazole de la formule (VII) et/ou un de ses sels physiologiquement acceptables dans des quantités allant de 2 à 15 % en poids, de préférence de 2,5 à 12,5 % en poids, de manière particulièrement préférée de 3 à 10 % en poids et en particulier de 4 à 8 % en poids.

10. Agent de coloration et d'éclaircissement de fibres de kératine selon l'une des revendications 1 à 9, **caractérisé en ce qu'**il contient en outre de 0,01 à 2 % en poids d'au moins un composé peroxo solide choisi parmi les persulfates d'ammonium et de métaux alcalins et les peroxodisulfates, les agents préférés contenant des peroxodisulfates qui sont de préférence choisis parmi le peroxodisulfate de sodium et/ou le peroxodisulfate de potassium et/ou le peroxodisulfate d'ammonium et des agents particulièrement préférés contenant au moins deux peroxodisulfates différents.

11. Agents de coloration et d'éclaircissement de fibres de kératine selon l'une des revendications 1 à 10, **caractérisé en ce qu'**il contient en outre de 0,1 à 25 % en poids, de préférence de 0,5 à 20 % en poids, de façon particulièrement préférée de 1 à 15 % en poids et en particulier de 1,5 à 10 % en poids d'au moins une substance des groupes
- des carbonates et/ou
- des carbonates de monoalkyle (monoesters d'acide carbonique) et/ou
- des monoamides d'acide carbonique et/ou
- des carbonates de silyle et/ou
- des carbamates de silyle.

12. Agent de coloration et d'éclaircissement de fibres de kératine selon l'une des revendications 1 à 11, **caractérisé en ce qu'**il contient en outre des ions Cu, Fe, Mn, Co, Ce, V, Ru ou des complexes de ces ions, des agents préférés contenant de 0,0001 à 2,5 % en poids, de préférence de 0,001 à 1 % en poids d'au moins un composé du groupe comportant le chlorure de cuivre (CuCl₂), le sulfate de cuivre (CuSO₄), le sulfate de fer(II), le sulfate de manganèse(II), le chlorure de manganèse(II), le chlorure de cobalt (II), le sulfate de cérium, le chlorure de cérium, le sulfate de vanadium, le dioxyde de manganèse (MnO₂).

13. Agent de coloration et d'éclaircissement de fibres de kératine selon l'une des revendications 1 à 12, **caractérisé en ce qu'**il contient en outre un ou plusieurs agents complexants chélatés des groupes comportant
(i) les acides polycarboxyliques dans lesquels la somme des groupes carboxyle et éventuellement hydroxyle est d'au moins 5,
(ii) les acides mono- ou polycarboxyliques azotés,
(iii) les acides diphosphoniques géminés,
(iv) les acides aminophosphoniques,
(v) les acides phosphonopolycarboxyliques,
(vi) les cyclodextrines
les agents préférés contenant les phosphonates, de préférence les phosphonates d'hydroxyalcanes ou d'aminoalcanes et en particulier le 1-hydroxyéthane-1,1-diphosphonate (HEDP) ou son sel disodique ou tétrasodique et/ou le phosphonate d'éthylènediaminetétraméthylène (EDTMP) ou son sel hexasodique et/ou le phosphonate de diéthylènetriaminepentaméthylène (DTPMP) ou son sel heptasodique ou octasodique.

14. Procédé de coloration et d'éclaircissement de fibres de kératine, en particulier de cheveux humains, **caractérisé en ce que**
- un agent de prétraitement M1 est appliqué, si on le souhaite, sur les fibres, puis
- un moyen M2 est appliqué sur les fibres, un autre agent M3 étant ajouté, si on le souhaite, à l'agent M2 avant application,
- cet agent M2 est rincé de la fibre au bout de 5 à 30 minutes,
- et éventuellement, après le traitement, un agent de post-traitement M4 est appliqué sur les fibres et rincé après une durée d'action de quelques minutes,
- au moins un des agents M1, M2 ou M3 étant un agent selon l'une des revendications 1 à 13.
